(19) Europäisches Patentamt
European Patent Office
Office européen des brevets

(11) EP 4 017 854 B1

(12) # EUROPEAN PATENT SPECIFICATION

(45) Date of publication and mention of the grant of the patent:
**13.08.2025 Bulletin 2025/33**

(21) Application number: **20772101.0**

(22) Date of filing: **20.08.2020**

(51) International Patent Classification (IPC):
***C07D 471/14*** *(2006.01)* ***C07D 487/04*** *(2006.01)*
***C07D 487/14*** *(2006.01)* ***A61P 31/04*** *(2006.01)*
***A61P 33/06*** *(2006.01)* ***A61K 31/519*** *(2006.01)*

(52) Cooperative Patent Classification (CPC):
**C07D 487/04; A61P 31/04; A61P 33/06; C07D 471/14; C07D 487/14;** Y02A 50/30

(86) International application number:
**PCT/IB2020/057831**

(87) International publication number:
**WO 2021/033159 (25.02.2021 Gazette 2021/08)**

(54) **PYRAZINO[1,2-B]QUINAZOLINE-3,6-DIONES DERIVATIVES, THEIR PRODUCTION AND USES THEREOF**

PYRAZINO[1,2-B]CHINAZOLIN-3,6-DIONDERIVATE, IHRE HERSTELLUNG UND IHRE VERWENDUNG

DÉRIVÉS DE PYRAZINO [1,2-B]QUINAZOLINE-3,6-DIONES, LEUR PRODUCTION ET LEURS UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.08.2019 PT 2019115744**

(43) Date of publication of application:
**29.06.2022 Bulletin 2022/26**

(73) Proprietors:
- **CIIMAR - Centro Interdisciplinar de Investigação Marinha e Ambiental**
  **4450-208 Matosinhos (PT)**
- **Universidade Do Porto**
  **4099-002 Porto (PT)**
- **UNIVERSIDADE NOVA DE LISBOA**
  **1099-085 Lisboa (PT)**

(72) Inventors:
- **DA SILVA PEREIRA DE SOUSA, Maria Emilia**
  **4050-313 Porto (PT)**
- **LONG, Solida**
  **4050-313 Porto (PT)**
- **DE MAGALHAES PINTO, Madalena Maria**
  **4050-313 Porto (PT)**
- **RESENDE, Diana**
  **4050-313 Porto (PT)**
- **KIJJOA, Anake**
  **4050-313 Porto (PT)**
- **RODRIGUES MARTINS DA COSTA, Paulo Manuel**
  **4050-313 Porto (PT)**
- **MACHADO FREITAS DA SILVA, Joana Manuela**
  **4050-313 Porto (PT)**
- **NOGUEIRA, Fatima**
  **1349-008 Lisboa (PT)**

(74) Representative: **Patentree**
**Edificio Net**
**Rua de Salazares, 842**
**4149-002 Porto (PT)**

(56) References cited:
**WO-A2-00/18769**

- **LIAO ET AL.: "Alkaloidal Metabolites from a Marine-Derived Aspergillus sp. Fungus", JOURNAL OF NATURAL PRODUCTS, vol. 78, no. 3, 12 January 2015 (2015-01-12), US, pages 349 - 354, XP055743848, ISSN: 0163-3864, DOI: 10.1021/np500683u**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 017 854 B1

- **LIAO ET AL.: "Supporting Information for Alkaloidal Metabolites from a Marine-Derived Aspergillus sp. Fungus", JOURNAL OF NATURAL PRODUCTS 2015, 78, 349-354, 12 January 2015 (2015-01-12), pages 1 - 36, XP055743855, Retrieved from the Internet <URL:https://ndownloader.figstatic.com/files/3816136> [retrieved on 20201026]**
- **GARCIA SILVA ET AL.: "Antibacterial activity from Penicillium corylophilum Dierckx", MICROBIOLOGICAL RESEARCH, vol. 159, no. 4, 15 December 2004 (2004-12-15), pages 317 - 322, XP004956704, ISSN: 0944-5013, DOI: 10.1016/J.MICRES.2004.06.003**
- **LONG ET AL.: "Synthesis of New Proteomimetic Quinazolinone Alkaloids and Evaluation of Their Neuroprotective and Antitumor Effects", MOLECULES, vol. 24, 534, no. 3, 1 February 2019 (2019-02-01), pages 1 - 17, XP055743814, DOI: 10.3390/molecules24030534**

## Description

### Technical Field

The present disclosure relates to pyrazino [1,2-*b*]quinazoline-3,6-diones compounds, in particular it relates to pyrazino [1,2-*b*]quinazoline-3,6-diones compounds having antibacterial activity and/or antimalarial activity.

### Background

Infectious diseases caused by microorganisms stand as a major threat to public health. Since antibiotics were first introduced as medicines, these drugs have been used to prevent or treat infections in several applications. Nonetheless, antibacterial resistance has increased dramatically, becoming an emergency in healthcare during the last 40 years. Among 50 emerging infectious agents that have been identified, 10% have developed resistance to multiple drugs including antibiotics such as vancomycin, methicillin, carbapenems, and cephalosporins. Despite enormous efforts, the number of therapeutically useful compounds that aim for circumventing the resistance is continuously decreasing and no truly novel class of compounds has been introduced into therapy, causing the world to face the "post-antibiotic era". In order to stop the clinical consequences of the development and spread of antimicrobial resistance both the preservation of current antimicrobials through their appropriate use, as well as the discovery and development of new agents are mandatory.

Malaria represents a major threat to the public health worldwide, with over 219 million clinical cases in 2017 with 435 thousand of deaths. Though the number of cases has shown a decrease since 2010, evidences of slower *Plasmodium falciparum* parasite clearance have appeared in some countries in Southeast Asia especially at Greater Mekong Subregion (GMS) including Lao PDR, Thailand, Cambodia, Myanmar, and Vietnam. These represent a serious threat to global malaria control and eradication. The frontline therapies for the treatment of symptomatic malaria are artemisinin (5) combination therapies (ACTs) for *P. falciparum* infections and in the case of infections with *P. vivax,* chloroquine (CQ, 6) or ACTs are usually employed. This evidence, along with widespread resistance to other historical antimalarials, highlights the need to identify new chemical diversity, ideally with novel antimalarial modes of action.

Several reports emphasized the discovery of new sophisticated antimicrobials from marine sources as a promising strategy to overcome the ever-increasing drug-resistant infectious diseases. In the last years, fungal alkaloids containing an indolomethyl pyrazino[1,2-b] quinazoline-3,6-dione scaffold were isolated from marine organisms and presented very interesting antimicrobial activities [1]. For instance, glyantypine (1) isolated from *Cladosporium* sp. PJX-41, exhibited moderate inhibitory activity against bacteria *Vibrio harvevi* (MIC = 32 μg/mL) and neofiscalin A **(2)** found in *Neosartorya siamensis* KUFC 6349 exhibited a potent antibacterial activity against *Staphylococcus aureus* and *Enterococcus faecalis* (MIC = 8 μg/mL) [2].

Strategies used for the development of novel antimalarial drugs include the discovery of new active molecules from natural products, repurposing of commercially available drugs, development of hybrid compounds, and rational drug design with molecular modifications of existing antimalarial and hits. The malarial chemotherapy has always been successfully influenced by natural products and nature is still an important source of antimalarial drugs. Recently, the analysis of Tres Cantos Antimalarial Set (TCAMS) suggested that indole-based antimalarials are the key core for the development of the next generation of antimalarial drugs since the indole scaffold is known as an important moiety present in several lead drug candidates with new mechanisms of action, such as the spiroindolone (7), febrifugine (9), and aminoindole derivatives. For example, TCMDC-134281 (8) exhibited very potent antiplasmodial properties against P. *falciparum* 3D7 strain ($EC_{50}$ = 34 nM). However, although TCMDC-134281 showed no significant cytotoxicity against human HepG2 hepatoma cell line ($EC_{50}$ > 10 μM), the presence of the 4-aminoquinolyl moiety (an essential pharmacophore of CQ) might be responsible for its cross-resistance with CQ (6) and poor-drug-like properties [5].

Lijuan Liao *et al.* discloses the isolation and characterization of fumiquinazoline F, and its activity against gram-positive B.subtilis and Gram-negative P.hauseri (Liao L, You M, Chung BK, Oh DC, Oh KB, Shin J. Alkaloidal metabolites from a marine-derived Aspergillus sp. fungus. J Nat Prod. 2015 Mar 27;78(3):349-54).

Marley Garci Silva *et al.* discloses the isolation and antimicrobial activity of fumiquinazoline F against M.luetus and S.aureus (Silva MG, Furtado NA, Pupo MT, Fonseca MJ, Said S, da Silva Filho AA, Bastos JK. Antibacterial activity from Penicillium corylophilum Dierckx. Microbiol Res. 2004;159(4):317-22).

WO0018769A2 discloses indolo[2,1-b]quinazole-6,12-diones as antimalarial agents.

Solida Long *et al.* discloses the neuroprotective and antitumor effects activity of fiscalin B, fumiquinazoline G, and derivatives.

### General Description

The present disclosure relates to four possible approaches to obtain indole-containing pyrazino[2,1-*b*]quinazoline-3,6-diones comprising a subclass of alkaloids mostly isolated from marine and terrestrial sources. These structurally unique alkaloids contain simultaneously a quinazoline core which can be found in the structure of the natural febrifugine (9) and an indole moiety commonly found in several drug lead candidates such as spiroindolone (7) and TCMDC-134281 (8). This hybrid structure comprises a quinazoline core and an indole core such that the observed inhibitory growth of MRSA may be observed and cross-resistance with CQ and ACTs may be overcome.

The first approach is based on the synthesis of enantiomeric pairs of two members of this quinazolinone family (structural modifications at C-1 and C-4 stereochemistry), including the marine-derived alkaloid fiscalin B (7A).

The second approach is based on the synthesis of other derivatives of these natural alkaloids, but with modification of the C-1 side chain and stereochemistry, by using different amino acids.

The third approach is based on the synthesis of indolomethyl pyrazino[1,2-b]quinazoline-3,6-dione analogs: the introduction of halogen atoms in the aromatic ring of the anthranilic acid (Ant).

The fourth approach is based on the synthesis of ring A variations on the pyrazino[2,1-b]quinazoline-3,6-dione scaffold or with an additional indole moiety.

The present disclosure relates to a compound of formula I

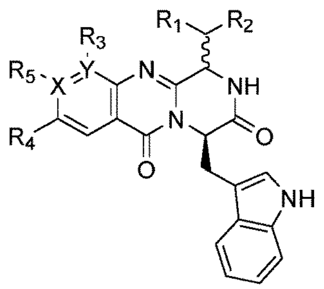

wherein

$R^1$, $R^2$, $R^3$, $R^4$, $R^5$, X and Y are independently selected from each other;
$R^1$ and $R^2$ are selected from H or $CH_3$ or $CH(CH_3)_2$ or $CH_2CH_3$,
$R^3$ is selected from H or Cl or Br or F or OH or $OCH_3$,
$R^4$ is selected from H or Cl or Br or I or F or OH or $OCH_3$;
$R^5$ is H or

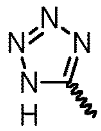
,

and
X and Y are selected from N or C;
or a pharmaceutically acceptable salt, or solvate thereof,
provided that
when X and Y are C then $R^4$ is different from H; or
when X and Y are C then $R^4$ is H and $R^5$ is

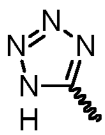
;

or
when X is N then $R^5$ is absent or
when Y is N then $R^3$ is absent.

In an embodiment, X and Y may be C.

In an embodiment, $R^1$ may be H or $CH_3$.

In an embodiment, $R^2$ may be $CH_3$ or $CH(CH_3)_2$ or $CH_2CH_3$.

In an embodiment, $R^3$ may be H or Cl or I.

In an embodiment, $R^4$ may be Cl or I, preferably $R^4$ may be Cl.

In an embodiment, $R^5$ may be H.

In an embodiment, the compound may be

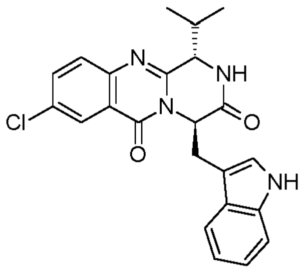

or

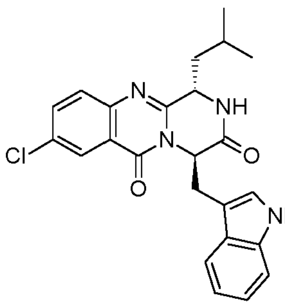

or

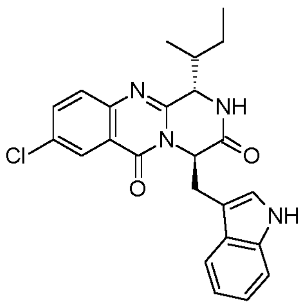

,

preferably the compound may be

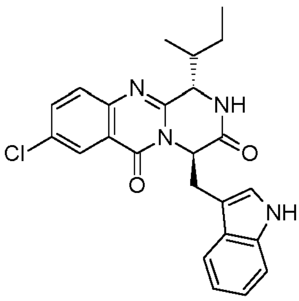

.

In an embodiment, the compound may be

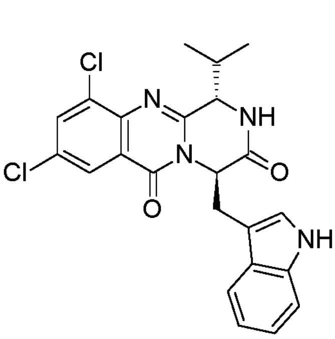

or,

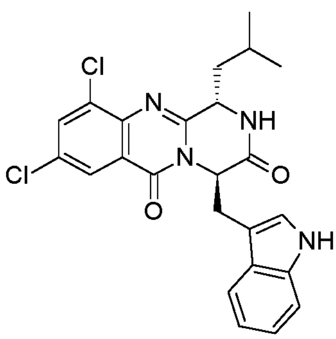

or,

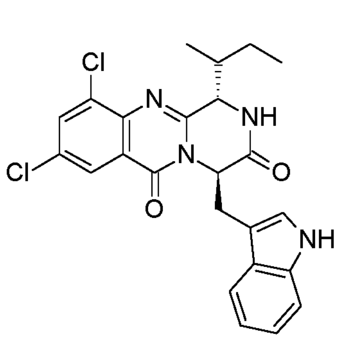

or,

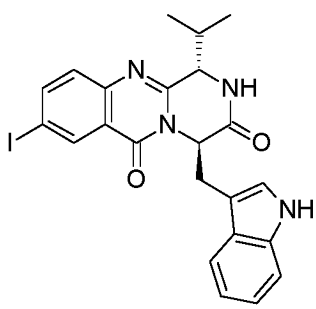 or, 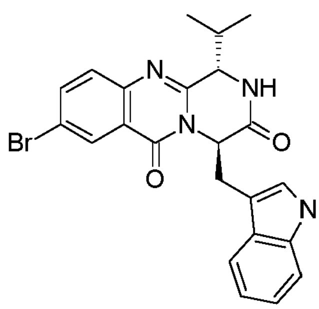 or, 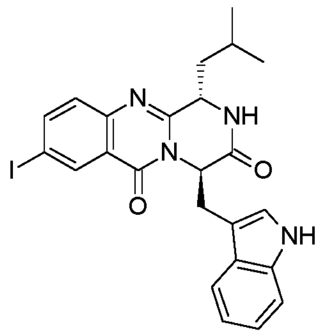

or,

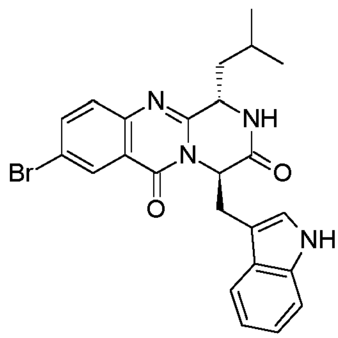 or, 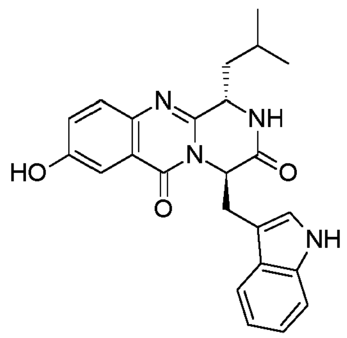

or,

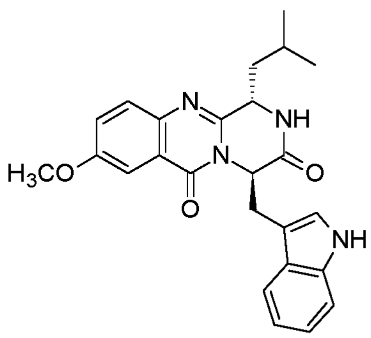 or, 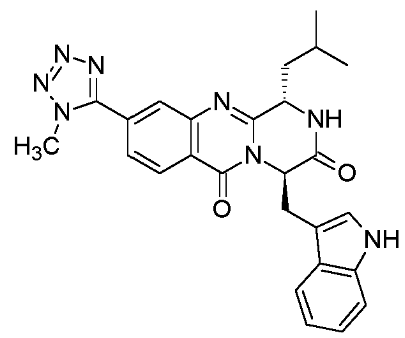

or,

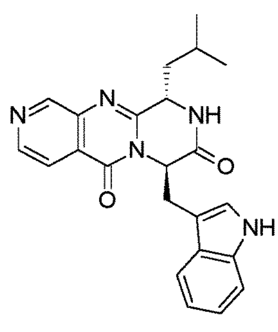 or, 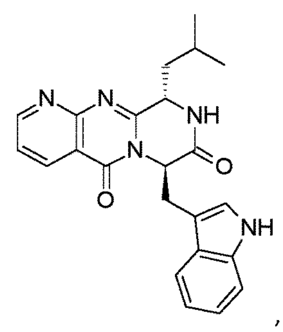 ,

preferably the compound may be

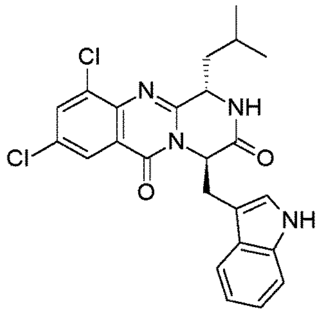

or

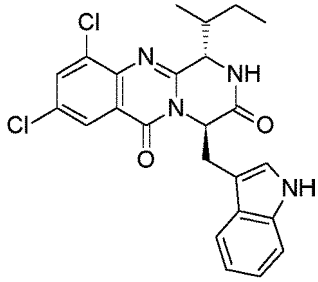

.

The present disclosure also relates to a compound for use in medicine. Preferably, the compound of formula I is

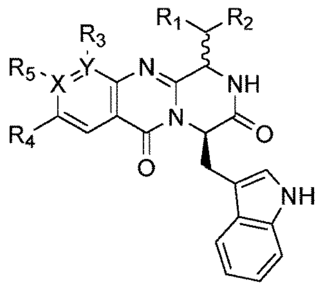

wherein

$R^1$, $R^2$, $R^3$, $R^4$, $R^5$, X and Y are independently selected from each other;
$R^1$ and $R^2$ are selected from H or $CH_3$ or $CH(CH_3)_2$ or $CH_2CH_3$,
$R^3$ is selected from H or Cl or Br or F or OH or $OCH_3$,
$R^4$ is selected from H or Cl or Br or I or F or OH or $OCH_3$;
$R^5$ is H or

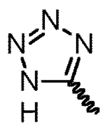

,

and
X and Y are selected from N or C;
or a pharmaceutically acceptable salt, or solvate thereof,
provided that
if X and Y are C then $R^4$ is different from H; or
if X and Y are C then $R^4$ is H and $R^5$ is

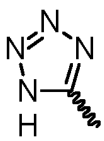

;

or
when X is N then $R^5$ is absent or
when Y is N then $R^3$ is absent
for use in the treatment or prevention of bacterial infections and/or for use in the treatment or prevention of malaria.

In an embodiment, the compounds may be selected from

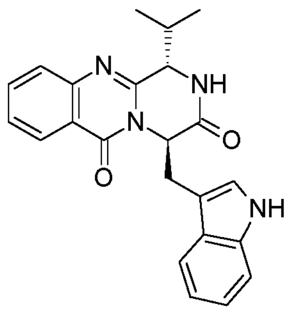

or,

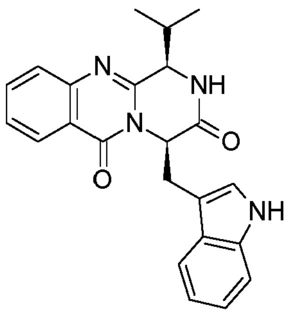 or, 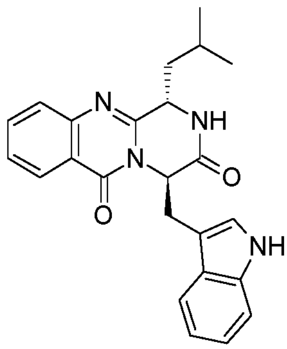 or, 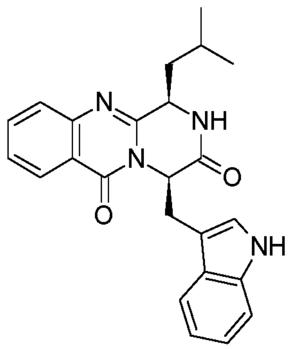

or,

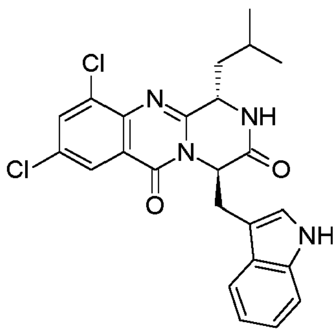

and it may be for use in the treatment or prevention of malaria.

In an embodiment, any of the compounds herein disclosed may be for use in the treatment of Gram-positive bacterial infections, preferably caused by *Staphylococcus* spp. and/or *Enterococcus* spp., more preferably caused by *Staphylococcus* aureus and/or *Enterococcus faecalis.*

In an embodiment, any of the compounds herein disclosed may be for use in the treatment of Gram-positive bacterial infections, preferably caused *by Staphylococcus aureus* and *Enterococcus faecalis,* wherein the compound may be

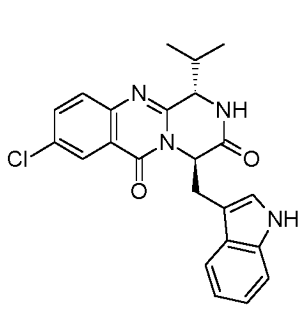 or, 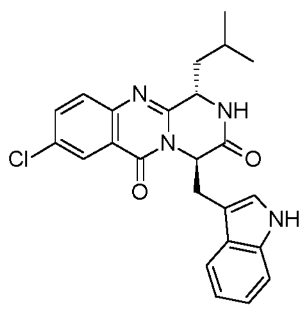

or,

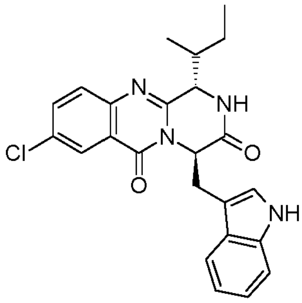

,

preferably wherein the compound may be

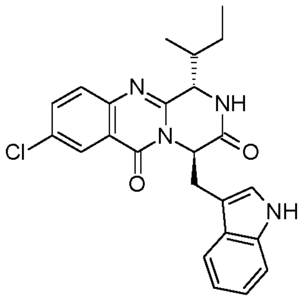

.

In an embodiment, any of the compounds herein disclosed may be for use in the treatment of Gram-positive bacterial infections, preferably caused by *Staphylococcus* aureus, wherein the compound may be

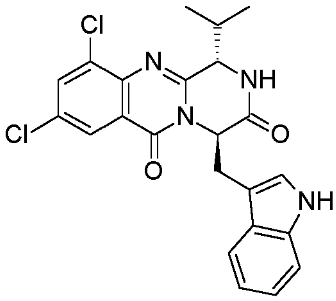

or,

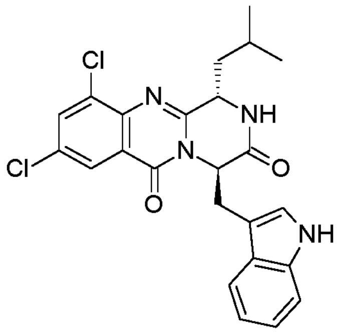

or,

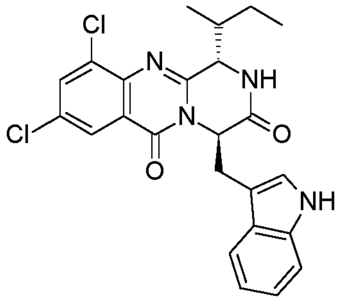

or,

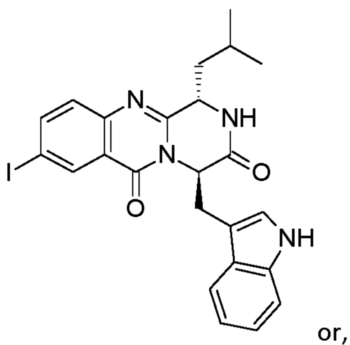

or,

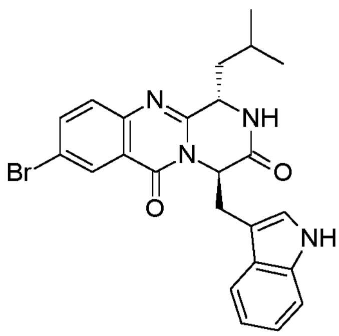

,

preferably wherein the compound may be

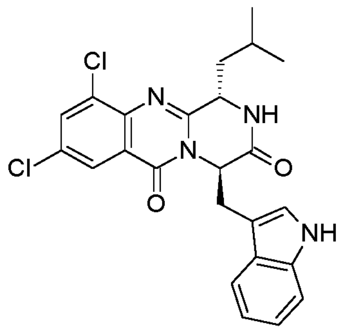

or

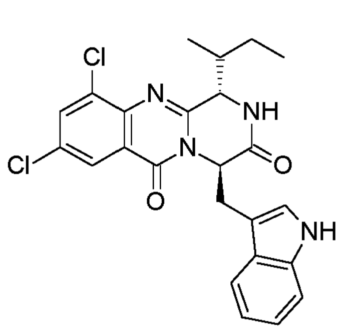

.

The present disclosure also relates to a composition comprising any of the compounds herein disclosed and a pharmaceutically acceptable excipient, wherein any of the compounds herein disclosed is in a therapeutically effective amount.

In an embodiment, the above-mentioned composition may further comprise an antibiotic, preferably wherein the antibiotic is a fluoroquinolone, preferably selected from ciprofloxacin, norfloxacin, pefloxacin, enofloxacin, ofloxacin, levofloxacin, moxifloxacin, or mixtures thereof.

## **Brief description** of **the drawings**

The following figures provide preferred embodiments for the present disclosure and should not be seen as limiting the scope of the disclosure.

**Figure 1.** Examples of marine antimicrobials.

**Figure 2.** Current antimalarial drugs **5** and **6,** indole containing antimalarial compounds **7** and **8,** natural antimalarial compound **9,** and the scaffold of the target compounds, indole-containing pyrazino[2,1-*b*]quinazoline-3,6-dione.

**Figure 3.** Structures of the third and fourth approaches of indole-containing pyrazino[2,1-b]quinazoline-3,6-diones synthesized.

**Figure 4.** Structure-activity relationship for antibacterial activity of the library of quinazolinones 10-37.

**Figure 5.** The inhibition of polimerization of hemozoin in vitro of compound **12, 16, 31,** and CQ (6). The error bars represent a mean ± SD.

**Figure 6.** Hemolysis of healthy erythrocytes *in vitro* induced by the compounds. The error bars represent the mean ± standard deviation of % hemolysis of the compounds relative to the positive control obtained by action of Triton® X-100.

**Figure 7. (A)** Ribbon representation of Prolyl-tRNA Synthetase (PRS) (PDB code: 4YDQ) with crystallographic HF and top scored test molecules **12, 13, 16,** and **31. (B)** Crystallographic HF; **(C) 13, (E) 12, 16,** and **31** docked into PRD active site. Relevant amino acids are represented in capped sticks and labeled. AMPPNP is represented as light gray sticks. Polar interactions are represented in light gray broken lines. Capped surface representation of PRS with docked conformations of (D) crystallographic HF and 13, and (F) crystallographic HF, **12, 16,** and **31.** Some PRS residues are omitted for simplification. Polar interactions are represented in light gray broken lines.

**Figure 8.** Separation performance on the amylose tris-3,5 dimethylphenylcarbamate phase for compounds **27** wherein $k_1$ =1.95, $\alpha$ =1.76 *Rs* =8.43(A analytical column; B semipreparative column), **28** wherein $k_1$ = 2.64, $\alpha$ =1.94 *Rs* =8.15 (C analytical column; D semipreparative column), and **31** wherein $k_1$ =5.60, $\alpha$ =1.75 *Rs* =11.69, wherein $k_1$ =1.95, $\alpha$ =1.76 *Rs* =8.43 (E analytical column; F semipreparative column) [a] Flow rate: 0.5 ml/min, loop 20 μL, detection: 254 nm, column: Lux® 5 μm Amylose-1, (250 x 4.6 mm), mobile phase hexane: EtOH, 90:10. k: retention factor, $\alpha$: enantioselective selectivity, *Rs*: resolution index; [b] Flow rate: 2 mL/min, loop 200 μL, loading ca. 1.5 mg/mL in hexane:EtOH (50:50), detection 254 nm, column: amylose tris-3,5-dimethylphenylcarbamate coated with Nucleosil (200 mm x 7 mm); mobile phase hexane: EtOH, 90:10.

## Detailed description

The present disclosure relates to antibacterial activity and/or to antimalarial activity of the compounds herein disclosed.

The compounds herein disclosed are synthetized using the approaches ($1^{st}$, $2^{nd}$, $3^{rd}$ and $4^{th}$ approaches) summarized in **Figure 3.**

The chemistry of compounds of the $1^{st}$ approach (compounds **10-17)** and $2^{nd}$ approach (compounds **19, 21, 23, 25 and 26)** is described in references 3 and 4. It was, however, surprisingly found that compounds of the $1^{st}$ and $2^{nd}$ approaches may have antimalarial activity, as it will be described below.

**Chemistry for the $3^{nd}$ approach.** The eleven new indolomethyl pyrazino[1,2-b]quinazoline-3,6-dione derivatives of the third approach were synthesized by a previously described approach using a microwave assisted multicomponent polycondensation of amino acids (Table 1). The coupling of halogenated commercial anthranilic acids **(47)** to *N*-protected L-α-amino acids **(48),** and further dehydrative cyclization using triphenyl phosphite [$(PhO)_3P$], generated the intermediates benzoxazin-4-ones **49** which, followed by the addition of D-tryptophan methyl ester **(50)** under microwave irradiation, furnished the desirable final products **27-37** (2-14 % yield) with partial epimerization (Table 1). Using this methodology only *anti* isomers were produced (1*S,* 4*R*) and the different side chains at C-1 were obtained by selecting diverse L-α amino acids - valine, leucine, and isoleucine. The purities of the compounds were determined by reversed-phase liquid chromatography, (RP-LC, C18, MeOH: $H_2O$; 50:50) and was found to be higher than 95 % while for compound **30** and **37** purities were of 90%.

**Table 1.** Synthesis of halogenated quinazolinone derivatives **27-37** [a]

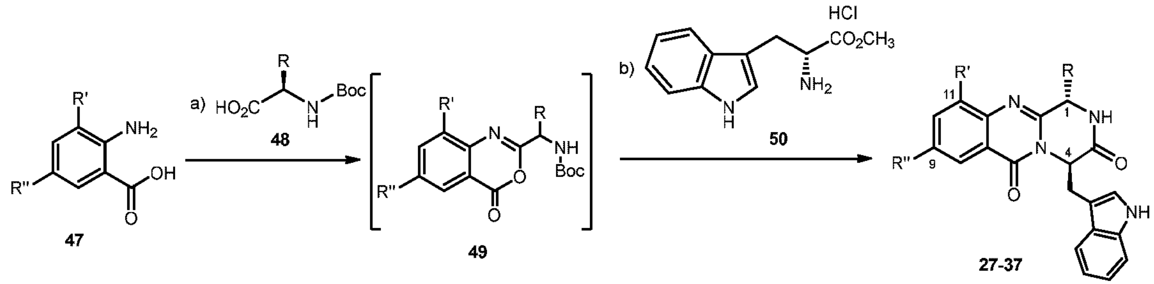


47, 48, 49, 50, 27-37

| Compound | R | R' | R" | Yield (%) | $[\alpha]_D$[b] | e.r.[c] | %[d] |
|---|---|---|---|---|---|---|---|
| **27** | *i*-Pr | Cl | H | 5 | -273 | 56 (27a):44 (27b) | 92 |
| **28** | *i*-Bu | Cl | H | 3 | +154 | 44 (28a):56 (28b) | 99 |
| **29** | *s*-Bu | Cl | H | 2 | +130 | 46:54 | 93 |
| **30** | *i*-Pr | Cl | Cl | 5 | +140 | 43:57 | 90 |
| **31** | *i*-Bu | Cl | Cl | 4.5 | -169 | 60 (31a):40 (31b) | >99 |
| **32** | *s*-Bu | Cl | Cl | 2.6 | -264 | 71:29 | >99 |
| **33** | *i*-Pr | I | H | 4.1 | -175 | 51:49 | 95 |
| **34** | *i*-Pr | Br | H | 1.2 | -170 | 50:50 | 95 |
| **35** | *i*-Bu | I | H | 11.8 | -165 | 51:49 | 98 |
| **36** | *i*-Bu | Br | H | 13.8 | -243 | 51:49 | 98 |
| **37**[e] | *i*-Bu | I | I | 3.5 | -229 | 54:46 | 90 |
| **38** | $CH_2C_6H_4OCH_2C_6H_5$ | Cl | Cl | 2.2 | +244 | 67:33 | 90 |

[a] Reaction conditions: a) dried-pyridine, $(PhO)_3P$, 55 °C, 16-24 h; b) dried-pyridine, $(PhO)_3P$, 220 °C, 1.5 min; [b] Optical rotation; [c] *e.r.* = enantiomeric ratio determined by enantiosselectiv LC (column: amylose, Lux® 5 μm Amylose-1, 250 x 4.6 mm, flow rate: 0.5 ml/min, mobile phase: hexane/EtOH, 9:1), numbers atributted with letters a and b correspond to the respective enantiomers [d] = % purity determined by RP-LC.
[e] Compound 37 is not an embodiment of the present invention.

In the present disclosure, the general conditions for the synthesis of compounds **27-37** is as follows. In a closed vial, 5-chloro anthranilic acid **(47** in which R'=H and R"=Cl, 34 mg, 200 $\mu$mol) for **27, 28,** and **29,** or 3,5-dichloro anthranilic acid, **(47 in** which R' and R"=Cl, 41 mg, 200 $\mu$mol) for **30, 31,** and **32,** or 5-iodoanthranilic acid, **(47** in which R'=H and R"=I, 53 mg, 200 $\mu$mol) for **33** and **35,** or 5-bromo anthranilic acid, **(47** in which R'=H and R"=Br, 43 mg, 200 $\mu$mol) for **34** and **36,** or 3,5-diodo anthranilic acid **(47** in which R' and R"=I, 78 mg, 200 $\mu$mol) for **37;** was added N-Boc-L-valine **(48** in which R=i-Pr, 44 mg, 200 $\mu$mol) for **27, 30, 33** and **34,** or N-Boc-L-leucine **(48 in** which R=i-Bu, 46 mg, 200 $\mu$mol) for **28, 31, 35,** and **37,** or *N*-Boc-L-isoleucine **(48** in which R=s-Bu, 46 mg, 200 $\mu$mol) for 29 and **32** (as present in **Table** 1), and triphenylphosphite (63 $\mu$L, 220 $\mu$mol) were added along with 1 mL of dried pyridine. The vial was heated in heating block with stirring at 55 °C for 16-24 h. After cooling the mixture to room temperature, D-tryptophan methyl ester hydrochloride (50, 51 mg, 200 $\mu$mol) was added, and the mixture was irradiated in the microwave at a constant temperature at 220 °C for 1.5 min. Four reaction mixtures were prepared in the same conditions and treated in parallel. After removing the solvent with toluene, the crude product was purified by flash column chromatography using hexane: EtOAc (60:40) as a mobile phase. The preparative TLC was performed using $CH_2Cl_2$:$Me_2CO$ (95:5) as mobile phase. The major compound appeared as a black spot with no fluorescence under the UV light. The desired compounds were collected as yellow solids. Before analysis, compounds were recrystallized from methanol.

In an embodiment, the characterization of (1S,4R)-4-((1H-indol-3-yl)methyl)-8-chloro-1-isopropyl-1,2-dihydro-6H-pyrazino[2,1-b]quinazoline-3,6(4H)-dione **(27)** is as follows: Yield: 39.8 mg, 7%; *e.r* = 56:44; mp: 200.3-202.4°C; $[\alpha]_D^{30}$ = - 273 ; (*c* 0.05); $CHCl_3$); $v_{max}$(KBr) 3277, 2924, 1682, 1592, 1470, 1323, 741 $cm^{-1}$; $^1H$ NMR (300 MHz, $CDCl_3$): $\delta$ 8. 33 (d, 1H, *J* = 2.5 Hz, CH), 8.33 (br, 1H, NH-indol), 7.70 (dd, 1H,*J* = 8.7 and 2.5 Hz, CH), 7.50 (d, *J* = 8.7 Hz, CH), 7.39 (d, 1H, *J* = 8.0 Hz, CH-Trp), 7.30 (d, *J* = 8.1 Hz, CH-Trp), 7.12 (t, 1H, *J* = 8.0 Hz, CH-Trp), 6.92 (t, 1H, *J* = 8.0 Hz, CH-Trp), 6.63 (d, 1H, *J* = 2.3 Hz, CH-Trp), 5.64 (dd, 1H, *J* = 5.4 and 2.7, CH*-Trp), 5.72 (s, 1H, NH-amide), 3.73 (dd, 1H, *J* = 15.0 and 2.7 Hz, $CH_2$-Trp), 3.63 (dd, 1H, *J* = 15.0 and 5.4 Hz, $CH_2$-Trp), 2.76 (d, *J* = 2.3 Hz, CH*-val), 2.60 (dtd, 1H, *J* = 13.9, 6.9, and 2.3 Hz, CH-val), 0.64 (d, 6H, *J* = 6.1 Hz, $CH_3$-val); $^{13}C$ NMR (75 MHz, $CDCl_3$): $\delta$ 169.2 (C=O), 159.9 (C=O), 150.6 (C=N), 145.6 (C), 136.1 (C-Trp 135.7 (CH), 132.8 (C), 128.9 (CH), 127.2 (C-Trp), 126.2 (CH), 123.6 (CH-Trp), 122.5 (CH-Trp), 121.2 (C), 119.9 (CH-Trp), 118.6 (CH-Trp), 111.1 (CH-Trp), 109.1 (C-Trp), 57.0 (CH*-Trp), 58.0 (CH*-val), 29.3 (CH-val), 27.3 ($CH_2$-Trp), 18.8 ($CH_3$-val), 14.8 ($CH_3$-val); (+)-HRMS-ESI *m/z:* 421.1442 $(M + H)^+$, 443.1264 $(M + Na)^+$ (calculated for $C_{23}H_{22}N_4O_2Cl$, 421.1432; $C_{23}H_{21}N_4O_2ClNa$, 443.1252).

In an embodiment, the characterization of (1*S*,4*R*)-4-((1*H*-indol-3-yl)methyl)-8-chloro-1-isobutyl-1,2-dihydro-6*H*-pyrazino[2,1-b]quinazoline-3,6(4H)-dione **(28)** is as follows: Yield: 12.3 mg, 3%; *e.r* = 44:56; mp: 208.8-210.1°C; $[\alpha]_D^{30}$ = +154 (*c* 0.15; $CHCl_3$); $v_{max}$ (KBr) ) 3277, 2924, 1682, 1592, 1470, 1323, 741 $cm^{-1}$; $^1H$ NMR (300 MHz, $CDCl_3$): $\delta$ 8. 33 (d, 1H, J = 2.4 Hz, CH), 8.07 (br, 1H, NH-indol), 7.70 (dd, 1H,*J* = 8.7 and 2.4 Hz, CH), 7.54 (d, *J* = 8.7 Hz, CH), 7.46 (d, 1H, *J* = 7.8 Hz, CH-Trp), 7.29 (d, *J* = 7.8 Hz, CH-Trp), 7.13 (t, 1H, *J* = 7.8 Hz, CH-Trp), 6.98 (t, 1H, *J* = 7.8 Hz, CH-Trp), 6.65 (d, 1H, *J* = 2.4 Hz, CH-Trp), 5.65 (dd, 1H, *J* = 5.3 and 2.7, CH*-Trp), 5.71 (s, 1H, NH-amide), 3.76 (dd, 1H, *J* = 15.1 and 2.7 Hz, $CH_2$-Trp), 3.63 (dd, 1H, *J* = 15.1 and 5.3 Hz, $CH_2$-Trp), 2.70 (dd, *J* = 9.7 and 2.3 Hz, CH*-Leu), 1.97 (ddd, 1H, *J* = 11.8, 7.7, and 2.1 Hz, CH-Leu), 1.39-1.30 (m, 2H, $CH_2$-Leu), 0.77 (d, 3H, *J* = 6.4 Hz, $CH_3$-Leu), 0.28 (d, 3H, *J* = 6.5 Hz, $CH_3$-Leu); $^{13}C$ NMR (75 MHz, $CDCl_3$): $\delta$ 169.1 (C=O), 159.8 (C=O), 151.9 (C=N), 145.5 (C), 136.0 (C-Trp 135.1 (CH), 132.9 (C), 129.1 (CH), 127.2 (C-Trp), 126.2 (CH), 123.6 (CH-Trp), 122.7 (CH-Trp), 121.2 (C), 120.2 (CH-Trp), 118.7 (CH-Trp), 111.1 (CH-Trp), 109.5 (C-Trp), 57.5 (CH*-Trp), 50.8 (CH*-Leu), 40.2 ($CH_2$-Leu), 27.2 ($CH_2$-Trp), 24.1 (CH-Leu), 23.3 ($CH_3$-Leu), 19.7 ($CH_3$-Leu); (+)-HRMS-ESI m/z: 435.1579 $(M + H)^+$, 457.1206 $(M + Na)^+$ (calculated for $C_{24}H_{24}N_4O_2Cl$, 435.1588; $C_{24}H_{23}N_4O_2ClNa$, 457.1408).

In an embodiment, the characterization of (1*S*,4*R*)-4-((1iH-indol-3-yl)methyl)-1-((S)-sec-butyl)-8-chloro-1,2-dihydro-6H-pyrazino[2,1-*b*]quinazoline-3,6(4H)-dione **(29)** is as follows: Yield: 16.7 mg, 3%; *e.r* = 46:54; mp: 209.1-211.2 °C; $[\alpha]_D^{30}$ = +130 (c 0.03 $CHCl_3$); $v_{max}$ (KBr) 3277, 2924, 1682, 1592, 1470, 1323, 741 $cm^{-1}$; $^1H$ NMR (300 MHz, $CDCl_3$): $\delta$ 8. 33 (d, 1H, *J* = 2.4 Hz, CH), 8.05 (br, 1H, NH-indol), 7.70 (dd, 1H,*J* = 8.7 and 2.4 Hz, CH), 7.49 (d, *J* = 8.7 Hz, CH), 7.38 (d, 1H, J = 8.0 Hz, CH-Trp), 7.29 (d, *J* = 8.0 Hz, CH-Trp), 7.13 (t, 1H, *J* = 8.0 Hz, CH-Trp), 6.92 (t, 1H, *J* = 8.0 Hz, CH-Trp), 6.63 (d, 1H, *J* = 2.4 Hz, CH-Trp), 5.64 (dd, 1H, *J* = 5.3 and 2.8, CH*-Trp), 5.80 (s, 1H, NH-amide), 3.72 (dd, 1H, *J* = 15.1 and 2.8 Hz, $CH_2$-Trp), 3.62 (dd, 1H, *J* = 15.1 and 5.3 Hz, $CH_2$-Trp), 2.69 (d, *J* = 2.2 Hz, CH*-Ile), 2.29 (ddd, 1H, *J* = 11.6, 7.7, and 4.8 Hz, CH*-Ile), 0.99-0.79 (m, 2H), 0.70 (d, 3H, *J* = 7.7 Hz, $CH_3$-Ile), 0.63 (d, 3H, *J* = 7.2 Hz, $CH_3$-Ile); $^{13}C$ NMR (75 MHz, $CDCl_3$): $\delta$ 169.1 (C=O), 159.9 (C=O), 150.7 (C=N), 145.5 (C), 136.0 (C-Trp 135.1 (CH), 132.8 (C), 128.9 (CH), 127.2 (C-Trp), 126.2 (CH), 123.5 (CH-Trp), 122.7 (CH-Trp), 121.1 (C), 120.1 (CH-Trp), 118.6 (CH-Trp), 111.1 (CH-Trp), 109.2 (C-Trp), 58.3 (CH*-Ile), 57.0 (CH*-Trp), 36.2 (CH-Leu), 27.3 ($CH_2$-Trp), 23.1 ($CH_2$-Ile), 15.6 ($CH_3$-Ile), 12.0 ($CH_3$-Ile); (+)-HRMS-ESI *m/z:* 435.1580 $(M + H)^+$, 457.1394 $(M + Na)^+$ (calculated for $C_{24}H_{24}N_4O_2Cl$, 434.1588; $C_{24}H_{23}N_4O_2ClNa$, 457.1408).

In an embodiment, the characterization of (1*S*,4*R*)-4-((1H-indol-3-yl)methyl)-8,10-dichloro-1-isopropyl-1,2-dihydro-6*H*-pyrazino[2,1-b]quinazoline-3,6(4H)-dione **(30)** is as follows: Yield: 22.1 mg, 5%; *e.r* = 43:57; mp: 232.9-235.1

°C; $[\alpha]_D^{30}$ = +140 ($c$ 0.038; $CHCl_3$); $v_{max}$ (KBr) 3293, 2954, 1671, 1611, 1511, 1465, 1240, 772, and 697 $cm^{-1}$; $^1H$ NMR (300 MHz, DMSO-$d_6$): $\delta$ 10.2 (br, 1H, NH-indol), 8. 20 (d, 1H, $J$ = 2.4 Hz, CH), 7.83 (d, 1H, $J$ = 2.4 Hz, CH), 7.37 (d, 1H, $J$ = 8.1 Hz, CH-Trp), 7.33 (d, $J$ = 8.1 Hz, CH-Trp), 7.11 (s, 1H, NH-amide), 7.07 (t, 1H, $J$ = 7.6 Hz, CH-Trp), 6.87 (t, 1H, $J$ = 7.6 Hz, CH-Trp), 6.66 (d, 1H, J = 2.3 Hz, CH-Trp), 5.50 (dd, 1H, $J$ = 5.3 and 2.9, CH*-Trp), 3.69 (dd, 1H, $J$ = 14.9 and 2.9 Hz, $CH_2$-Trp), 3.58 (dd, 1H, $J$ = 14.9 and 5.3 Hz, $CH_2$-Trp), 2.76 (d, $J$ = 2.2 Hz, CH*-val), 2.60- 254 (m, 1H, CH-val), 0.71 (dd, 6H, $J$ = 8.4 and 7.2 Hz, $CH_3$-val); $^{13}C$ NMR (75 MHz, $CDCl_3$): $\delta$ 169.2 (C=O), 159.9 (C=O), 150.6 (C=N), 145.7 (C), 136.0 (C-Trp), 135.1 (CH), 132.8 (C), 128.9 (CH), 127.2 (C-Trp), 126.2 (CH), 123.6 (CH-Trp), 122.7 (CH-Trp), 121.2 (C), 120.1 (CH-Trp), 118.6 (CH-Trp), 111.1 (CH-Trp), 109.2 (C-Trp), 58.1 (CH*-val), 57.0 (CH*-Trp), 29.3 (CH-val), 27.3 ($CH_2$-Trp), 18.8 ($CH_3$-val), 14.8 ($CH_3$-val; (+)-HRMS-ESI m/z: 455.1436 (M + H)$^+$ (calculated for $C_{23}H_{21}N_4O_2Cl_2$, 455.1041).

In an embodiment, the characterization of (1$S$,4$R$)-4-((1$H$-indol-3-yl)methyl)-8,10-dichloro-1-isobutyl-1,2-dihydro-6$H$-pyrazino[2,1-b]quinazoline-3,6(4H)-dione **(31)** is as follows: Yield: 41.8 mg, 4.5%; *e.r* = 60:40; mp: 253.4-254.3 °C; $[\alpha]_D^{30}$ = -169 ($c$ 0.04; $CHCl_3$); $v_{max}$(KBr) 3289, 2960, 1680, 1600, 1556, 1315, 757, 720 $cm^{-1}$; $^1H$ NMR (300 MHz, DMSO-$d_6$): 10.22 ( br, 1H, NH-indol), $\delta$ 8.13 (d, 1H, $J$ = 2.4 Hz, CH), 7.75 (d, 1H, $J$ = 2.4 Hz, CH), 7.33 (d, 1H, $J$ = 8.0 Hz, CH-Trp), 7.25 (d, $J$ = 8.0 Hz, CH-Trp), 7.19 (br, NH-amide), 7.00 (t, 1H, $J$ = 8.0 Hz, CH-Trp), 6.82 (t, 1H, $J$ = 8.0 Hz, CH-Trp), 6.60 (d, 1H, $J$ = 2.4 Hz, CH-Trp), 5.42 (dd, 1H, $J$ = 5.4 and 2.9, CH*-Trp) , 3.63 (dd, 1H, $J$ = 15.0 and 2.9 Hz, $CH_2$-Trp), 3.50 (dd, 1H, $J$ = 15.0 and 5.4 Hz, $CH_2$-Trp), 2.68 (dd, $J$ = 7.3 and 4.9 Hz, CH*-Leu), 1.94-1.86 (m, 1H $CH_2$-Leu), 1.50 (tt, 1H, $J$ = 13.2 and 6.5 Hz, CH-Leu), 1.29-1.22 (m, 1H, $CH_2$-Leu), 0.56 (d, 3H, $J$ = 6.6 Hz, $CH_3$-Leu), 0.35 (d, 3H, J = 6.6 Hz, $CH_3$-Leu); $^{13}C$ NMR (75 MHz,DMSO-$d_6$): $\delta$ 168.4 (C=O), 158.8 (C=O), 152.8 (C=N), 142.0 (C), 135.9 (C-Trp), 134.1 (CH), 132.6 (C), 131.4 (C), 126.5 (C-Trp), 124.3 (CH), 123.5 (CH-Trp), 121.6 (CH-Trp), 121.5 (C), 118.9 (CH-Trp), 117.7 (CH-Trp), 111.1 (CH-Trp), 107.7 (C-Trp), 57.3 (CH*-Trp), 50.6 (CH*-Leu), 39.6 ($CH_2$-Leu), 26.2 ($CH_2$-Trp), 23.8 (CH-Leu), 22.1 ($CH_3$-Leu), 20.5 ($CH_3$-Leu); (+)-HRMS-ESI m/z: 469.1186 (M + H)$^+$, 491.1008 (M + Na)$^+$ (calculated for $C_{24}H_{23}N_4O_2Cl_2$, 469.1198; $C_{24}H_{22}N_4O_2Cl_2Na$, 491.1018).

In an embodiment, the characterization of (1S,4R)-4-((1H-indol-3-yl)methyl)-1-((S)-sec-butyl)-8,10-dichloro-1,2-dihydro-6H-pyrazino[2,1-b]quinazoline-3,6(4H)-dione **(32)** is as follows: Yield: 22.4 mg, 2.6%; *e.r* = 71:29; mp: 252.9-254.7 °C; $[\alpha]_D^{30}$ = -264 ($c$ 0.034; $CHCl_3$); $v_{max}$ (KBr) 3373, 3074, 2922, 1698, 1609, 1550, 1450, 1262, 794 $cm^{-1}$; $^1H$ NMR (300 MHz, $CDCl_3$): $\delta$ 8. 33 (d, 1H, J = 2.4 Hz, CH), 8.05 (br, 1H, NH-indol), 7.70 (d, 1H, $J$ = 2.4 Hz, CH), 7.38 (d, 1H, $J$ = 7.9 Hz, CH-Trp), 7.29 (d, $J$ = 7.9 Hz, CH-Trp), 7.13 (t, 1H, $J$ = 7.9 Hz, CH-Trp), 6.92 (t, 1H, $J$ = 7.9 Hz, CH-Trp), 6.63 (d, 1H, $J$ = 2.4 Hz, CH-Trp), 5.64 (dd, 1H, $J$ = 5.3 and 2.8, CH*-Trp), 5.80 (s, 1H, NH-amide), 3.72 (dd, 1H, $J$ = 15.0 and 2.8 Hz, $CH_2$-Trp), 3.62 (dd, 1H, $J$ = 15.0 and 5.3 Hz, $CH_2$-Trp), 2.69 (d, $J$ = 2.2 Hz, CH*-Ile), 2.29 (ddd, 1H, $J$ = 11.6, 7.9, and 4.8 Hz, CH*-Ile), 0.99-0.79 ( m, 2H, $CH_2$-Ile), 0.70 (d, 3H, $J$ = 7.3 Hz, $CH_3$-Ile), 0.63 (d, 3H, $J$ = 7.3 Hz, $CH_3$-Ile); $^{13}C$ NMR (75 MHz, $CDCl_3$): $\delta$ 168.9 (C=O), 159.5 (C=O), 151.3 (C=N), 142.5 (C), 136.1 (C-Trp) 135.0 (CH), 133.2 (C), 132.4 (C), 127.1 (C-Trp), 125.1 (CH), 123.5 (CH-Trp), 122.9 (CH-Trp), 122.1 (C), 120.2 (CH-Trp), 118.6 (CH-Trp), 111.1 (CH-Trp), 109.2 (C-Trp), 58.2 (CH*-Ile), 57.3 (CH*-Trp), 36.2 (CH-Leu), 27.1 ($CH_2$-Trp), 23.6 ($CH_2$-Ile), 15.5 ($CH_3$-Ile), 12.1 ($CH_3$-Ile; (+)-HRMS-ESI $m/z$: 469.1186 (M + H)$^+$, 491.1024 (M + Na)$^+$ (calculated for $C_{24}H_{23}N_4O_2Cl_2$, 469.1198; $C_{24}H_{22}N_4O_2Cl_2Na$, 491.1018).

In an embodiment, the characterization of (1$S$,4$R$)-4-((1$H$-indol-3-yl)methyl)-8-iodo-1-isopropyl-1,2-dihydro-6$H$-pyrazino[2,1-b]quinazoline-3,6(4$H$)-dione **(33)** is as follows: Yield: 21.2 mg, 4.1%; *e.r* = 51:49; mp: 246.5-248.2 °C; $[\alpha]_D^{30}$ = -175 ($c$ 0.041 $CHCl_3$); $v_{max}$ (KBr) 3311, 3192, 2963, 1681, 1655, 1588, 1464, 1246, 828, and 741 $cm^{-1}$; $^1H$ NMR (300 MHz, $CDCl_3$): $\delta$ 8. 71 (d, 1H, $J$ = 2.4 Hz, CH), 8.04 (br, 1H, NH-indol), 8.02 (dd, 1H, $J$ = 8.6 and 2.1 Hz, CH), 7.41 (d, 1H, $J$ = 8.0 Hz, CH-Trp), 7.30 (d, $J$ = 8.4 Hz, CH) 7.29 (d, $J$ = 8.4 Hz, CH-Trp), 7.13 (ddd, 1H, $J$ = 8.0, 7.1 and 0.9 Hz, CH-Trp), 6.94 (ddd, 1H, $J$ = 8.0, 7.1 and 0.9 Hz, CH-Trp), 6.61 (d, 1H, $J$ = 2.4 Hz, CH-indol), 5.64 (dd, 1H, $J$ = 5.4 and 2.8, CH*-Trp), 5.67 (s, 1H, NH-amide), 3.73 (dd, 1H, $J$ = 14.9 and 2.7 Hz, $CH_2$-Trp), 3.61 (dd, 1H, $J$ = 15.1 and 5.4 Hz, $CH_2$-Trp), 2.64 (d, $J$ = 2.4 Hz, CH*-val), 2.63-2.56 (m, 1H, CH-val), 0.63 (d, 6H, $J$ = 6.8 Hz, $CH_3$-val); $^{13}C$ NMR (75 MHz, $CDCl_3$): δ 169.1 (C=O), 159.5 (C=O), 151.0 (C=N), 146.3 (C), 143.5 (CH), 136.0 (C-Trp 135.7 (CH), 129.0 (CH), 127.2 (C-Trp), 123.5 (CH-indol), 122.7 (CH-Trp), 121.7 (C), 120.2 (CH-Trp), 118.7 (CH-Trp), 111.1 (CH-Trp), 109.3 (C-indol), 91.4 (C), 58.1 (CH*-val), 57.0 (CH*-Trp) 29.7 (CH-val), 27.3 ($CH_2$-Trp), 18.8 ($CH_3$-val), 14.8 ($CH_3$-val); (+)-HRMS-ESI $m/z$: 513.0778 (M + H)$^+$ (calculated for $C_{23}H_{22}N_4O_2I$, 513.0787).

In an embodiment, the characterization of (1$S$,4$R$)-4-((1$H$-indol-3-yl)methyl)-8-bromo-1-isopropyl-1,2-dihydro-6H-pyrazino[2,1-b]quinazoline-3,6(4H)-dione (34) is as follows: Yield: 10.9 mg, *1.2%; e.r* = 50:50; mp: 236.5-238.0°C; $[\alpha]_D^{30}$ = -170 ($c$ 0.03; $CHCl_3$); $v_{max}$ (KBr) 3292, 3193, 2958, 1681, 1666, 1592, 1466, 1237, 832, and 742 $cm^{-1}$; $^1H$ NMR (300 MHz, $CDCl_3$): $\delta$ 8. 50 (d, 1H, $J$ = 2.2 Hz, CH), 8.05 (br, 1H, NH-indol), 7.84 (dd, 1H, $J$ = 8.7 and 2.2 Hz, CH), 7.41 (, $J$ = 8.7 Hz, CH), 7.29 (dd, 2H, $J$ = 8.0 and 2.2 Hz, CH-Trp (2)), 7.13 (ddd, 1H, $J$ = 8.0, 7.1 and 1.0 Hz, CH-Trp), 6.93 (ddd, 1H, $J$ = 8.0, 7.1 and 1.1 Hz, CH-Trp), 6.62 (d, 1H, $J$ = 2.4 Hz, CH-Trp), 5.64 (dd, 1H, $J$ = 5.4 and 2.8, CH*-Trp), 5.63 (s, 1H, NH-amide), 3.73 (dd, 1H, $J$ = 14.9 and 2.8 Hz, $CH_2$-Trp), 3.62 (dd, 1H, $J$ = 15.0 and 5.4 Hz, $CH_2$-Trp), 2.66 (d, $J$ = 2.4 Hz, CH*-val), 2.60 (m, 1H, CH-val), 0.65 (d, 3H, $J$ = 6.5 Hz, $CH_3$-val), 0.63 (d, 3H, $J$ = 6.4 Hz, $CH_3$-val); $^{13}C$ NMR (75 MHz, $CDCl_3$): $\delta$ 169.1 (C=O), 159.7 (C=O), 150.9 (C=N), 145.9 (C), 138.1 (CH), 136. (C-Trp), 129.4 (C), 129.1 (CH), 127.2 (C-trp), 123.5 (CH-indol), 122.7 (CH-Trp), 121.5 (C), 120.6 (CH-Trp), 120.2 (C), 118.7 (CH-Trp), 111.1 (CH-Trp), 109.3 (C-Trp), 57.0 (CH*-trp), 53.8 (CH*-val), 29.7 (CH-val), 27.3 ($CH_2$-Trp), 18.8 ($CH_3$-val), 14.8 ($CH_3$-val); (+)-HRMS-ESI *m/z:* 465.0987 (M + H)$^+$, 487.0726 (M + Na)$^+$ (calculated for $C_{23}H_{22}N_4O_2Br$: 465.0926; $C_{23}H_{21}N_4O_2BrNa$: 487.0746).

In an embodiment, the characterization of (1S,4R)-4-((1*H*-indol-3-yl)methyl)-8-iodo-1-isobutyl-1,2-dihydro-6*H*-pyrazino[2,1-b]quinazoline-3,6(4H)-dione **(35)** is as follows: Yield: 62.4 mg, 11.8%; *e.r* = 51:49; mp: 192.1-194.3 °C; $[\alpha]_D^{30}$ = -165 (*c* 0.038; $CHCl_3$); $v_{max}$ (KBr) 3318, 2956, 1671, 1686, 1593, 1464, 1247, 790, and 740 cm$^{-1}$; $^1H$ NMR (300 MHz, $CDCl_3$): $\delta$ 8. 70 (d, 1H, $J$ = 2.1 Hz, CH), 8.03 (br, 1H, NH-indol), 8.03 (dd, 1H, $J$ = 8.6 and 2.1 Hz, CH), 7.44 (d, $J$ = 7.9 Hz, CH-Trp), 7.33 (d, 1H, $J$ = 8.6 Hz, CH), 7.29 (d, $J$ = 7.9 Hz, CH-Trp), 7.13 (t, 1H, $J$ = 7.9 Hz, CH-Trp), 6.98 (t, 1H, $J$ = 7.9 Hz, CH-Trp), 6.68 (d, 1H, $J$ = 2.4 Hz, CH-Trp), 5.96 (s, 1H, NH-amide), 5.65 (dd, 1H, $J$ = 5.2 and 2.8, CH*-Trp) , 3.76 (dd, 1H, $J$ = 15.0 and 2.8 Hz, $CH_2$-Trp), 3.63 (dd, 1H, J = 15.0 and 5.2 Hz, $CH_2$-Trp), 2.69 (dd, $J$ = 9.6 and 3.3 Hz, CH*-Leu), 2.02-1.92 (m, 1H, CH-Leu), 1.40-1.30 ( m, 2H, $CH_2$-Leu), 0.79 (d, 3H, $J$ = 6.5 Hz, $CH_3$-Leu), 0.29 (d, 3H, $J$ = 6.4 Hz, $CH_3$-Leu); $^{13}C$ NMR (75 MHz, $CDCl_3$): $\delta$ 169.5 (C=O), 159.4 (C=O), 152.1 (C=N), 146.3 (C), 143.4 (CH), 136.1 (C-Trp), 135.7 (C), 129.2 (CH), 127.1 (C-Trp), 123.5 (CH-Trp), 122.9 (CH-Trp), 121.8 (C), 120.4 (CH-Trp), 118.7 (CH-Trp), 111.2 (CH-Trp), 109.5 (C-Trp), 91.5 (C), 57.4 (CH*-Trp), 51.0 (CH*-Leu), 40.1 ($CH_2$-Leu), 27.1 ($CH_2$-Trp), 24.1 (CH-Leu), 23.3 ($CH_3$-Leu), 19.7 ($CH_3$-Leu); (+)-HRMS-ESI *m/z*: 527.0936 (M + H)$^+$, 549.0748 (M + Na)$^+$ (calculated for $C_{24}H_{24}N_4O_2I$, 527.0944; $C_{24}H_{23}N_4O_2INa$, 549.0764).

In an embodiment, the characterization of (1*S*,4*R*)-4-((1*H*-indol-3-yl)methyl)-8-bromo-1-isobutyl-1,2-dihydro-6H-pyrazino[2,1-b]quinazoline-3,6(4*H*)-dione **(36)** is as follows: Yield: 64.6 mg, 13.8%; *e.r* = 51:49; mp: 227.0-228.2 °C; $[\alpha]_D^{30}$ = - 243 (*c* 0.037; $CHCl_3$); $v_{max}$ (KBr) 3284, 2959, 1686, 1658, 1599, 1433, 1245, 746, and 684 cm$^{-1}$; $^1H$ NMR (300 MHz, $CDCl_3$): $\delta$ 8. 50 (d, 1H, $J$ = 2.3 Hz, CH), 8.06 (br, 1H, NH-indol), 7.84 (dd, 1H, $J$ = 8.5 and 2.3 Hz, CH), 7.47 (dd, 2H, $J$ = 8.1 and 1.9 Hz, CH-Trp (2)), 7.29 (d, $J$ = 8.5 Hz, CH-Trp), 7.14 (t, 1H, $J$ = 8.0 Hz, CH-Trp), 6.92 (t, 1H, $J$ = 7.9 Hz, CH-Trp), 6.65 (d, 1H, $J$ = 2.4 Hz, CH-Trp), 5.65 (dd, 1H, $J$ = 5.2 and 2.9, CH*-Trp), 5.71 (s, 1H, NH-amide), 3.76 (dd, 1H, $J$ = 15.0 and 2.9 Hz, $CH_2$-Trp), 3.63 (dd, 1H, $J$ = 15.0 and 5.2 Hz, $CH_2$-Trp), 2.70 (dd, $J$ = 9.7 and 3.3 Hz, CH*-Leu), 2.07-1.89 (m, 1H, CH-Leu), 1.38-1.21 ( m, 2H, $CH_2$-Leu), 0.77 (d, 3H, $J$ = 6.3 Hz, $CH_3$-Leu), 0.28 (d, 3H, $J$ = 6.5 Hz, $CH_3$-Leu); $^{13}C$ NMR (75 MHz, $CDCl_3$): $\delta$ 169.1 (C=O), 159.7 (C=O), 152.0 (C=N), 145.8 (C), 137.8 (CH), 136.8 (C-Trp), 129.4 (C), 129.2 (CH), 127.1 (C-Trp), 123.8 (CH-Trp), 122.9 (CH-Trp), 121.6 (C), 120.6 (CH), 120.4 (CH-Trp), 118.7 (CH-Trp), 111.2 (CH-Trp), 109.6 (C-Trp), 57.5 (CH*-Trp), 50.8 (CH*-Leu), 40.1 ($CH_2$-Leu), 27.1 ($CH_2$-Trp), 24.1 (CH-Leu), 23.3 ($CH_3$-Leu), 19.7 ($CH_3$-Leu); (+)-HRMS-ESI *m/z:* 479.1086 (M + H)$^+$, 501.0912 (M + Na) $^+$ (calculated for $C_{24}H_{24}N_4O_2Br$, 479.1082; $C_{24}H_{23}N_4O_2BrNa$, 501.0900).

In an embodiment, the characterization of (1*S*,4*R*)-4-((1H-indol-3-yl)methyl)-8,10-diiodo-1-isobutyl-1,2-dihydro-6H-pyrazino[2,1-*b*]quinazoline-3,6(4H)-dione **(37)** is as follows: Yield: 22.5 mg, 3.5%; *e.r* = 54:46; mp: 242.8-243.8 °C; $[\alpha]_D^{30}$ = -229 (*c* 0.032; $CHCl_3$); $v_{max}$ (KBr) 3313, 2955, 1681, 1599, 1462, 1261, 772, and 669 cm$^{-1}$; $^1H$ NMR (300 MHz, DMSO-$d_6$): 10.17 ( br, 1H, NH-indol), $\delta$ 8. 62 (d, 1H, $J$ = 1.9 Hz, CH), 8.55 (d, 1H, $J$ = 1.9 Hz, CH), 7.41 (d, 1H, $J$ = 8.0 Hz, CH-Trp), 7.33 (d, $J$ = 8.0 Hz, CH-Trp), 7.11 (br, NH-amide), 7.09 (t, 1H, $J$ = 7.9 Hz, CH-Trp), 6.91 (t, 1H, $J$ = 7.9 Hz, CH-Trp), 6.68 (d, 1H, $J$ = 2.3 Hz, CH-indol), 5.50 (dd, 1H, $J$ = 5.2 and 2.9, CH*-Trp) , 3.72 (dd, 1H, $J$ = 14.9 and 2.9 Hz, $CH_2$-Trp), 3.58 (dd, 1H, $J$ = 15.0 and 5.2 Hz, $CH_2$-Trp), 2.75 (dd, $J$ = 6.6 and 5.3 Hz, CH*-Leu), 2.11-1\95 (m, 1H, $CH_2$-Leu), 1.68-1.53 (m, 1H $CH_2$-Leu), 1.38-1.23 (m, 1H, $J$ = 13.2 and 6.5 Hz, $CH_2$-Leu), 0.62 (t, 3H, $J$ = 6.5 Hz, $CH_3$-Leu), 0.47 (d, 3H, $J$ = 6.6 Hz, $CH_3$-Leu); $^{13}C$ NMR (75 MHz,DMSO-$d_6$): $\delta$ 168.4 (C=O), 162.0 (C=O), 153.0 (C=N), 151.1 (C), 136.2 (C-Trp), 135.9 (C), 127.1 (C-Trp), 123.4 (CH-Trp), 121.8 (C), 121.5 (CH-Trp), 119.0 (CH-Trp), 117.8 (CH-Trp), 111.0 (CH-Trp), 107.8 (C-Trp), 91.5 (CH), 89.2 (CH), 57.4 (CH*-Trp), 50.5 (CH*-Leu), 39.4 ($CH_2$-Leu), 26.3 ($CH_2$-Trp), 23.9 (CH-Leu), 21.8 ($CH_3$-Leu), 20.6 ($CH_3$-Leu); (+)-HRMS-ESI *m/z:* 652.9915 (M + H)$^+$, 674.9746 (M + Na)$^+$ (calculated for $C_{24}H_{23}N_4O_2Cl_2$, 652.9910; $C_{24}H_{22}N_4O_2Cl_2Na$, 674.9730).

The quantitative analysis of enantioselective liquid chromatography was carried out as follows. Compounds **27-37** were prepared using HPLC grades *n*-hexane:EtOH (50:50) at a final concentration 50 μg/mL. The HPLC system comprised a JASCO model 880-PU intelligent HPLC pump (JASCO corporation, Tokyo, Japan), equipped with a 7125 injector (Rheodyne LCC, Rohnert Park, CA, USA) fitted with a 20 μL LC loop, a JASCO model 880-30 solvent mixer involving a 875-UV intelligent UV/VIS detector, a system equipped with a chiral column (Lux® 5 μm Amylose-1, 250 × 4.6 mm). The data acquisition was performed using ChromNAC chromatography Data system (version 1.19.1) from JASCO Corporation (Tokyo, Japan). The mobile phase consisted of the mixture of n-hexane:EtOH (90:10, v/v), at a flow rate of 0.5 mL/min. The mobile phase was prepared in a volume/volume ratio and degassed in an ultrasonic bath for at least 15 min before use. The chromatographic analyses were carried out in isocratic mode at 22 ± 2 °C, in duplicate. The UV detection was performed at a wavelength of 254 nm. The volume void time was considered to be equal to the peak of solvent front and was taken from each particular run. The enantiomeric ratio (e.r) were determined by the mean percentage of peak area of eluted peaks.

The semipreparative enantioselective resolution was as follows. Compound **27, 28** and **31** were prepared in the mixture of HLCP grade solvent n-hexane:EtoH (50:50) at the concentration 10 mg/mL, and the injection volume was 100-200 μL. The HPLC system is similar to what described in quantitative analysis equipped with an in-house column amylose tris-3,5-dimethylphenylcarbamate coated with Nucleosil (500 A, 7mm, 20%, w/w) packed into a stainless-steel (200 mm x 7 mm I.D. size) column, prepared in the UFSCar laboratory. Semipreparative chromatographic separations were first achieved through multiple injection with 200 μL at a flow rate of 2 mL/min. The chromatographic analyses were carried out in isocratic mode at 22 ± 2 °C. The UV detection was performed at a wavelength of 254 nm. The fraction collected was analyzed using the analytical column to determine their enantiomeric ratio/excess with the condition described above.

**Chemistry for the 4$^{th}$ approach.** Regarding the fourth approach of indole-containing pyrazino[2,1-b]quinazoline-3,6-diones **39-46,** the compounds were also prepared via the highly effective and environmentally friendly microwave-assisted multicomponent polycondensation of amino acids. This methodology allowed us to prepare the fourth approach of pyrazinoquinazoline alkaloids through treatment of the anthranilic acid **(51)** derivatives with N-Boc-L-amino acids **(52)** and $(PhO)_3P$ at 55 °C for 16-20 h. Thereafter, D-tryptophan methyl ester hydrochloride **(53)** was added, and the mixture was stirred under microwave irradiation (300 W) at 220 °C for 1.5 min to furnish the final products **39-46 (Table 2).**

**Table 2:** Microwave-assisted multicomponent synthesis of indole-containing quinazolinone alkaloids **39-46.**

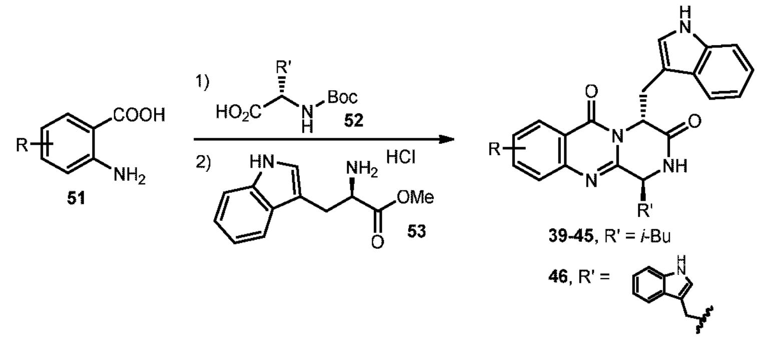


**51** **52** **53**

**39-45**, R' = *i*-Bu

**46**, R' =

| Anthranilic acid | Product | Compound | CLogP[a] | MW | Yield[b]/ *er*[c] |
|---|---|---|---|---|---|
| 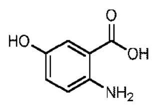 | 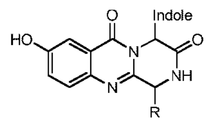 | **39** | 3.671 | 416.48 | 6.9/47:53 |
| 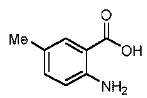 | 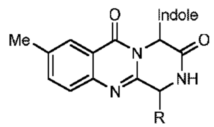 | **40** | 4.088 | 414.51 | 3.1/42:58 |
| 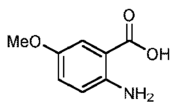 | 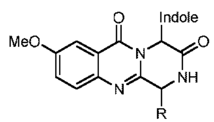 | **41** | 3.814 | 430.51 | 5.8/30:70 |
| 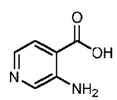 | 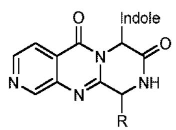 | **42** | 2.456 | 401.47 | 9.4/46:54 |
| 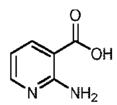 | 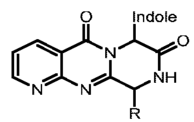 | **43** | 2.456 | 401.47 | 12.1/46:54 |

(continued)

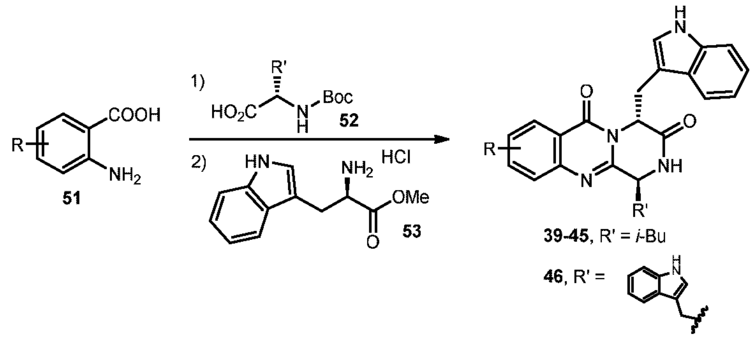


| Anthranilic acid | Product | Compound | CLogP[a] | MW | Yield[b]/ *er*[c] |
|---|---|---|---|---|---|
| 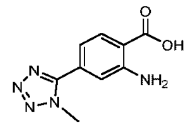 | 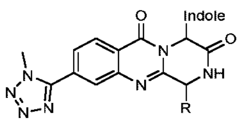 | **44** | 3.082 | 482.55 | 1.0/47:53 |
| 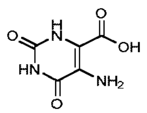 | 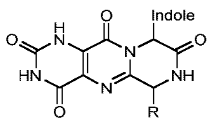 | **45** | 1.277 | 434.55 | 2.3/57:43 |
| **Anthranilic acid** | **Product** | **Compound** | **CLogP[a]** | **MW** | **Yield[b]/ *er*[c]** |
| 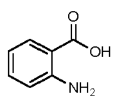 | 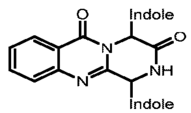 | **46** | 3.690 | 473.54 | 5.7/99:1 |

Reagents and conditions: 1) (PhO)3P, py, 55 °C, 24 h, 2) (PhO)3P, py, 220 °C, 1.5 min, a) calculated based on Cambio Draw, b) obtained after purification, c) determined by enantioselective liquid chromatography.

In the present disclosure, the general conditions for the synthesis of quinazolinone-3,6-(4H)-diones compounds **39-46** is as follows. In a closed vial, 5-hydroxy-anthranilic acid **(51a,** 184 mg, 1.2 mmol) for **39,** 5-methyl-anthranilic acid **(51b,** 181 mg, 1.2 mmol) for **40,** 5-methoxy-anthranilic acid **(51c,** 200 mg, 1.2 mmol) for **41,** 3-aminoisonicotinic acid **(51d,** 116 mg, 1.2 mmol) for **42,** 2-aminoisonicotinic acid **(51e,** 116 mg, 200 μmol) for **43,** 4-triazole-anthranilic acid **(51f,** 124 mg, 1.2 mmol) for **44,** or 5-aminoordotic acid **(51g,** 205 mg, 1.2 mmol) for **45,** or anthranilic **acid (51h,** 140 mg, 1.2 mmol) for **46** with N-Boc-L-leucine **(52a,** 299 mg, 1.2 mmol) for **39-45** or *N*-Boc-L-tryptophan, **(52b,** 365 mg,1.2 mmol) for **46** and triphenyl phosphite (495 μL, 1.44 mmol) were added along with 6 mL of dried pyridine. The vial was heated in heating block with stirring at 55 °C for 16-24 h. After cooling the mixture to room temperature, D-tryptophan methyl ester hydrochloride **(53,** 306 mg, 1.2 mmol) was added, and the mixture was divided into 3 individual vials, and irradiated in the microwave at the constant temperature at 220 °C for 1.5 min. After removing the solvent with toluene, the crude product was purified by flash column chromatography using n-hexane: EtOAc (60:40) as a mobile phase. The preparative TLC was performed using $CH_2Cl_2$:$Me_2CO$ (95:5) as mobile phase. The major compound appeared as a black spot with no fluorescence under the UV light. The desirable compounds **39-46** were collected as yellow solids. Before analysis, compounds were recrystallized from methanol.

In an embodiment, the characterization of (1S,4R)-4-((1*H*-indol-3-yl)methyl)-8-hydroxy-1-isobutyl-1,2-dihydro-6H-pyrazino[2,1-*b*]quinazoline-3,6(4H)-dione **(39)** is as follows: Yield: 38.5 mg, 6.9 %; mp: 162.4-163.5 °C (MeOH); $[\alpha]_D^{30}$ = - 74.60 (c 0.042; $CHCl_3$); $v_{max}$ (KBr) 3185, 3070, 1666, 1617, 1431, 1247 and 776 $cm^{-1}$; $^1H$ NMR (300 MHz, $CDCl_3$ ): δ 8.63 (d, 1H, J = 3.6 Hz, CH), 8.02 (s, 1H, NH-Trp), 7.73 (d, 1H, J = 2.9 Hz, OH), 7.53 (d, 2H, J 8.9 Hz, CH(2)), 7.34 (dd, 2H, J 7.7 and 4.0 Hz, CH-Trp (2)), 7.14 (t, 1H, J 7.1 Hz, CH-Trp), 7.00 (t, 1H, J 7.2 Hz, CH-Trp), 6.64 (d, 1H, J 2.3 Hz, CH-Trp), 5.66 (dd, 1H, J 5.3 and 3.0 Hz, CH*-Trp), 5.60 (s, 1H, NH-amide), 3.76 (dd, 1H, J 14.9 and 2.8 Hz, $CH_2$-Trp), 3.64 (dd, 1H, J 15.3 and 5.4 Hz, $CH_2$-Trp), 2.70 (dd, 1H, J 8.3 and 4.0 Hz, CH*-Leu), 1.70-1.59 (m, 1H, CH-Leu), 1.42-1.33 (m, 2H, $CH_2$-Leu) 0.77 (d, 3H, J = 6.3 Hz, $CH_3$-Leu), 0.27 (d, 3H, J = 6.4 Hz, $CH_3$-Leu) ; $^{13}C$ NMR (75 MHz, Acetone $d_6$): δ 169.7 (C=O), 161.1 (C=O), 157.2 (C-OH),150.1 (C=N), 141.6 (C), 137.3 (C-Trp), 129.9 (CH), 128.3 (C-Trp), 124.9 (CH-Trp), 124.7 (CH), 122.5 (CH-Trp), 122.3 (C), 119.9 (CH-Trp), 119.1 (CH-Trp), 112.1(CH-Trp), 110.0 (CH), 109.8 (C-Trp), 58.5 (CH*-Trp), 51.4 (CH*-Leu), 40.7 (CH-Leu), 27.4 ($CH_2$-Trp), 24.8 ($CH_2$-Leu), 23.3 ($CH_3$-Leu), 21.1 ($CH_3$-Leu).

In an embodiment, the characterization of (1S,4R)-4-((1*H*-indol-3-yl)methyl)-1-isobutyl-8-methyl-1,2-dihydro-6H-pyrazino[2,1-b]quinazoline-3,6(4H)-dione **(40)** is as follows: Yield: 15.6 mg, 3.1%; mp:156.7-157.0 °C (MeOH); $[\alpha]_D^{30}$ = -182 (*c* 0.055 $CHCl_3$); $v_{max}$ (KBr) 3067, 2915, 1682, 1470, and 770 $cm^{-1}$; $^1H$ NMR (300 MHz, $CDCl_3$): δ 8. 16 (s, 1H, CH), 8.04 (br, 1H, NH-Trp), 7.59 (dd, 1H, J 8.3, 2.0 Hz, CH), 7.50 (d, 2H, J 8.2 Hz, CH & CH-Trp), 7.28 (d, 1H, J 8.2 Hz, CH-Trp), 7.13 (t, 1H, J 7.6 Hz, CH-Trp), 6.99 (t, 1H, J 7.9 Hz, CH-Trp), 6.63 (d, 1H, J 2.3 Hz, CH-Trp), 5.70 (s, 1H, NH-amide), 5.68 (dd, 1H, J 5.4 and 2.9 Hz, CH*-Trp), 3.76 (dd, 1H, J 15.0 and 2.8 Hz, $CH_2$-Trp), 3.65 (dd, 1H, J 15.1 and 5.4 Hz, $CH_2$-Trp), 2.71 (dd, 1H, J 9.8 and 3.3 Hz, CH*-Leu), 2.53 (s, 3H, $CH_3$), 1.99 (ddd, 1H, J 13.7, 10.4, and 3.2 Hz, CH-Leu), 1.40-1.27 (m, 2H, $CH_2$-Leu), 0.77 (d, 3H, J 6.4 Hz, $CH_3$-Leu), 0.27 (d, 3H, J 6.5 Hz, $CH_3$-Leu) ; $^{13}C$ NMR (75 MHz, $CDCl_3$): δ 169.5 (C=O), 160.9 (C=O), 150.6 (C=N), 145.0 (C), 137.3 (C), 136.2 (CH), 136.1(C-Trp), 127.2 (CH), 126.2 (CH), 123.5 (CH-Trp), 122.8 (CH-Trp), 120.3 (C), 119.9 (CH-Trp), 118.9 (CH-Trp), 111.1 (CH-Trp), 109.8 (C-Trp), 57.2 (C*-Trp), 50.7 (C*-Leu), 40.2 ($CH_2$-Leu), 27.1 ($CH_2$-Trp), 24. 13 (CH-Leu), 23.3 ($CH_3$-Leu), 21.4 ($CH_3$), 19.7 ($CH_3$-Leu).

In an embodiment, the characterization of (1*S*,4*R*)-4-((1H-indol-3-yl)methyl)-1-isobutyl-8-methoxy-1,2-dihydro-6H-pyrazino[2,1-b]quinazoline-3,6(4*H*)-dione **(41)** is as follows: Yield: 36.6 mg, 5.83 %; mp: 152.7-153.3 °C (MeOH); $[\alpha]_D^{30}$ = - 222.22 (c 0.06; $CHCl_3$); $v_{max}$ (KBr) 3184, 2956, 1666, 1617, 1464, 1247, and 776 $cm^{-1}$; $^1H$ NMR (300 MHz, DMSO-$d_6$): δ 10.35 (s, 1H, NH-Trp), 7.69 (d, 1H, J 2.7 Hz, CH), 7.52 (d, 1H, J 8.9 Hz,CH-Trp), 7.38 (d, 1H, J 8.0 Hz, CH), 7.36 (dd, 1H, J 7.9 and 4.0 Hz, CH), 7.31 (d, 1H, J 8.1 Hz, CH-Trp), 7.21 (s, 1H, NH-amide), 7.05 (t, 1H, J 7.1 Hz, CH-Trp), 6.87 (t, 1H, J 7.4 Hz, CH-Trp), 6.68 (d, 1H, J 2.3 Hz, CH-Trp), 5.56 (dd, 1H, J 5.1 and 3.1 Hz, CH*-Trp), 3.97 (s, 3H, O-$CH_3$), 3.68 (dd, 1H, J 14.8 and 3.0 Hz, $CH_2$-Trp), 3.60 (dd, 1H, J 14.9 and 5.3 Hz, $CH_2$-Trp), 2.77 (dd, 1H, J 8.6 and 3.8 Hz, CH*-Leu), 2.08-1.87 (m, 1H, CH-Leu), 1.58-1.26 (m, 2H, $CH_2$-Leu), 0.69 (d, J 6.5 Hz, $CH_3$-Leu), 0.37 (d, 3H, J 6.5 Hz, $CH_3$-Leu); $^{13}C$ NMR (75 MHz, DMSO-$d_6$): δ 168.6 (C=O), 160.0 (C=O), 157.9 (C-O),150.0 (C=N), 141.2 (C), 136.2 (C), 128.9 (CH), 127.1 (C-Trp), 124.3 (CH-Trp), 121.4 (CH-Trp), 120.7 (C), 118.8 (CH-Trp), 118.8 (CH), 117.9 (CH-Trp),111.5 (CH-Trp), 108.2 (C-Trp), 57.3 (C*-Trp), 55.7 ($CH_3$), 50.3 (C*-Leu), 39.3 ($CH_2$-Leu), 26.4 ($CH_2$-Trp), 23.6 (CH-Leu), 22.9 ($CH_3$-Leu), 20.9 ($CH_3$-Leu).

In an embodiment, the characterization of (1S,4R)-4-((1*H*-indol-3-yl)methyl)-1-isobutyl-1,2-dihydro-6*H*-pyrazino[1,2-*a*]pyrido[3,4-*d*]pyrimi dine-3,6(4*H*)-dione **(42)** is as follows: Yield: 45.2 mg, 9.37 %; mp: 115.3-116.6 °C (MeOH); $[\alpha]_D^{30}$ = -176.30 (c 0.043; $CHCl_3$); $v_{max}$ (KBr) 3265, 2956, 1682, 1469, 1233, and 741$cm^{-1}$; $^1H$ NMR (300 MHz, $CDCl_3$): δ 9.05 (s, 1H, CH), 8.74 (d, 1H, J 5.2 Hz, CH), 8.14 (s, 1H, NH-Trp), 8.13 (d, J 4.9 Hz, CH), 7.45 (d, 1H, J 8.0 Hz, CH-Trp), 7.31 (d, 1H, J 8.2 Hz, CH-Trp), 7.15(dt, 1H, J 7.6 and0.8 Hz, CH-Trp), 6.97 (dt, 1H, J 7.6 and 0.8 Hz, CH-Trp), 6.66 (d, 1H, J 2.1 Hz, CH-Trp), 5.73(s, 1H, NH-amide), 5.66 (dd, 1H, J 5.2 and 1.9 Hz, CH*-Trp), 3.79 (dd, 1H, J 15.1 and 2.8 Hz, $CH_2$-Trp), 3.63 (dd, 1H, J 15.1 and 5.3 Hz, $CH_2$-Trp), 2.75 (dd, 1H, J 9.6 and 3.3 Hz, CH*-Leu), 2.08-1.87 (m, 1H, CH-Leu), 1.45-1.28

(m, 2H, $CH_2$-Leu), 0.78 (d, J 6.3 Hz, $CH_3$-Leu), 0.30 (d, 3H, J 6.5 Hz, $CH_3$-Leu); $^{13}$C NMR (75 MHz, $CDCl_3$): $\delta$ 168.8 (C=O), 159.8 (C=O), 153.81 (C=N), 151.2 (CH), 146.4 (CH), 136.2 (C-Trp), 127.0 (C-Trp), 123.5 (CH-Trp),123.0 (CH-Trp),120.5 (CH-Trp), 118.7 (CH), 118.6 (CH-Trp), 111.3 (CH-Trp), 109.4 (C-Trp), 57.7 (C*-Trp), 50.9 (C*-Leu), 40.2 ($CH_2$-Leu), 27.0 ($CH_2$-Trp), 24.2 (CH-Leu) 23.3 (CH-Leu), 19.7 ($CH_3$-Leu).

In an embodiment, the characterization of (7*R*,10*S*)-7-((1*H*-indol-3-yl)methyl)-10-isobutyl-9,10-dihydro-5*H*-pyrazino[1,2-a]pyrido[2,3-*d*] pyrimidine-5,8(7*H*)-dione **(43)** is as follows: Yield: 58.3 mg, 12.1%; mp: 111.3-111.5 °C (MeOH); $[\alpha]_D^{30}$ = -153.15 (c 0.037; $CHCl_3$); $v_{max}$ (KBr) 3295, 3067, 2915, 1682, 1600, 1470, 770, and 697 $cm^{-1}$; $^1$H NMR (300 MHz, $CDCl_3$): δ 9.01 (d, 1H, J 4.6 Hz, CH), 8.71 (d, 1H, J 7.9 Hz, CH), 8.07 (s, 1H,NH-Trp), 7.54 (d, 1H, J 7.8 Hz, CH-Trp), 7.51 (dd, 1H, J 7.9 and 4.5 Hz, CH), 7.31 (d, 1H, J 8.2 Hz, CH-Trp), 7.15 (dt, 1H, J 7.6 and 0.8 Hz, CH-Trp), 7.01 (dt, 1H, J 7.6 and 0.8 Hz, CH-Trp), 6.61 (d, 1H, J 2.4 Hz, CH-Trp), 5.69 (s, 1H, NH-amide), 5.64 (dd, 1H, J 5.3 and 2.8 Hz, CH*-Trp), 3.81 (dd, 1H, J 15.1 and 2.6 Hz, $CH_2$-Trp), 3.61 (dd, 1H, J 15.0 and 5.3 Hz, $CH_2$-Trp), 2.76 (dd, 1H, J 10.4 and 3.1 Hz, CH*-Leu), 1.21-1.14 (m, 1H, CH-Leu), 1.05-0.98 (m, 2H, $CH_2$-Leu), 0.78 (d, J 6.5 Hz, $CH_3$-Leu), 0.22 (d, 3H, J 6.5 Hz, $CH_3$-Leu); $^{13}$C NMR (75 MHz, $CDCl_3$): δ 169.1 (C=O), 160.0 (C=O), 153.81 (C=N), 147.1 (CH), 138.6 (CH), 136.1 (C-Trp), 127.2 (C-Trp), 123.4 (CH-Trp),121.1 (CH-Trp), 119.8 (CH), 118.4 (CH-Trp), 111.3 (CH-Trp), 109.4 (C-Trp), 57.5 (C*-Trp), 51.0 (C*-Leu), 40.2 ($CH_2$-Leu), 27.2 ($CH_2$-Trp), 24.1 (CH-Leu) 23.5 (CH-Leu), 19.6 ($CH_3$-Leu).

In an embodiment, the characterization of (1*S*,4*R*)-4-((1*H*-indol-2-yl)methyl)-1-isobutyl-9-(1-methyl-1*H*-tetrazol-5-yl)-1,2-dihydro-6H-pyrazino [2,1-*b*]quinazoline-3,6(4H)-dione (44) is as follows: Yield: 5.8 mg, 1 %; mp: 202.8-203.2 °C (MeOH); $[\alpha]_D^{30}$ = -125.68 (c 0.061; $CHCl_3$); $v_{max}$ (KBr) 3356, 3119, 3053, 1671, 1457, 1261, and 740 $cm^{-1}$; $^1$H NMR (300 MHz, $CDCl_3$): δ 8.47 (d, 1H, J 8.3 Hz, CH), 8.41 (d, 1H, J 1.1 Hz, CH), 8.27 (dd, 1H, J 8.3 and 1.5 Hz, CH), 8.10 (s, 1H, NH-Trp), 7.50 (d, 1H, J 8.0 Hz, CH-Trp), 7.30 (d, 1H, J 8.3 Hz, CH-Trp), 7.13 (t, 1H, J 7.6 Hz, CH-Trp), 6.98 (d, 1H, J 7.5 Hz, CH-Trp), 6.67 (d, 1H, J 2.3 Hz, CH-Trp), 5.75 (s, 1H, NH-amide), 5.68 (dd, 1H, J 5.1 and 2.8 Hz, CH*-Trp), 4.45 (s, 3H, $CH_3$-N), 3.79 (dd, 1H, J 15.0 and 2.8 Hz, $CH_2$-Trp), 3.66 (dd, 1H, J 15.1 and 5.3 Hz, $CH_2$-Trp), 2.74 (dd, 1H, J 9.2 and 3.4 Hz, CH*-Leu),2.09-1.96 (m, 1H, CH-Leu), 1.43-1.30 (m, 2H, $CH_2$-Leu), 0.76 (d, 3H, J 6.2 Hz, $CH_3$-Leu), 0.31 (d, 3H, J 6.4 Hz, $CH_3$-Leu); $^{13}$C NMR (75 MHz, $CDCl_3$): δ 169.3 (C=O), 164.2 (C=N-Tetrazol), 160.5 (C=O), 152.35 (C=N),147.4 (C), 136.1 (C-Trp), 133.2 (C-Ctriazole), 127.8 (CH), 127.1 (C-Trp), 125.7 (CH), 125.0 (CH), 123.6 (CH-Trp), 122.8 (CH-Trp), 121.2 (C), 120.4 (CH-Trp),118.7 (CH-Trp), 111.2(CH-Trp), 109.6 (C-Trp), 57.4 (CH*-Trp), 50.9 (CH*-Leu), 40.7 (CH-Leu), 39.7 ($CH_3$), 27.1 ($CH_2$-Trp), 24.2 ($CH_2$-Leu), 23.2 ($CH_3$-Leu), 19.9 ($CH_3$-Leu).

In an embodiment, the characterization of (6*S*,9*R*)-9-((1H-indol-3-yl)methyl)-6-isobutyl-6,7-dihydro-1H-pyrimido[5,4-*d*]pyrimidine-2,4,8,11 (3*H*,9*H*)-tetraone **(45)** is as follows: Yield: 11.6 mg, 2.3 %; mp: 306-306.5 °C (MeOH); $[\alpha]_D^{30}$ = - 226.7 (c 0.025; $CHCl_3$); $v_{max}$ (KBr) $cm^{-1}$; 3384, 2956, 1670, 1457, 1322, and 1095; $^1$H NMR (300 MHz, $CDCl_3$): δ 8.21 (s, 1H, NH-Trp), 7.65 (d, 1H, J 7.8 Hz, CH-Trp), 7.39 (d, 1H, J 8.0 Hz, CH-Trp),7.22 (dd, J 8.1 and 1.1 Hz, CH-Trp), 7.18-7.12 (m, 1H, CH-Trp), 7.11 (d, 1H, J 2.3 Hz, CH-Trp), 6.73 (s, 1H, NH-amide), 5.98 (s, 1H, NH-Ant), 5.96 (s, 1H, NH-Ant), 4.28 (m, 1H,CH*-Trp), 3.52 (dd, 1H, J 14.6 and 3.6 Hz, $CH_2$-Trp), 3.44 (m, 1H, CH*-Leu), 3.19 (dd, 1H, J 14.7 and 8.5 Hz, $CH_2$-Trp), 1.69 (m, 1H, CH-Leu),1.54 (m, 2H, $CH_2$-Leu), 0.90 (d, J 6.1 Hz, $CH_3$-Leu), 0.76 (d, 3H, J 6.1 Hz, $CH_3$-Leu); $^{13}$C NMR (75 MHz, $CDCl_3$): δ 168.7 (C=O), 168.2 (C=O), 165.9 (C=O), 160.2 (C=O), 150.5 (C=N), 146.9 141.74 (C), 136.5 (C-Trp), 126.7 (C-Trp), 123.9 (CH-Trp), 122.8 (CH-Trp), 122.3 (C),120.2 (CH-Trp), 118.8 (CH-Trp),111.4 (CH-Trp), 109.3 (C-Trp), 54.7 (C*-Trp), 53.1 (C*-Leu), 42.1 ($CH_2$-Leu), 29.9 ($CH_2$-Trp), 24.0 (CH-Leu), 23.1 (CH-Leu), 20.8 ($CH_3$-Leu).

In an embodiment, the characterization of (1S,4R)-1,4-bis((1H-indol-3-yl)methyl)-1,2-dihydro-6H-pyrazino[2,1-b]quinazoline-3,6(4H)-dione **(46)** is as follows: Yield: 27.4 mg, 5.7%; *er* = 99:0; mp: 177.4-178.2 °C (MeOH); $[\alpha]_D^{30}$ = -66.17 (*c* 0.013; $CHCl_3$); $v_{max}$ (KBr): 3404, 3060, 2923, 1681, 1597, 1455, 695 and 668$cm^{-1}$; $^1$H NMR (300 MHz, $CDCl_3$): $\delta$ 8.41 (dd, 1H, J8.0 and 1.3, CH), 8.10 (s, 1H, NH-Trp), 8.04 (s, 1H, NH-Trp), 7.82 (ddd, 1H, *J* 8.5, 7.2 and 1.5 Hz, CH), 7.66 (d, 1H, *J* 7.7 Hz, CH), 7.57 (ddd, 1H, J 8.1, 7.5 and 1.2 Hz, CH), 7.36 (d, 2H, *J* 8.1 Hz, CH-Trp), 7.34 (d, 2H, *J* 8.0 Hz, CH-Trp), 7.22 (ddd, 1H, *J* 8.1, 7.5 0.8 Hz, CH-Trp), 7.14 (t, 1H, *J* 7.3 Hz, CH-Trp), 7.08 (ddd, 1H, *J* 8.1, 7.5 0.8 Hz, CH-Trp), 6.80 (t, 1H *J* 7.3, CH-Trp), 6.65 (d, 1H J 2.3 Hz, CH-Trp), 6.42 (d, 1H J 1.9 Hz, CH-Trp), 5.68 (s, 1H, NH-amide), 5.66 (dd, 1H, J 7.8, 3.8 Hz, CH*-Trp), 3.73 (dd, *J* 2.3 Hz, CH*-Trp), 3.67 (dd, 2H, *J* 18.1 and 3.7 Hz, $CH_2$-Trp ), 3.11 (dd, 1H, *J* 11.0 and 3.4 Hz- CH2-Trp), 2.75 (dd, 15.1 and 11.1 Hz, $CH_2$-Trp);$^{13}$C NMR (75 MHz, $CDCl_3$): $\delta$ 169.6 (C=O), 161.7 (C=O), 154.6, (C=N), 146.2 (C), 136.2 (C-Trp), 135.9 (C-Trp), 134.9 (CH), 133.3 (CH), 131.4 (CH), 127.8 (CH), 127.5 (C-Trp), 127.3 (C-Trp), 123.9 (CH), 123.7 (CH), 122.4 (CH-Trp), 122.3 (CH-Trp), 121.6 (CH-Trp), 119.7 (C-Trp(2)), 118.2 (CH-Trp), 114.1 (CH-Trp), 111.4 (CH-Trp), 110.1 (C-Trp), 64.6 (CH*-Trp), 54.0 (CH*-Trp), 30.9 ($CH_2$-Trp), 25.1 ($CH_2$-Trp).

In the present disclosure, all reagents were from analytical grade. Dried pyridine and triphenylphosphite were purchased from Sigma (Sigma-Aldrich Co. Ltd., Gillinghan, UK). Anthranilic acids **(47)** and protected amino acids **48** and **50** were purchased from TCI (Tokyo Chemical Industry Co. Ltd., Chuo-ku, Tokyo, Japan). Column chromatography purifications were performed using flash silica Merck 60, 230-400 mesh (EMD Millipore corporation, Billerica, MA, USA) and preparative TLC was carried out on precoated plates Merck Kieselgel 60 $F_{254}$ (EMD Millipore corporation, Billerica, MA, USA), spots were visualized with UV light (Vilber Lourmat, Marne-la-Vallée, France). Melting points were measured in

a Köfler microscope and are uncorrected. Infrared spectra were recorded in a KBr microplate in a FTIR spectrometer Nicolet iS10 from Thermo Scientific (Waltham, MA, USA) with Smart OMNI-Transmission accessory (Software 188 OMNIC 8.3). $^1$H and $^{13}$C NMR spectra were recorded in $CDCl_3$ (Deutero GmbH, Kastellaun, Germany) at room temperature unless otherwise mentioned on Bruker AMC instrument (Bruker Biosciences Corporation, Billerica, MA, USA), operating at 300 MHz for $^1$H and 75 MHz for $^{13}$C). Carbons were assigned according to HSQC and or HMBC experiments. Optical rotation was measured at 25 °C using the ADP 410 polarimeter (Bellingham + Stanley Ltd., Tunbridge Wells, Kent, UK), using the emission wavelength of sodium lamp, concentrations are given in g/100 mL. High resolution mass spectra (HRMS) were measured on a Bruker FTMS APEX III mass spectrometer (Bruker Corporation, Billerica, MA, USA) recorded as ESI (Electrospray) made in Centro de Apoio Cientifico e Tecnolóxico á Investigation (CACTI, University of Vigo, Pontevendra, Spain). The purity of synthesized compounds was determined by reversed-phase LC with diode array detector (DAD) using C18 column (Kimetex®, 2.6 EV0 C18 100 Å, 250 × 4.6 mm), the mobile phase was methanol: water (50:50), and the flow rate was 1.0 mL/min. Enantiomeric ratio was determined by enantioselective LC (LCMS-2010EV, Shimadzu, Lisbon, Portugal), employing a system equipped with a chiral column (Lux® 5 μm Amylose-1, 250 × 4.6 mm) and UV-detection at 254 nm, mobile phase was hexane:EtOH (90:10) and the flow rate was 0.5 mL/min. for semipreparative chromatography, a HLPC system consisted of a Shimadzu LC-6AD pump with a 200 μL loop was used with an amylose tris-3,5-dimethylphenylcarbamate coated with Nucleosil (500 A, 7m, 20%, w/w) packed into a stainless-steel (200 mm x 7 mm I.D. size) column, prepared in the UFSCar laboratory[39A].

**Antibacterial Activity**

The present disclosure also relates to antibacterial activity of the compounds herein disclosed.

In the present disclosure, two Gram-positive - *Staphylococcus aureus* ATCC 29213 and *Enterococcus faecalis* ATCC 29212- and two Gram-negative - *Escherichia coli* ATCC 25922 and *Pseudomonas aeruginosa* ATCC 27853 - reference bacterial strains were used. When it was possible to determine a minimal inhibitory concentration (MIC) value for these strains, clinically relevant strains were also used. These included methicillin-resistant S. *aureus* (MRSA) 66/1, isolated from public buses, as well as a isolate sensitive to the most commonly used antibiotic families (*S. aureus* 40/61/24); and two *vancomycin-resistant Enterococcus (VRE) strains isolated from* river water, *E. faecalis* B3/101 and E. *faecalis* A5/102, which is sensitive to ampicillin. Frozen stocks of all strains were grown on Mueller-Hinton agar (MH - BioKar Diagnostics, Allone, France) at 37 °C for 24 h. All bacterial strains were sub-cultured on MH agar and incubated overnight at 37 °C before each assay, in order to obtain fresh cultures.

An initial screening of the antibacterial activity of the compounds was performed by the Kirby-Bauer disk diffusion method, as recommended by the Clinical and Laboratory Standards Institute (CLSI). Briefly, sterile 6 mm blank paper disks (Oxoid, Basingstoke, England) impregnated with 15 μg of each compound were placed on inoculated MH agar plates. A blank disk with DMSO was used as a negative control. MH inoculated plates were incubated for 18-20 hours at 37 °C. At the end of the incubation, the inhibition halos where measured. The minimal inhibitory concentration (MIC) was used to determine the antibacterial activity of each compound, in accordance with the recommendations of the CLSI. Two-fold serial dilutions of the compounds were prepared in Mueller-Hinton Broth 2 (MHB2 - Sigma-Aldrich, St. Louis, MO, USA) within the concentration range of 0.062-64 μg/mL. Cefotaxime (CTX) ranging between 0.031-16 μg/mL was used as a control. Sterility and growth controls were included in each assay. Purity check and colony counts of the inoculum suspensions were also performed in order to ensure that the final inoculum density closely approximates the intended number ($5 \times 10^5$ CFU/mL). The MIC was determined as the lowest concentration at which no visible growth was observed. The minimal bactericidal concentration (MBC) was assessed by spreading 10 μL of culture collected from wells showing no visible growth on MH agar plates. The MBC was determined as the lowest concentration at which no colonies grew after 16-18 hours incubation at 37 °C. These assays were performed in duplicate.

In order to evaluate the combined effect of the compounds and clinically relevant antimicrobial drugs, a screening was conducted using the disk diffusion method, as previously described. A set of antibiotic disks (Oxoid, Basingstoke, England) to which the isolates were resistant was selected: cefotaxime (CTX, 30 μg) for extended spectrum beta-lactamase-producer *E. coli* SA/2, oxacillin (OX, 1 μg) for S. *aureus* 66/1, and vancomycin (VA, 30 μg) for *E. faecalis* B3/101. Antibiotic disks alone (controls) and antibiotic disks impregnated with 15 μg of each compound were placed on MH agar plates seeded with the respective bacteria. Sterile 6 mm blank papers impregnated with 15 μg of each compound alone were also tested. A blank disk with DMSO was used as a negative control. MH inoculated plates were incubated for 18-20 hours at 37 °C. Potential synergism was recorded when the halo of an antibiotic disk impregnated with a compound was greater than the halo of the antibiotic or compound-impregnated blank disk alone.

Therefore, an initial screening of the antibacterial activity of the compounds 10-37 against the above-mentioned different reference strains of Gram-positive bacteria, Gram-negative bacteria, as well as clinically relevant multidrug-resistant (MDR) strains was performed by the disk diffusion method. This primary assessment was followed by the determination of minimal inhibitory concentrations (MIC) of reference strains. For active compounds, this determination was also made for MDR strains. In the range of concentrations tested, none of the compounds was active against Gram-negative bacteria, and none of **10-25, 26, 33, 34** and **37** was active against any of the tested strains (results not shown). The results of antibacterial activity on Gram-positive strains regarding all other compounds are presented in **Table 3.**

**Table 3.** Antibacterial activity of quinazolinones **27-32, 35** and **36** on Gram-positive reference and clinically relevant strains.

| | ***S. aureus* ATCC 29213** | | ***S. aureus* 40/61/24** | | ***S. aureus* 66/1 (MRSA)** | | ***E. faecalis* ATCC 29212** | | ***E. faecalis* A5/102 (VRE)** | | ***E. faecalis* B3/101 (VRE)** | |
|---|---|---|---|---|---|---|---|---|---|---|---|---|
| | **MIC** | **MBC** | **MIC** | **MBC** | **MIC** | **MBC** | **MIC** | **MBC** | **MIC** | **MBC** | **MIC** | **MBC** |
| **27** | 32 | > 64 | 64 | >64 | >64 | >64 | 64 | >64 | 64 | >64 | >64 | >64 |
| **27a** | >64 | >64 | ND | ND | ND | ND | >64 | >64 | ND | ND | ND | ND |
| **27b** | >64 | >64 | ND | ND | ND | ND | >64 | >64 | ND | ND | ND | ND |
| **28** | 32 | > 64 | 64 | >64 | >64 | >64 | 32 | >64 | 64 | >64 | >64 | >64 |
| **28b** | >64 | ND | ND | ND | ND | ND | >64 | >64 | ND | ND | ND | ND |
| **29** | 16 | > 64 | 64 | >64 | >64 | >64 | 32 | >64 | 64 | >64 | >64 | >64 |
| **30** | 16 | > 64 | >64 | >64 | >64 | >64 | >64 | >64 | ND | ND | ND | ND |
| **31** | 4 | > 64 | 8 | >64 | 8 | >64 | >64 | >64 | ND | ND | ND | ND |
| **31a** | 4 | >64 | 4 | >64 | 4 | >64 | >64 | >64 | ND | ND | ND | ND |
| **31b** | >64 | >64 | ND | ND | ND | ND | >64 | >64 | ND | ND | ND | ND |
| **32** | 4 | > 64 | 8 | >64 | 8 | >64 | >64 | >64 | ND | ND | ND | ND |
| **35** | 16 | > 64 | 64 | >64 | >64 | >64 | >64 | >64 | ND | ND | ND | ND |
| **36** | 16 | > 64 | >64 | >64 | >64 | >64 | >64 | >64 | ND | ND | ND | ND |

MIC, minimal inhibitory concentration; MBC, minimal bactericidal concentration; VRE, vancomycin-resistant *Enterococcus;* MRSA, methicillin-resistant *Staphylococcus aureus;* ND, not determined. MIC and MBC are expressed in μg/mL.

None of the derivatives exhibit antibacterial activity against Gram-negative bacteria, similarly to the described for the natural isolated neofiscalin A **(2A).** Regarding antimicrobial activity against Gram-positive bacteria, **27, 28,** and **29** had an inhibitory effect on both *Enterococcus faecalis* ATCC 29212 and *Staphylococcus aureus* ATCC 29213 reference strains, while **30, 31, 32, 35,** and **36** only showed an inhibitory effect on S. *aureus* ATCC 29213. The most effective compounds against S. *aureus* reference strain were **31** and **32,** with MIC values of 4 μg/mL. All of those compounds presented a bacteriostatic activity, with minimal bactericidal concentrations (MBC) greater than 64 μg/mL.

Analog **29** was the most effective, with MIC values of 32 μg/mL and 16 μg/mL against *E. faecalis* ATCC 29212 and S. *aureus* ATCC 29213, respectively. When tested against *vancomycin-resistant Enterococcus* (VRE) that was sensitive to ampicillin, the MICs obtained for **27, 28** and **29** were higher than those obtained for the reference strain (64 μg/mL as opposed to 32 μg/mL). In the range of concentrations tested, all these compounds were ineffective against *E. faecalis* B3/101, a VRE strain that was also resistant to ampicillin. Regarding *S. aureus,* **27, 28** and **29** inhibited the growth of the strain 40/61/24 (MIC 64 μg/mL), which is sensitive to the most commonly used antibiotic families, but not of methicillin-resistant S. *aureus* (MRSA) 66/1. More importantly, compounds **31** and **32** showed a greater inhibitory capacity on both sensitive (40/61/24) and methicillin-resistant S. *aureus* (66/1) strains, with MIC values of 8 μg/mL.

In an embodiment, the synergistic effects with vancomycin and oxacillin were evaluated for MDR strains, but no effect was found. These antibiotics are relevant in the treatment of infections caused by *Enterococcus* spp. and *Staphylococcus aureus,* respectively.

The compounds showed activity only for Gram-positive strains and, overall, this activity was greater for reference strains than for clinically relevant strains, whether MDR or not. Regarding Gram-positive strains, the range was not equal for all compounds, with a greater number of compounds being active against S. *aureus* than *E. faecalis.* Whereas for *E. faecalis* there appeared to exist an inverse relationship between compound activity and resistance against clinically important antibiotics, there was not a clear tendency for S. *aureus.* It would be interesting to further study the promising inhibitory effect of ccompounds **31** and **32** on MRSA. Noteworthy, the first series of compounds ($1^{st}$ aapproach) showed no relevant effect in the growth of non-malignant cells.

In an embodiment, in order to evaluate the *in vitro* activities, such as antibacterial, the most promising derivatives **27, 28,** and **31,** were obtained in milligram scale by semipreparative enantioselective liquid chromatography, employing a tris-3,5-dimethylphenylcarbamate amylose column with multiple injection in a 200 μL loop.

The analytical method presented good separation (α > 1.2) and resolution values (Rs > 8) for all compounds to allow the scale-up to the preparative mode. The semipreparative separation was optimized by adjusting the sample

volume from the analytical method. The optimized mobile phase of analytical system (hexane: EtOH, 90:10) was transferred without any modification to semipreparative mode and 254 was chosen as minimum wavelength absorption. The column diameter was enlarged to a scale-up factor of 3. The flow rate was increase from 0.5 to 2 mL/min, and the retention times were between 15 to 50 min. The loading effect in semipreparative mode was examined by keeping the concentration of the feed solution at the maximum (1.5 mg/mL) and by varying the volume (100 to 200 μL). The mobile phase composition, chromatograms, and chromatographic parameter are summarized in **Figure 8 at** analytical and semipreparative scales.

The elution order, specific rotation, and enantiomeric ratio (*e.r*) of resolved enantiomers were measured and the data is presented in **Table 4.** The *e.r* was greater than 97% for each enantiomer.

**Table 4:** Elution order, specific rotation, and enantiomeric excess (*e.r*) of the resolved compound **27, 28,** and **31** enantiomers.

| Enantiomer | Elution order | [α]D (c)[a] | *e.r* (%)[b] |
|---|---|---|---|
| (-)-27 (27a) | First order | -0.06 (0.08) | >99 |
| (+)-27 (27b) | Second order | +0.04 (0.10) | >99 |
| (-)-28 (28a) | First order | -0.08 (0.05) | >99 |
| (+)-28 (28b) | Second order | +0.22 (0.12) | >99 |
| (-)-31 (31a) | First order | -0.16 (0.03) | 97:3 |
| (+)-31 (31b) | Second order | +0.15 (0.03) | >99 |

[a] Specific rotation in methanol with c = concentration in g/mL

[b] Enantiomeric ratio (e.r) determinated by enantioselective LC under condition.

The pure enantiomers of **27, 28,** and **31** were evaluated for antibacterial and antifungal activity. Enantiomer **31a** showed a MIC of 4 μg/mL for reference strain *S. aureus* ATCC 29213, sensitive clinical isolate *S. aureus* 40/61/24, and methicillin-resistant strain *S. aureus* 66/1, while enantiomer **31b** showed no effect **(Table 3).** Noteworthy, these derivatives showed higher potency than the natural product neofiscalin A **(2),** (tested by the group with the same conditions)[24-26]. None of the pure enantiomers was active against the fungi tested.

Regarding antibacterial activity, the structure-activity relationship (SAR) study suggested that the presence of a halogen atom at positions C-9 or C-11 plays a crucial role for this activity, since all the non-halogenated compounds were inactive against all the tested strains **(Figure 4).** In fact, compounds containing chlorine atoms at one or both positions exhibited better antibacterial activity compared to those having bromine and iodine. Higher antibacterial activities were obtained when the halogen atom is present at both C-9 and C-11 positions (e.i., compound **30, 31, 32** and **37)** and/or the presence of longer side chains at C-1. The enantiopure compound **31a** showed significant antibacterial effect against a resistant strain of *S. aureus* while its antipode **(31b)** did not. This emphasizes that configuration (*1S*,*4R*) is crucial for antibacterial activity of quinazolinone scaffold.

**Antimalarial Activity**

The present disclosure also relates to antimalarial activity of the compounds herein disclosed.

The principle of the *in vitro* susceptibility test to malaria is to assess the degree of development of parasites *P. falciparum* in the presence of different concentrations of the compounds. In this assay, *P*. falciparum 3D7, a CQ-susceptible strain, was used to evaluate the antimalarial activity of the 29 quinazolinones. Activity was described in terms of IC50 (concentration that inhibits the growth of 50 % of P. falciparum parasite present in the culture) for 14 compounds (Table 5). The remaining 15, exhibited non-appreciable antiparasitic activity, they fail to produce dose-response curves and/or displayed > 75 % survival at 10 μM (data not shown).

To evaluate the antimalarial potential of the pyrazino[2,1-b]quinazoline-3,6-dione scaffold, the following compounds were screened: compounds **10-17** (1st approach), having 4 types of stereoisomers; compounds **19, 21, 23, 25** and **26** (2nd approach), compounds **28, 29, 31, 32, 35-38** (3rd approach) and compounds **39-46 (4th** approach).

**It was observed** that anti-isomers *1S, 4R,* like compounds **12** (fiscalin B) and **16,** exhibited the highest **antimalarial activity** while syn-isomers *1S, 4S* were inactive (compounds **10** and **14)** and *syn*-isomers *1R,4R* had decreased activity (compounds **13** and **17).** Furthermore, compounds in the 1st approach **(10-17)** demonstrated that increasing the size of the C-1 substituent increased the antimalarial activity, for example, compound **16** with C-1 having an isobutyl the same position. Compounds **12, 13, 16, 17,** and **31,** preferably **12, 13, 16** and **31,** showed the highest antimalarial activity against *P. falciparum* strain 3D7.

To further evaluate the effect of C-1 substituent on the activity, the compounds of the 2nd approach **(19, 21, 23, 25**

and **26)** was evaluated, and SAR indicates that a sulfur substituent at C-1 do not favor activity (compounds **21).**

In the investigation of the 3rd approach of compounds **(28, 29, 31, 32, 35-38)** with different substituents on A ring, only compound **31 having** chlorine atom at position 9 and 11 showed favourable antimalarial activity with an $IC_{50}$ value of 0.2 μM (weaker than compounds **12** and **16),** while other derivatives (substituted with Br or I) showed to be inactive.

For the 4th approach of pyrazino[2,1-*b*]quinazoline-3,6-diones **(39-46),** isosteric substitutions with the nitrogen atom at different positions of ring A (positions 9, 10, 11), led to a decrease/inactivation of the antimalarial activity (compounds **42** and **43).** Compounds **39** and **41** each bearing a hydroxy or methoxy group at position 9 of ring A also showed a decrease in activity. Contrary to other reports of febrifugine derivatives, compound **44** with a tetrazole group at position 10 also showed a weak activity against *P. falciparum.*

**Table 5.** *In vitro* activity against *Plasmodium falciparum* 3D7 strain.

| ***P. falciparum*** **(3D7)** | | | |
|---|---|---|---|
| **Compounds** | **$IC_{50}$ [μM]** | **Compound** | **$IC_{50}$ [μM]** |
| **12** (1st approach) | 0.10±0.02 | **31** (3rd approach) | 0.20±0.14 |
| **13** (1st approach) | 0.15±0.05 | **32** (3rd approach) | 1.51±0.53 |
| **15** (1st approach) | 2.00±0.32 | **38** (3rd approach) | 4.00±0.020 |
| **16** (1st approach) | 0.05±0.02 | **39** (4th approach) | 0.73±0.07 |
| **17** (1st approach) | 0.47±0.22 | **42** (4th approach) | 4.00±0.02 |
| **26** (2nd approach) | 3.68±0.62 | **43** (4th approach) | 3.76±060 |
| **25** (2nd approach) | 4.18±0.03 | **44** (4th approach) | 1.02±0.27 |
| **CQ (6)*** | 15.08±0.08 | | |
| * the $IC_{50}$ value of CQ is in nM. | | | |

An important criterion in evaluating active antimalarial compounds is their cytotoxicity in mammalian host cells. Compounds that showed the lowest $IC_{50}$ values against *P. falciparum* **(12, 16,** and **31)** were selected to evaluate their cytotoxicity. The cell lines used for *in vitro* cytotoxicity assay were the V79 from non-tumor cell line of Chinese hamster lung fibroblasts and CQ **(6)** was used as control. The results showed relatively low $LD_{50}$ values ($LD_{50}$ concentration that inhibits the growth of 50 % of cells present in the culture) when compared to CQ (6) **(Table 6).** Nonetheless, the selectivity index (SI; calculated by $LD_{50}/IC_{50}$) for compounds **12, 16,** and **31** were between 19-70 **(Table 6)** and within the acceptable safety range (SI values greater than 10 indicates that a compound has an acceptable therapeutic window for the development of antimalarial drugs).

In general, the higher the SI, the more promising as an anti-malarial are the compounds, due to its selective action against the parasite.

**Table 6.** Cytotoxicity against mammalian cells of compounds **12, 16,** and **31.**

| **Compounds** | ***P. falciparum*** **(3D7)** | **Mammalian cells (V79)** | **SI** |
|---|---|---|---|
| | $IC_{50}$ (μM) | $DL_{50}$ (μM) | |
| **12** (1st approach) | 0.10 ± 0.02 | 1.91 ± 0.44 | 19 |
| **16** (1st approach) | 0.05 ± 0.02 | 1.78 ± 0.47 | 34 |
| **31** (3rd approach) | 0.20 ± 0.14 | 14.00 ± 1.41 | 70 |
| **CQ (6)*** | 15.08 + 0.80* | 167.00 ± 42.00 | 11074 |

* the $IC_{50}$ value of CQ is in nM; SI - Selectivity Index. The results of $IC_{50}$ and $LD_{50}$ are presented as mean ± standard deviation.

In an embodiment, the evaluation of hemotoxicity *in vitro* was performed as follows. The *in vitro* hemolysis assay evaluates the release of hemoglobin in the medium (as an indicator of lysis of erythrocytes) after exposure to the test compounds. Drug-induced hemolysis can occur by two mechanisms: allergic hemolysis (toxicity caused by an immunological reaction in patients previously sensitized to a drug) and toxic hemolysis (direct toxicity of the drug, its metabolite or an excipient in the formulation) [26B]. This test was intended to determine the potential toxic hemolytic effect of the *hit* compounds **12, 16,** and **31 on** healthy/non-parasitized erythrocytes **(Figure 6).** The % of hemolysis induced by the

compounds was also determined under standard culture conditions of *P. falciparum.*

The % of hemolysis of healthy erythrocytes induced by **12, 16,** and **31** was lower than 6% **(Figure** 6) and within the range of that of CQ (6). Compounds **12, 16,** and **31** and CQ **(6)** had no hemolytic activity at ≤ 10 μM. CQ (6) is considered a non-hemolytic antimalarial drug in healthy human erythrocytes. Compounds **12, 16,** and **31** did not present hemolytic activity, since the % hemolysis was <10 % (% hemolysis > 25 % is considered as indicative of risk of hemolysis).

The assay of inhibition of the polymerization of hemozoin (β-hematin) *in vitro* was based on the protocol of Basilico *et al.* with some modifications and was carried out for compounds **12, 16, 31** and CQ (6) by using a hemin solution (ferriprotoporphyrin IX chloride). In this assay, CQ (6) was used as a positive control to evaluate the quality of the test since compound 6 binds to portions of hemozoin produced from the proteolytic process of hemoglobin in infected erythrocytes, thus interfering with hemozoin detoxification. Compounds, **12, 16,** and **31** did not show to inhibit the polymerization of β-hematin *in vitro* **(Figure 5).** Febrifuge (compound **9)** significantly inhibits the formation of hemozoin required for the maturation of the parasite *Plasmodium spp.* in the trophozoite stage via axial ligand or π-π interaction to heme. Even though pyrazino[2,1-*b*]quinazoline-3,6-diones **12, 16, and 31** possess structure similarities with febrifugine (compound **9),** results suggested that the mechanism of action of these derivatives might be different from febrifugine (compound **9).**

Recently, the cytoplasmic prolyl-tRNA synthetase of *P. falciparum* (PfcPRS), a member of the aminoacyl-tRNA synthetase (aaRS) family that drive protein translation, has been identified as the functional target of febrifugine (compound 9) and analogues, such as halofuginone (HF), a semisynthetic analogue in clinical trials. Therefore, a putative target for this approach of new antimalarials could be the PfcPRS and this hypothesis was explored with *in silico* studies. The computational docking study on inhibitory effect of prolyl-tRNA synthetase was carried out as follows. The binding affinity of twenty-nine pyrazinoquinazolinones **(10-17, 19, 21, 23, 25, 26, 28, 29, 31, 32, 35-46) to** PRS enzyme target was predicted using computational docking AutodockTools. The positive controls were febrifugine (9, FF), HF, tetrahydroquinazolinone febrifugine (ThFF), and 6-fluorofebrifugine (6FFF) that were predicted as having high binding affininy to PRS, with docking scores between -9.3 and 9.7 $kcal.mol^{-1}$, wherase the negative control, CQ **(6),** revealed a docking score of -7.4 $kcal.mol^{-1}$. The most active antimalarials *in vitro,* compounds **12, 13, 16, 17** and **31,** preferably **12, 13, 16** and **31,** presented docking score from -9.1 to - 11.4 $kcal.mol^{-1}$, predicted as forming complexes with PRS enzyme **(Table 7, Figure 7A).**

**Table 7.** Docking scores of the test compounds and controls onto 4ydq PRS binding site.

| Compounds | Docking scores ($kcal.mol^{-1}$) |
|---|---|
| **12** ($1^{st}$ approach) | -9.1 |
| **13** ($1^{st}$ approach) | -11.4 |
| **16** ($1^{st}$ approach) | -10.0 |
| **31** ($3^{rd}$ approach | -9.9 |
| **Febrifugine (9, FF)** | -9.5 |
| **Halofuginone (HF)** | -9.3 |
| **ThFF** | -9.5 |
| **6FFF** | -9.7 |
| **CQ (6)** | -7.4 |

Halofuginone (HF) is described as being mimetic of the enzyme substrates L-Pro and adenine-76 of tRNA, binding into the active site pockets simultaneously with ATP. Other quinazolinone-based compounds such as FF, 6FFF, and ThFF have also been described as specific for PfPRS when in the presence of the ATP analogue adenosine 5'-(β,γ-imido)triphosphate (AMPPNP). The structure of the ternary complex of PfPRS-AMPPNP-HF reveals hydrogen interactions with Thr359, Glu361, Arg390, Thr478, and His480, and π-π stacking interactions with Phe335 **(Figure 7B).** Compound **13** fits the same binding pocket as HF, binding with some of the same residues as HF. The N atom of the indole ring forms hydrogen bonds with Thr478 and His480, and with AMPPNP phosphate groups; and the pyrazinoquinazolinone ring of **13** is mainly stabilized by hydrogen interactions with Glu361 and π-π stacking contacts with Phe335, but does not establish polar interactions with Arg390, suggesting chemical spaces available for additional modifications or derivatizations **(Figure 7C** and **D).** Compounds **12, 16,** and **31** bind in the same positions in the PRS cavity but do not stablish hydrogen interactions with AMPPNP. Hydrogen interactions are formed with residues Glu361, Leu325, and Asn330; π-π stacking interactions are stablished with Phe335 and His331 **(Figure 7E** and **F).** The indole ring of **12, 16,** and **31** dock into a lateral cavity flanked by His-331 that is not occupied by HF **(Figure 7F).** The binding pose of **13,** different from the binding poses of **12, 16,** and **31,** provides a hint on the relevance of chirality in the affinity of the binding to PRS target.

A series of halogenated and non-halogenated indolomethyl pyrazino [1,2-***b***]quinazoline-3,6-diones was designed and synthesized. Among all the obtained compounds, **31** and **32** exhibited a potent antibacterial activity against S. *aureus* strains, with MIC values of 4 μg/mL for a reference strain and MIC values of 8 μg/mL for a sensitive clinical isolate

(*S. aureus* 40/61/24) and a methicillin-resistant strain (S. *aureus* 66/1). Isolation of the enantiomers of **31** revealed that only enantiomer (*1S, 4R*), **31a,** was active, indicating that stereochemistry is vital for the referred activity. Comparing with the marine natural product neofiscalin A **(2),** an unexpected two-fold reduction in the MIC was observed. The presence of five stereocenters in neofiscalin A **(2)** makes its synthesis a challenge, while with this one-pot microwave-assisted multicomponent polycondensation of amino acids, highly active compounds were obtained in one single step.

Regarding antimalarial activity, the pyrazino[2,1-*b*]quinazoline-3,6-dione scaffold showed productive derivatives which demonstrated good antimalarial activity *in vitro* against *P. falciparum* strain 3D7. The compounds were not shown to be cytotoxic *in vitro* against non-tumor mammalian cells V79. These compounds did not show significant hemolytic activity in healthy human erythrocytes and also did not inhibit β-hematin *in vitro.* These new antimalarial compounds were hypothetized to interact with the prolyl-tRNA similarly to halofuginone.

In the present disclosure, the antimalarial activity was also evaluated. Each compound was lyophilized and solubilized in DMSO (Sigma-Aldrich) to obtain a final concentration of 5 mM. Some intermediate dilutions were made to achieve the final concentration of 10 μM in the first well of the plate. Chloroquine (CQ; Sigma-Aldrich) was prepared with RPMI-1640 (Invitrogen ™) supplemented with AlbuMAXII (Invitrogen ™) to obtain a final concentration of 10 μM.

In an embodiment, the culture of *P. falciparum* was carried out as follows. Laboratory-adapted *P. falciparum* 3D7 (chloroquine and mefloquine sensitive) were continuously cultured using the method of Trager and Jensen, with previously described modifications (Nogueira *et al,* 2010). Parasites were cultivated at 5 % hematocrit, 37 °C and atmosphere with 5 % of $CO_2$, human serum was replaced with 0.5 % AlbuMAXII (Invitrogen™) in the culture medium. Synchronized cultures were obtained by treatments with a 5 % (m/v) solution of D-sorbitol (Sigma-Aldrich).

In the present disclosure, the in vitro susceptibility assay of *P. falciparum* using SYBR Green I was carried out as follows. All compounds were screened for their *in vitro* antimalarial activity against chloroquine-susceptible (3D7) *P. falciparum* strain, using the Whole cell SYBR Green I assay as previously described with modifications. Briefly, early ring stage parasites (>80 % of rings, 3 % haematocrit and 1 % parasitaemia) were tested in duplicate in a 96-well plate and incubated with the compounds for 48 h (37 °C, 5 % $CO_2$), parasite growth was assessed with SYBRGreenl (Thermo Fisher Scientific). Each compound was tested in concentrations ranging from 10 to 0.001 μM. Fluorescence intensity was measured with a microplate reader with excitation and emission wavelengths of 485 and 535 nm, respectively, and analysed by nonlinear regression using GraphPad Prism 5 demo version to determine $IC_{50}$.

In the present disclosure, the cytotoxicity *in vitro* against mammalian cell was carried out as follows. Cytotoxicity was assessed on the mammalian cell line V79 (Chinese hamster lung), using an MTT based assay, as previously described [38B]. Tests were conducted in triplicate for each compound, at a range of concentrations (800 μM to 0.0512 μM), and with culture media containing 0.5 % DMSO (no drug control); incubation time 24 h. Absorbance was read at 570 nm on a multi-mode microplate reader to produce a log dose-dependence curve. The $LD_{50}$ value for each compound was estimated by non-linear interpolation of the dose-dependence curve (GraphPad Software).

In the present disclosure, the evaluation of hemotoxicity *in vitro* was performed as follows. In a 96-flat bottom plate 3 % HTC, 20 μL of 20 % Triton X-100, and 20 μL of PBS or RPMIc in 2 % DMSO was added. Compounds were tested in a 1:4 serial dilution in concentrations ranging from 10 μM to 0.04 μM. After the incubation of 60 minutes, the plate was centrifuged at 2000 rpm for 5 minutes. 100 mL of Supernatant was transferred to a flat bottom plate. The absorbance reading was made at 450 nm in a Mode (Triad, Dynex Technologies). Two independent tests were carried out in triplicate. The results are presented in the form of a percentage of hemolysis-% hemolysis, obtained by the following formula: % Hemolysis = ABS (sample)/abs (C +) × 100. Whereas C + is a Triton X-100 to 20 % solution RPMIc.

In the present disclosure, the evaluation of inhibition of polymerization of hemozoin was performed as follows. 100 μL of a freshly prepared solution of hemin (ferriprotoporphyrin IX chloride; Sigma-Aldrich) 4mM dissolved in 0.1 M NaOH (Sigma-Aldrich) was mixed with 50 μL of acetic acid (Sigma-Aldrich) and 50 μL of each tested compound. The mixture was incubated for 24 h at 37 °C in a U-bottom 96-well plate. Compounds were tested at the following doses: compounds 12 and 16 at 48.0 μM, 24.0 μM and 12.0 μM, compound 31 at 96.0 μM, 48.0 μM and 24.0 μM. After incubation, the resulting solution was spun down for 15 min at 4000 rpm, the supernatant discarded and the pellet was washed with 200 μL of DMSO (3 washes) after an additional final wash with water (200 μL), the pellet was dissolved in 0.1 M NaOH (200 μL). 50 μL of the solution was transferred to a flat-bottom 96-well clean plate and mixed with 150 μL of water and absorption measured at 405 nm using a multi microplate reader plate reader (Triad, Dynex Technologies).

In the present disclosure, the crystal structure of Prolyl-tRNA Synthetase (PRS) (PDB code: 4YDQ), downloaded from the protein databank (PDB), was used. Structure files of 60 test molecule, four positive (halofuginone (HF), febrifugine (FF, 9), 6-fluorofebrifugine (6F-FF), and tetrahydro quinazolinone febrifugine (Th-FF)) and one negative (chloroquine, CQ, 6) controls were created and minimized using the chemical structure drawing tool Hyperchem 7.5 (Hypercube, FL, USA) and prepared for docking using AutodockTools. Structure-based docking was carried out using AutoDock Vina (Molecular Graphics Lab, CA, USA). The active site was defined by a grid box (X: 19 Å; Y:14 Å; Z: 15 Å) drawn around the PRS crystallographic ligand HF. Default settings for small molecule-protein docking were used throughout the simulations. Top 9 poses were collected for each molecule and the lowest docking score value was associated with the more favorable binding conformation. PyMol1.3 (Schrödinger, NY, USA) was used for visual inspection of results and graphical

representations. To validate the docking approach for the protein structure used, the respective co-crystallized inhibitor HF was docked to the active site using Autodock Vina **(Figure 7).**

Compounds synthetized and tested in the present disclosure:

| | | | |
|---|---|---|---|
| **10** | 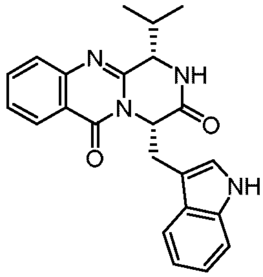<br>(1*S*,4*S*)-4-((1*H*-indol-3-yl)methyl)-1-isopropyl-1,2-dihydro-6*H*-pyrazino[2,1-*b*]quinazoline-3,6(4*H*)-dione | **28** | 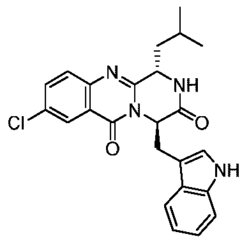<br>(1*S*,4*R*)-4-((1*H*-indol-3-yl)methyl)-8-chloro-1-isobutyl-1,2-dihydro-6*H*-pyrazino[2,1-*b*]quinazoline-3,6(4*H*)-dione |
| **11** | 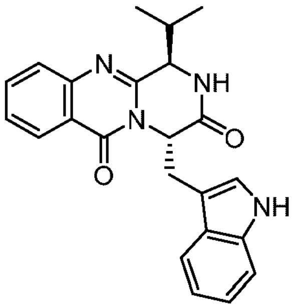<br>(1*R*,4*S*)-4-((1*H*-indol-3-yl)methyl)-1-isopropyl-1,2-dihydro-6*H*-pyrazino[2,1-*b*]quinazoline-3,6(4*H*)-dione | **29** | 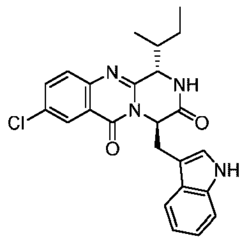<br>(1*S*,4*R*)-4-((1*H*-indol-3-yl)methyl)-1-((*S*)-*sec*-butyl)-8-chloro-1,2-dihydro-6*H*-pyrazino[2,1-*b*]quinazoline-3,6(4*H*)-dione |
| **12** | 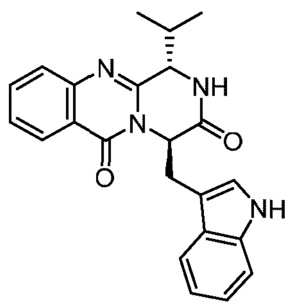<br>(1*S*,4*R*)-4-((1*H*-indol-3-yl)methyl)-1-isopropyl-1,2-dihydro-6*H*-pyrazino[2,1-*b*]quinazoline-3,6(4*H*)-dione | **30** | 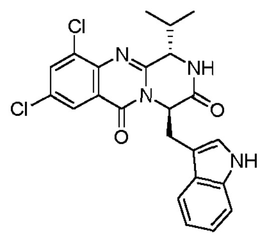<br>(1*S*,4*R*)-4-((1*H*-indol-3-yl)methyl)-8,10-dichloro-1-isopropyl-1,2-dihydro-6*H*-pyrazino[2,1-*b*]quinazoline-3,6(4*H*)-dione |
| **13** | 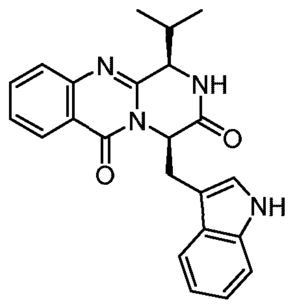<br>(1*R*,4*R*)-4-((1*H*-indol-3-yl)methyl)-1-isopropyl-1,2-dihydro-6*H*-pyrazino[2,1-*b*]quinazoline-3,6(4*H*)-dione | **31** | 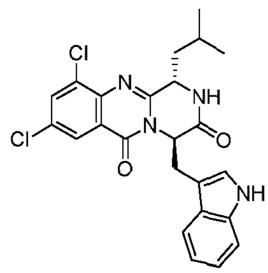<br>(1*S*,4*R*)-4-((1*H*-indol-3-yl)methyl)-8,10-dichloro-1-isobutyl-1,2-dihydro-6*H*-pyrazino[2,1-*b*]quinazoline-3,6(4*H*)-dione |

(continued)

| | | | |
|---|---|---|---|
| **14** | 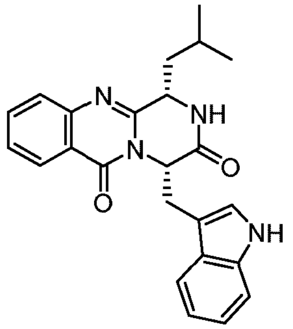 (1*S*,4*S*)-4-((1*H*-indol-3-yl)methyl)-1-isobutyl-1,2-dihydro-6*H*-pyrazino[2,1-*b*]quinazoline-3,6(4*H*)-dione | **32** | 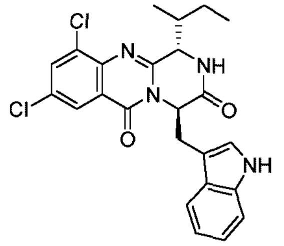 (1*S*,4*R*)-4-((1*H*-indol-3-yl)methyl)-1-((*S*)-*sec*-butyl)-8,10-dichloro-1,2-dihydro-6*H*-pyrazino[2,1-*b*]quinazoline-3,6(4*H*)-dione |
| **15** | 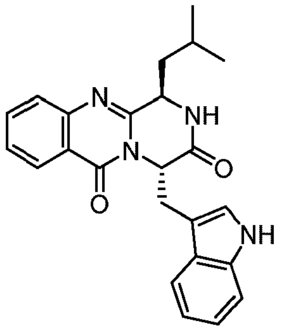 (1*R*,4*S*)-4-((1*H*-indol-3-yl)methyl)-1-isobutyl-1,2-dihydro-6*H*-pyrazino[2,1-*b*]quinazoline-3,6(4*H*)-dione | **35** | 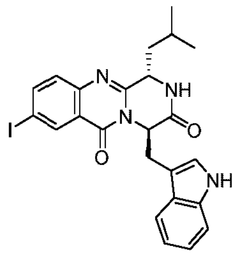 (1*S*,4*R*)-4-((1*H*-indol-3-yl)methyl)-8-iodo-1-isobutyl-1,2-dihydro-6*H*-pyrazino[2,1-*b*]quinazoline-3,6(4*H*)-dione |
| **16** | 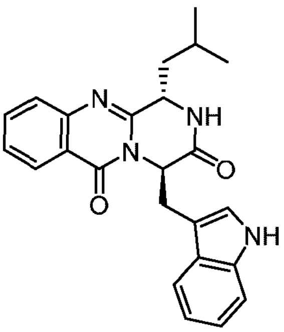 (1*S*,4*R*)-4-((1*H*-indol-3-yl)methyl)-1-isobutyl-1,2-dihydro-6*H*-pyrazino[2,1-*b*]quinazoline-3,6(4*H*)-dione | **36** | 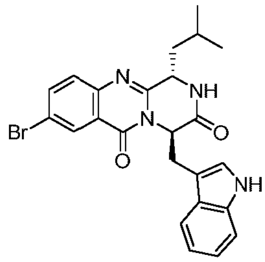 (1*S*,4*R*)-4-((1*H*-indol-3-yl)methyl)-8-bromo-1-isobutyl-1,2-dihydro-6*H*-pyrazino[2,1-*b*]quinazoline-3,6(4*H*)-dione |
| **17** | 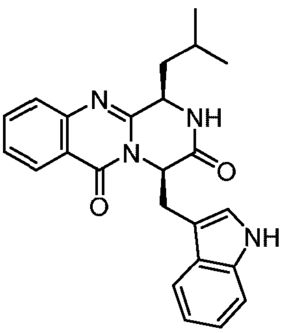 (1*R*,4*R*)-4-((1*H*-indol-3-yl)methyl)-1-isobutyl-1,2-dihydro-6*H*-pyrazino[2,1-*b*]quinazoline-3,6(4*H*)-dione | **37** | 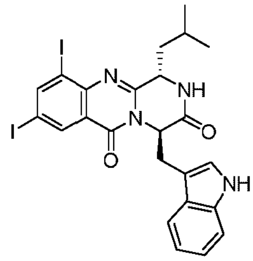 (1*S*,4*R*)-4-((1*H*-indol-3-yl)methyl)-8,10-diiodo-1-isobutyl-1,2-dihydro-6*H*-pyrazino[2,1-*b*]quinazoline-3,6(4*H*)-dione |

(continued)

| | | | |
|---|---|---|---|
| **26** | 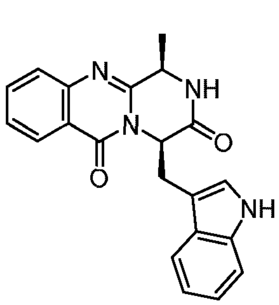<br>(1*R*,4*R*)-4-((1*H*-indol-3-yl)methyl)-1-methyl-1,2-dihydro-6*H*-pyrazino[2,1-*b*]quinazoline-3,6(4*H*)-dione | **38** | 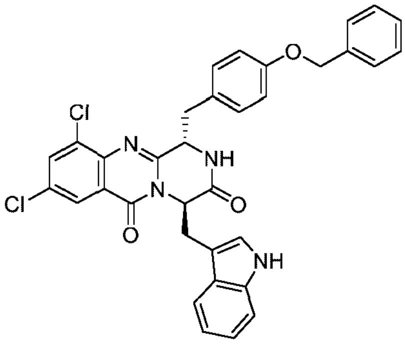<br>(1*S*,4*R*)-4-((1*H*-indol-3-yl)methyl)-1-(4-(benzyloxy)benzyl)-8,10-dichloro-1,2-dihydro-6*H*-pyrazino[2,1-*b*]quinazoline-3,6(4*H*)-dione |
| **18** | 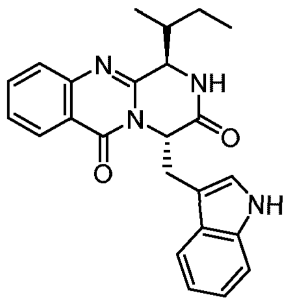<br>(1*R*,4*S*)-4-((1*H*-indol-3-yl)methyl)-1-((*R*)-*sec*-butyl)-1,2-dihydro-6*H*-pyrazino[2,1-*b*]quinazoline-3,6(4*H*)-dione | **39** | 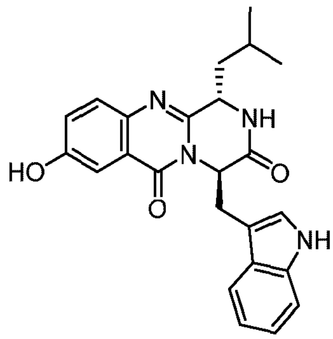<br>(1*S*,4*R*)-4-((1*H*-indol-3-yl)methyl)-8-hydroxy-1-isobutyl-1,2-dihydro-6*H*-pyrazino[2,1-*b*]quinazoline-3,6(4*H*)-dione |
| **19** | 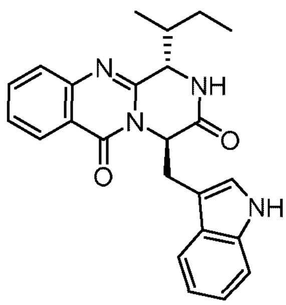<br>(1*S*,4*R*)-4-((1*H*-indol-3-yl)methyl)-1-((*S*)-sec-butyl)-1,2-dihydro-6*H*-pyrazino[2,1-*b*]quinazoline-3,6(4*H*)-dione | **40** | 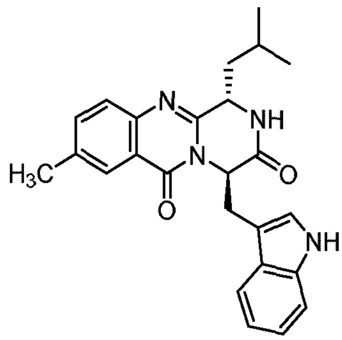<br>(1*S*,4*R*)-4-((1*H*-indol-3-yl)methyl)-1-isobutyl-8-methyl-1,2-dihydro-6*H*-pyrazino[2,1-*b*]quinazoline-3,6(4*H*)-dione |
| **20** | 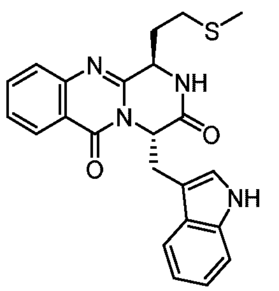<br>(1*R*,4*S*)-4-((1*H*-indol-3-yl)methyl)-1-(2-(methylthio)ethyl)-1,2-dihydro-6*H*-pyrazino[2,1-*b*]quinazoline-3,6(4H)-dione | **41** | 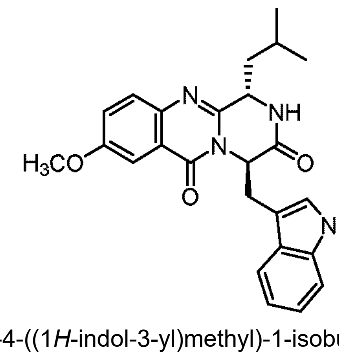<br>(1*S*,4*R*)-4-((1*H*-indol-3-yl)methyl)-1-isobutyl-8-methoxy-1,2-dihydro-6 *H*-pyrazino[2,1-*b*]quinazoline-3,6(4*H*)-dione |

(continued)

| | | | |
|---|---|---|---|
| **21** | 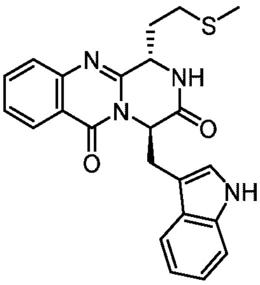 (1*S*,4*R*)-4-((1*H*-indol-3-yl) methyl)-1-(2-(methylthio)ethyl)-1,2-dihydro-6*H*-pyrazino[2,1-*b*]quinazoline-3,6(4*H*)-dione | **44** | 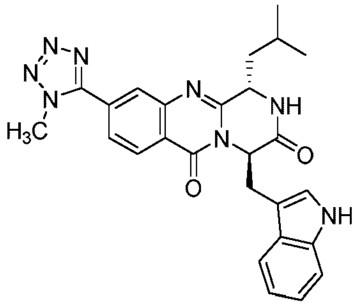 (1*S*,4*R*)-4-((1*H*-indol-3-yl)methyl)-1-isobutyl-9-(1-methyl-1*H*-tetrazol-5-yl)-1,2-dihydro-6*H*-pyrazino[2,1-*b*] quinazoline-3,6(4*H*)-dione |
| **22** | 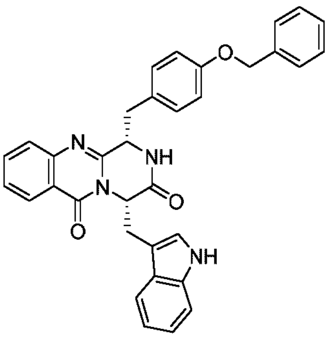 (1*S*,4*S*)-4-((1*H*-indol-3-yl)methyl)-1-(4-(benzyloxy)benzyl)-1,2-dihydro-6*H*-pyrazino [2,1-*b*]quinazoline-3,6(4*H*)-dione | **42** | 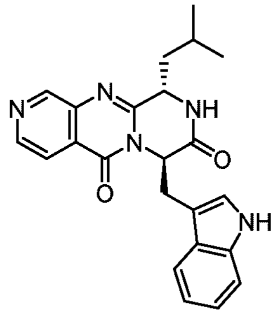 (1*S*,4*R*)-4-((1*H*-indol-3-yl)methyl)-1-isobutyl-1,2-dihydro-6*H*-pyrazino[1,2-*a*]pyrido[3,4-*d*]pyrimidine-3,6(4*H*)-dione |
| **23** | 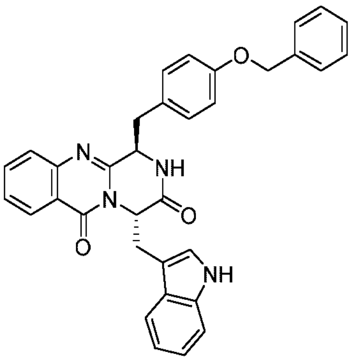 (1*R*,4*S*)-4-((1*H*-indol-3-yl)methyl)-1-(4-(benzyloxy)benzyl)-1,2-dihydro-6*H*-pyrazino [2,1-*b*]quinazoline-3,6(4*H*)-dione | **43** | 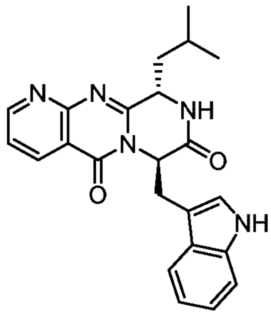 (7*R*,10*S*)-7-((1*H*-indol-3-yl)methyl)-10-isobutyl-9,10-dihydro-5*H*-pyrazino[1,2-*a*]pyrido[2,3-*d*]pyrimidine-5,8(7*H*)-dione |
| **24** | 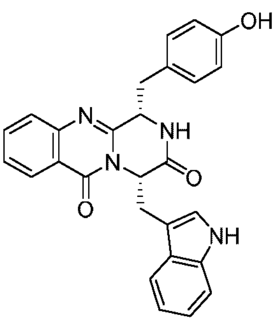 (1*S*,4*S*)-4-((1*H*-indol-3-yl)methyl)-1-(4-hydroxybenzyl)-1,2-dihydro-6*H*-pyrazino[2,1-*b*]quinazoline-3,6(4*H*)-dione | **45** | 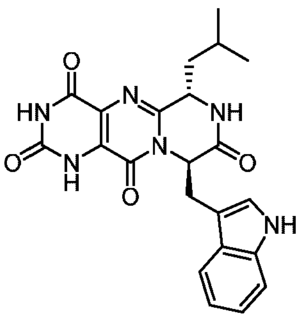 (6*S*,9*R*)-9-((1*H*-indol-3-yl)methyl)-6-isobutyl-6,7-dihydro-1*H*-pyrimido[5,4-*d*]pyrimidine-2,4,8,11(3*H*,9*H*)-tetraone |

(continued)

| | | | |
|---|---|---|---|
| **25** | 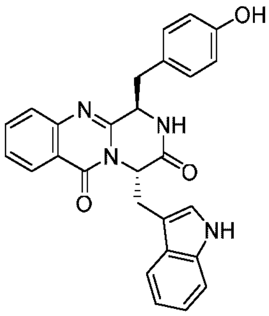 (1*R*,4*S*)-4-((1*H*-indol-3-yl)methyl)-1-(4-hydroxybenzyl)-1,2-dihydro-6*H*-pyrazino[2,1-*b*]quinazoline-3,6(4*H*)-dione | **46** | 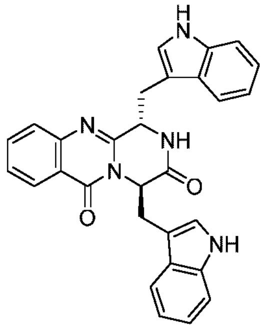 (1*S*,4*R*)-1,4-bis((1*H*-indol-3-yl)methyl)-1,2-dihydro-6*H*-pyrazino[2,1-*b*]quinazoline-3,6(4*H*)-dione |
| **27** | 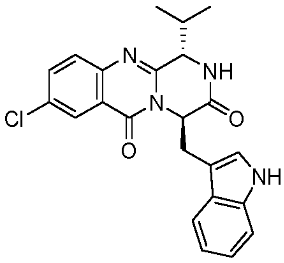 (1*S*,4*R*)-4-((1*H*-indol-3-yl)methyl)-8-chloro-1-isopropyl-1,2-dihydro-6*H*-pyrazino[2,1-*b*]quinazoline-3,6(4*H*)-dione | | |

REFERENCES

1. Resende, D. I. S. P.; Boonpothong, P.; Sousa, E.; Kijjoa, A.; Pinto, M. M. M., Chemistry of the fumiquinazolines and structurally related alkaloids. Natural Product Reports 2019, 36 (1), 7-34.

2. Bessa, L. J.; Buttachon, S.; Dethoup, T.; Martins, R.; Vasconcelos, V.; Kijjoa, A.; da Costa, P. M., Neofiscalin A and fiscalin C are potential novel indole alkaloid alternatives for the treatment of multidrug-resistant Gram-positive bacterial infections. FEMS Microbiol. Lett. 2016, 363 (15), 1-5.

3. Long, S.; Resende, D. I. S. P.; Kijjoa, A.; Silva, A. M. S.; Pina, A.; Fernández-Marcelo, T.; Vasconcelos, M. H.; Sousa, E.; Pinto, M. M. M., Antitumor Activity of Quinazolinone Alkaloids Inspired by Marine Natural Products. Marine Drugs 2018, 16 (8).

4. Long, S.; Resende, D. I. S. P.; Kijjoa, A.; Silva, A. M. S.; Fernandes, R.; Xavier, C. P. R.; Vasconcelos, M. H.; Sousa, E.; Pinto, M. M. M., Synthesis of New Proteomimetic Quinazolinone Alkaloids and Evaluation of Their Neuroprotective and Antitumor Effects. Molecules 2019, 24 (3), 534.

5. Gamo FJ, Sanz LM, Vidal J, De Cozar C, Alvarez E, Lavandera JL, Vanderwall DE, Green DVS, Kumar V, Hasan S et al: Thousands of chemical starting points for antimalarial lead identification. Nature 2010, 465(7296):305-310.


**Claims**

**1.** Compound of formula I

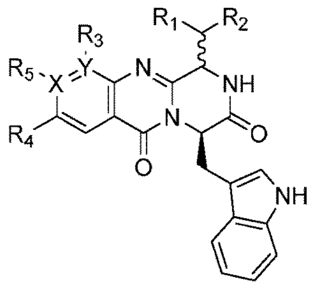

wherein

$R^1$, $R^2$, $R^3$, $R^4$, $R^5$, X and Y are independently selected from each other;
$R^1$ and $R^2$ are selected from H or $CH_3$ or $CH(CH_3)_2$ or $CH_2CH_3$;
$R^3$ is selected from H or Cl or Br or F or OH or $OCH_3$;
$R^4$ is selected from H or Cl or Br or I or F or OH or $OCH_3$;
$R^5$ is H or

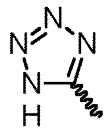

;

and
X and Y are selected from N or C;
or a pharmaceutically acceptable salt or solvate thereof;
provided that
if X and Y are C then $R^4$ is different from H; or
if X and Y are C then $R^4$ is H and $R^5$ is

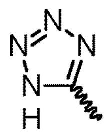

;

or
if X is N then $R^5$ is absent;
if Y is N then $R^3$ is absent.

**2.** Compound according to the previous claim, wherein X and Y are C and/or $R^1$ is H or $CH_3$.

**3.** Compound according to any of the previous claims wherein $R^2$ is $CH_3$ or $CH(CH_3)_2$ or $CH_2CH_3$.

**4.** Compound according to any of the previous claims, wherein $R^3$ is H or Cl.

**5.** Compound according to any of the previous claims, wherein $R^4$ is Cl or I, preferably $R^4$ is Cl.

**6.** Compound according to any of the previous claims, wherein $R^5$ is H.

**7.** Compound according to any of the previous claims, wherein the compound is

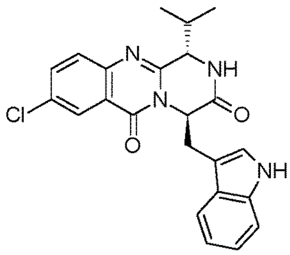

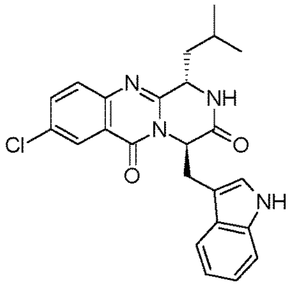

or,

or,

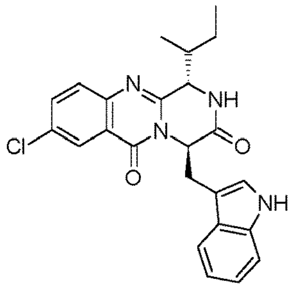

,

preferably wherein the compound is

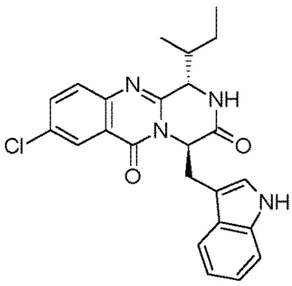

.

**8.** Compound according to any of the previous claims 1-6, wherein the compound is

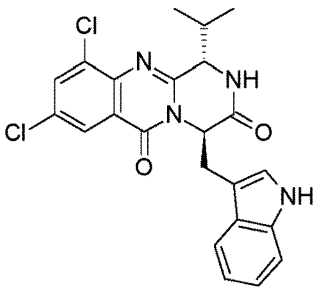

or,

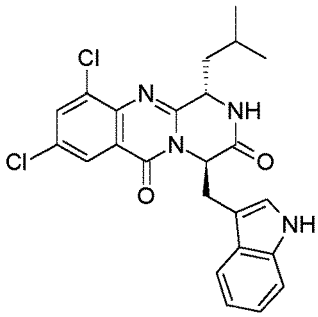

or,

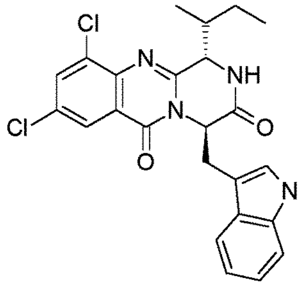

or,

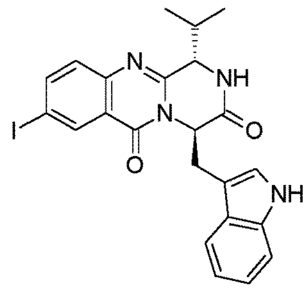

or,

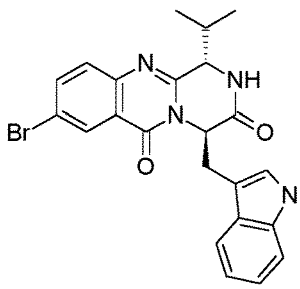

or,

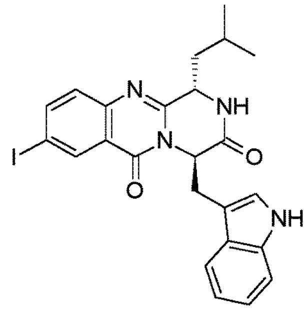

or,

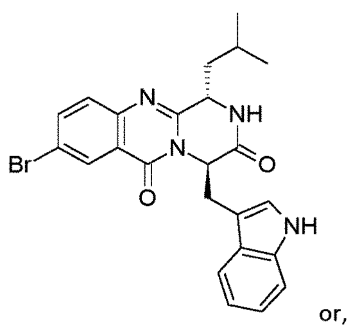

or,

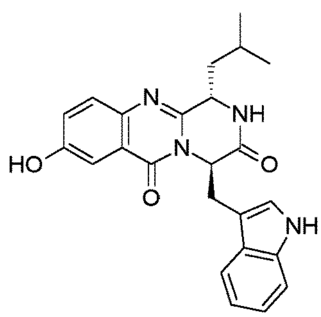

or,

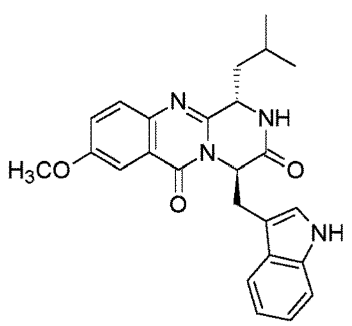

or,

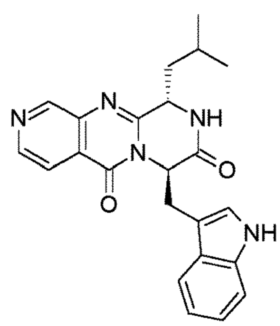

or,

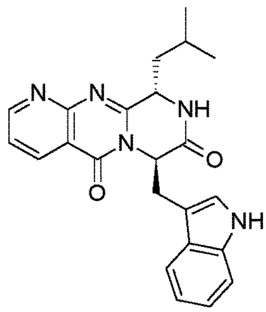

,

preferably wherein the compound is

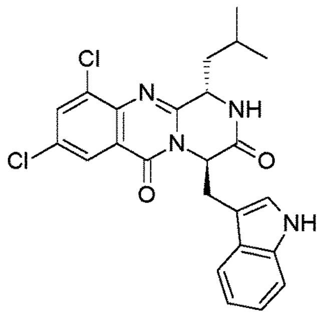

or

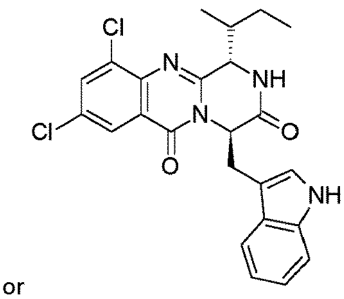

.

**9.** Compound of formula

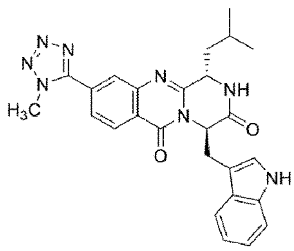

.

**10.** Compound according to any of the previous claims for use in medicine, preferably for use in the treatment or prevention of bacterial infections and/or for use in the treatment or prevention of malaria.

**11.** Compound for use in the treatment or prevention of malaria according to the previous claim 10, wherein the compound is

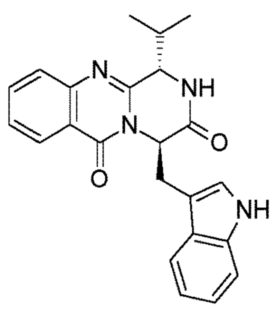

or,

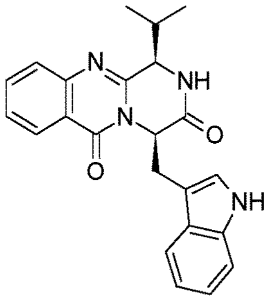 or, 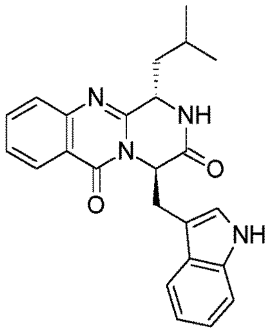 or, 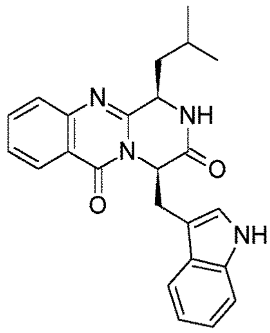

or,

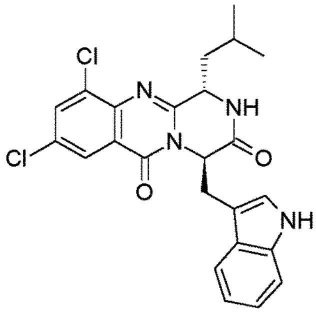 .

**12.** Compound according to the previous claim 10 for use in the treatment of Gram-positive bacterial infections, preferably caused by *Staphylococcus* spp. and/or *Enterococcus* spp., more preferably caused by *Staphylococcus* aureus and/or *Enterococcus faecalis,* wherein the compound is:

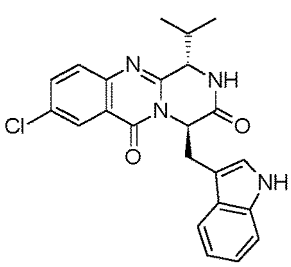 or, 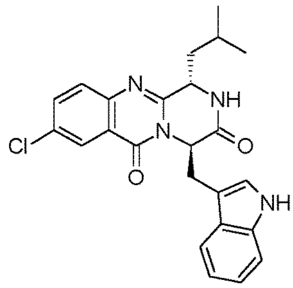 or, 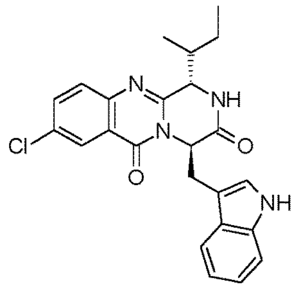 ,

preferably wherein the compound is

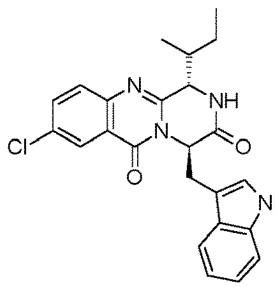 .

**13.** Compound according to the previous claim 10 and for use in the treatment of Gram-positive bacterial infections, preferably caused by *Staphylococcus* aureus, wherein the compound is

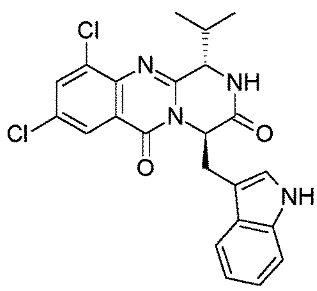

or,

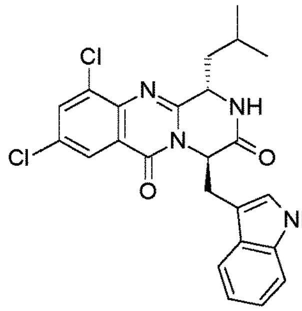

or,

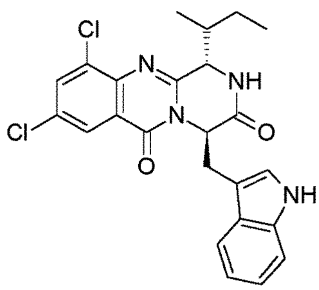

or,

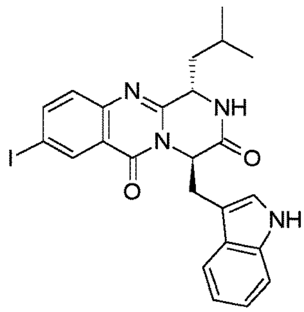

or,

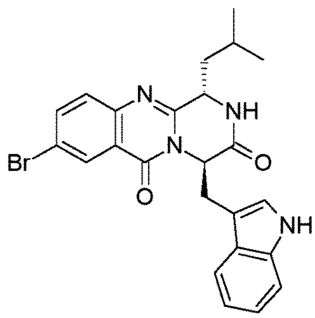

or, preferably wherein the compound is

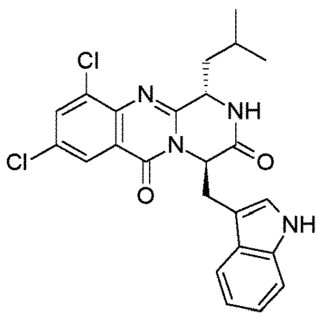

or

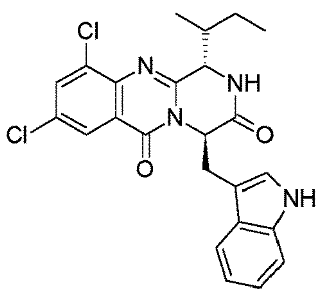

.

14. Composition comprising the compound according to any of the previous claims 1-9, and a pharmaceutical acceptable excipient wherein the compound is in a therapeutically effective amount.

15. Composition according to the previous claim further comprising an antibiotic, preferably wherein the antibiotic is a fluoroquinolone, preferably selected from ciprofloxacin, norfloxacin, pefloxacin, enofloxacin, ofloxacin, levofloxacin, moxifloxacin, or mixtures thereof.

## Patentansprüche

**1.** Zusammensetzung der Formel I

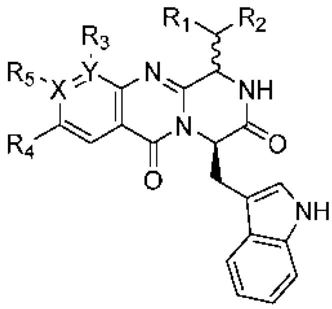

wobei

$R^1$, $R^2$, $R^3$, $R^4$, $R^5$, X, und Y unabhängig voneinander ausgewählt werden;
$R^1$ und $R^2$ ausgewählt werden aus H oder $CH_3$ oder $CH(CH_3)_2$ oder $CH_2CH_3$;
$R^3$ ausgewählt wird aus H oder Cl oder Br oder F oder OH oder $OCH_3$;
$R^4$ ausgewählt wird aus H oder Cl oder Br oder I oder F oder OH oder $OCH_3$;
$R^5$ H ist oder

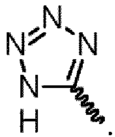

;

und
X und Y ausgewählt werden aus N oder C;
oder ein pharmazeutisch verträgliches Salz oder Solvat davon;
vorausgesetzt, dass
wenn X und Y C sind, dann ist $R^4$ verschieden von H; oder
wenn X und Y C sind, dann ist $R^4$ H und $R^5$

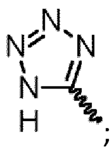

;

oder
wenn X N ist, dann ist $R^5$ nicht vorhanden;
wenn Y N ist, dann ist $R^3$ nicht vorhanden.

**2.** Verbindung nach dem vorangehenden Anspruch, wobei X und Y C sind und/oder $R^1$ H oder $CH_3$ ist.

**3.** Verbindung nach einem der vorangehenden Ansprüche, wobei $R^2$ $CH_3$ oder $CH(CH_3)_2$ oder $CH_2CH_3$ ist.

**4.** Verbindung nach einem der vorangehenden Ansprüche, wobei $R^3$ H oder Cl ist.

**5.** Verbindung nach einem der vorangehenden Ansprüche, wobei $R^4$ Cl oder I, bevorzugt $R^4$ Cl ist.

**6.** Verbindung nach einem der vorangehenden Ansprüche, wobei $R^5$ H ist.

**7.** Verbindung nach einem der vorangehenden Ansprüche, wobei die Verbindung

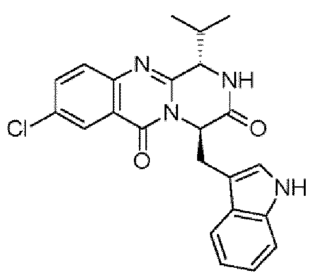 oder, 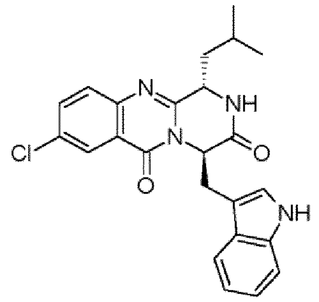

oder,

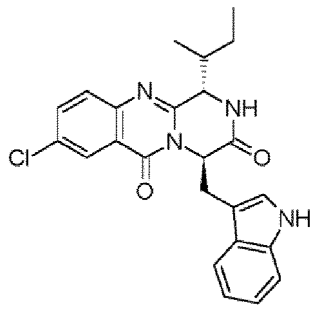

ist, wobei die Verbindung bevorzugt

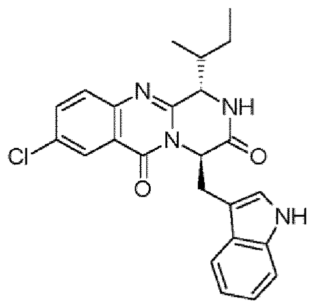 i

ist.

**8.** Verbindung nach einem der vorangehenden Ansprüche 1-6, wobei die Verbindung

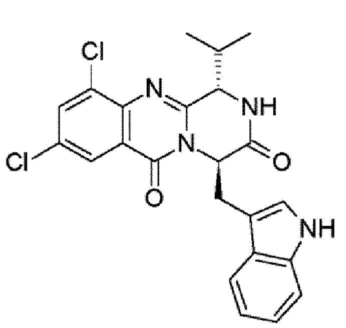 oder, 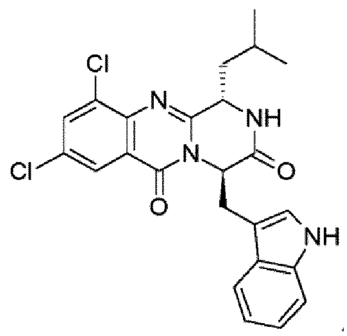 .

oder,

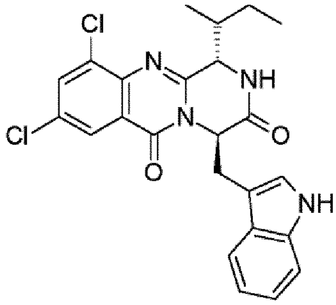

oder,

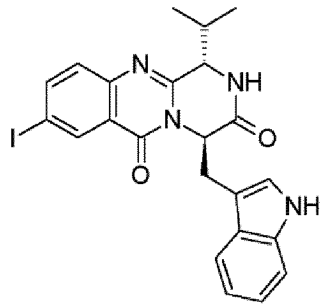

oder,

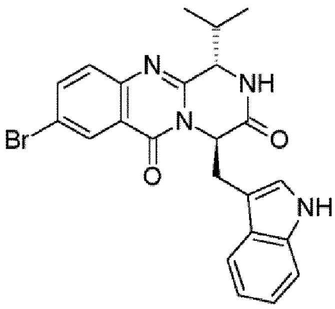

oder,

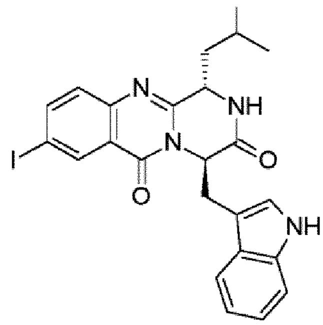

oder,

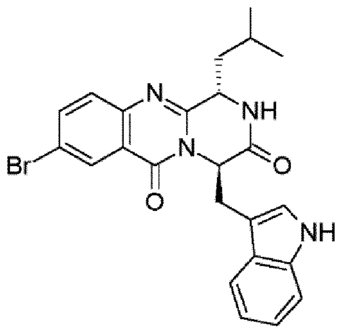

oder,

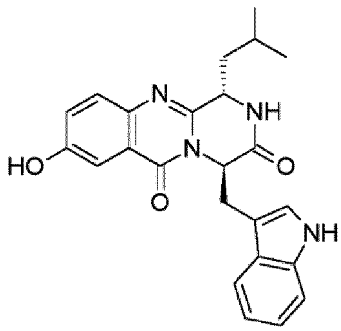

oder,

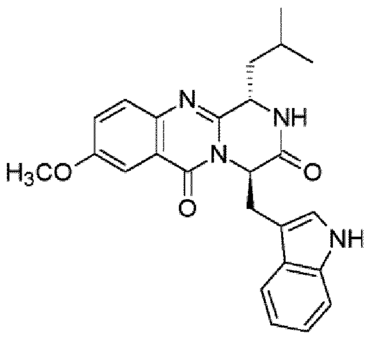

oder,

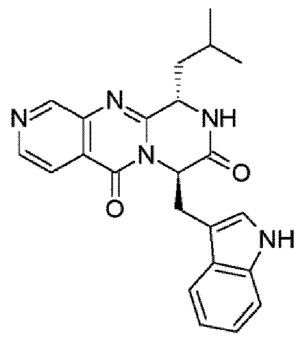

oder,

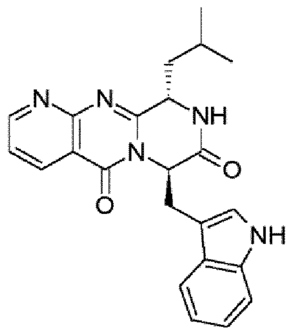

wobei die Verbindung bevorzugt

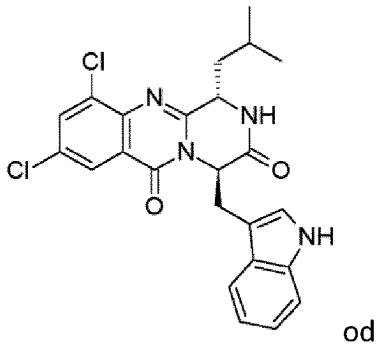 oder 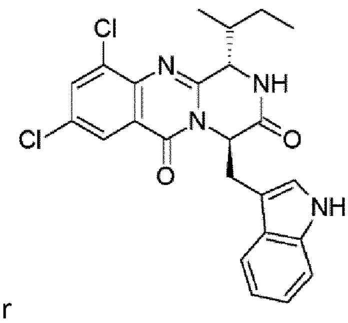

ist.

**9.** Zusammensetzung der Formel

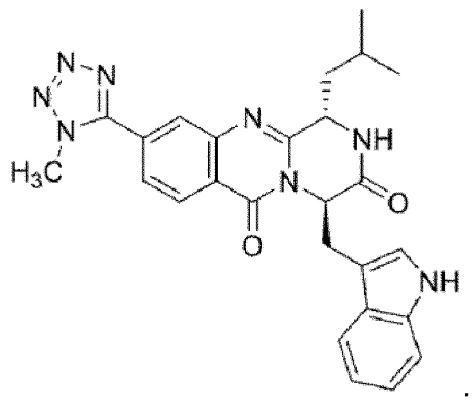

.

**10.** Verbindung nach einem der vorangehenden Ansprüche zur Verwendung in der Medizin, bevorzugt zur Verwendung bei der Behandlung oder Prävention von bakteriellen Infektionen und/oder zur Verwendung bei der Behandlung oder Prävention von Malaria.

**11.** Verbindung zur Verwendung bei der Behandlung oder Prävention von Malaria nach dem vorangehenden Anspruch 10, wobei die Verbindung

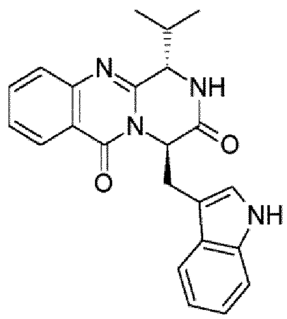

oder,

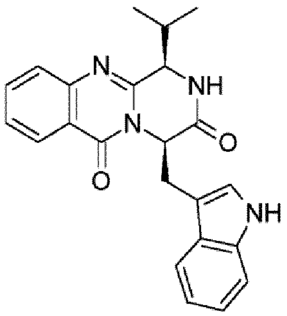 oder, 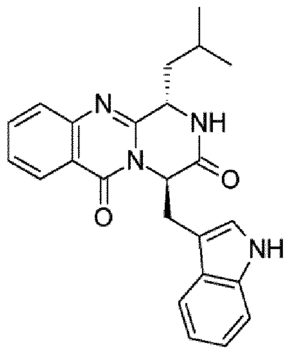 oder, 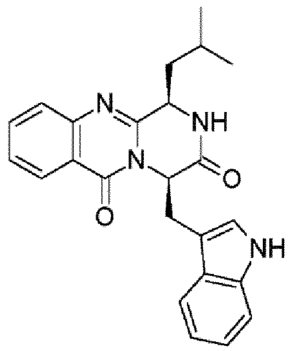

oder,

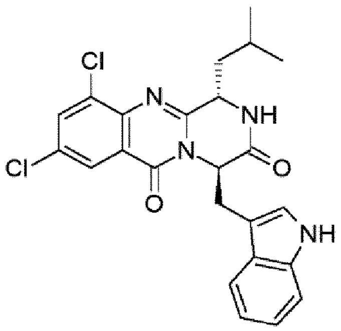

ist.

**12.** Verbindung nach dem vorangehenden Anspruch 10 zur Verwendung bei der Behandlung von Infektionen mit grampositiven Bakterien, bevorzugt verursacht durch *Staphylococcus spp.* und/oder *Enterococcus spp.,* besonders bevorzugt verursacht durch *Staphylococcus aureus* und/oder *Enterococcus faecalis,* wobei die Verbindung:

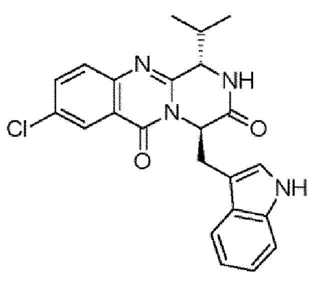 oder, 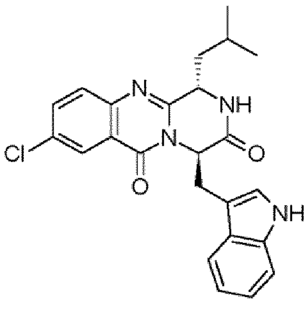 oder, 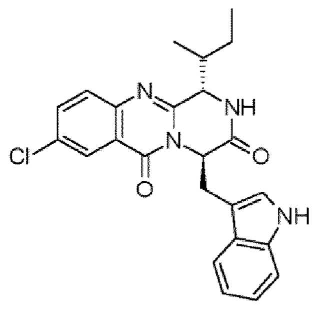

ist, wobei die die Verbindung bevorzugt

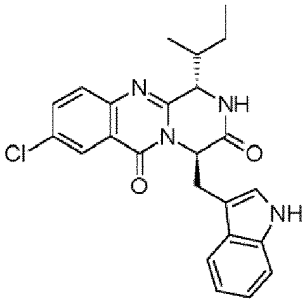

ist.

**13.** Verbindung nach dem vorangehenden Anspruch 10 und zur Verwendung bei der Behandlung von Infektionen mit grampositiven Bakterien, bevorzugt verursacht durch *Staphylococcus aureus,* wobei die Verbindung

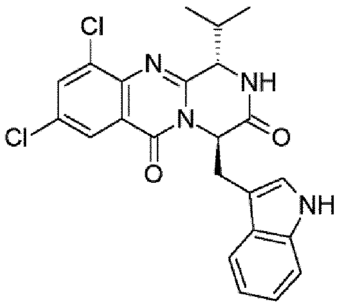

oder,

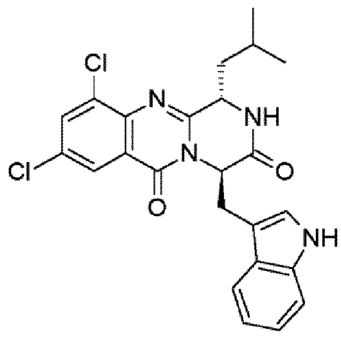

oder,

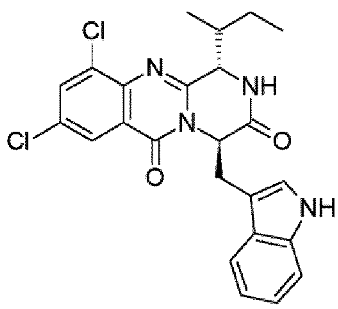

oder,

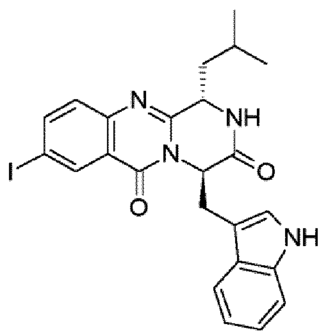

oder,

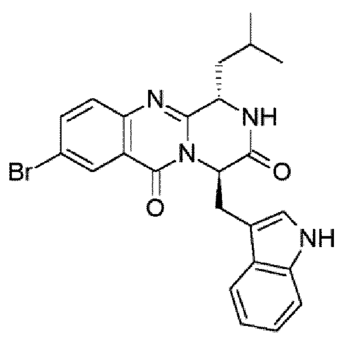

ist oder, wobei die Zusammensetzung bevorzugt

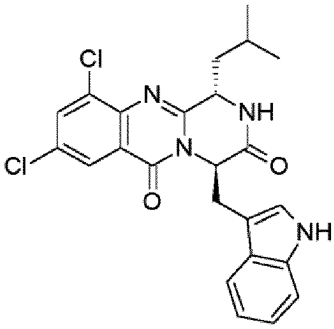

oder

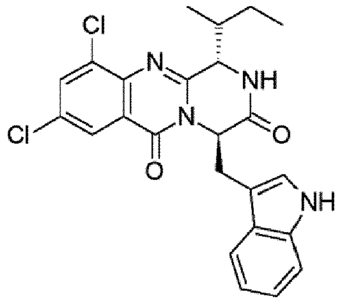

ist.

**14.** Zusammensetzung, umfassend die Verbindung nach einem der vorangehenden Ansprüche 1-9 und einen pharmazeutisch verträglichen Hilfsstoff, wobei die Verbindung in einer therapeutisch wirksamen Menge vorliegt.

**15.** Zusammensetzung nach dem vorangehenden Anspruch, ferner umfassend ein Antibiotikum, wobei das Antibiotikum bevorzugt ein Fluorchinolon ist, das bevorzugt ausgewählt ist aus Ciprofloxacin, Norfloxacin, Pefloxacin, Enofloxacin, Ofloxacin, Levofloxacin, Moxifloxacin oder Mischungen davon.

## Revendications

**1.** Composé de formule I

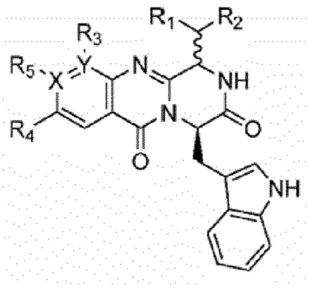

dans lequel

$R^1$, $R^2$, $R^3$, $R^4$, $R^5$, X et Y sont choisis indépendamment les uns des autres ;
$R^1$ et $R^2$ sont choisis parmi H ou $CH_3$ ou $CH(CH_3)_2$ ou $CH_2CH_3$ ;
$R^3$ est choisi parmi H ou Cl ou Br ou F ou OH ou $OCH_3$ ;
$R^4$ est choisi parmi H ou Cl ou Br ou I ou F ou OH ou $OCH_3$ ;
$R^5$ est H ou

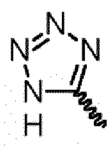

;

et
X et Y sont choisis parmi N ou C ;
ou un sel ou solvate pharmaceutiquement acceptable de ceux-ci ;
à condition que
si X et Y sont C, $R^4$ soit différent de H ; ou
si X et Y sont C, $R^4$ soit H et $R^5$ soit

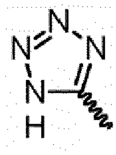 ;

ou
si X est N, $R^5$ soit absent ;
si Y est N, $R^3$ soit absent.

2. Composé selon la revendication précédente, dans lequel X et Y sont C et/ou $R^1$ est H ou $CH_3$.

3. Composé selon l'une quelconque des revendications précédentes, dans lequel $R^2$ est $CH_3$ ou $CH(CH_3)_2$ ou $CH_2CH_3$.

4. Composé selon l'une quelconque des revendications précédentes, dans lequel $R^3$ est H ou Cl.

5. Composé selon l'une quelconque des revendications précédentes, dans lequel $R^4$ est Cl ou I, $R^4$ étant préférablement Cl.

6. Composé selon l'une quelconque des revendications précédentes, dans lequel $R^5$ est H.

7. Composé selon l'une quelconque des revendications précédentes, dans lequel le composé est

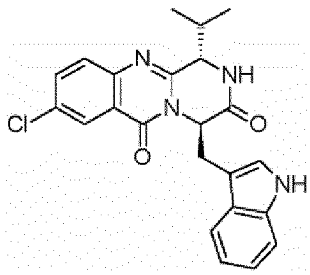 ou, 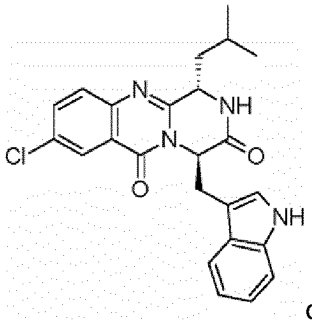

ou,

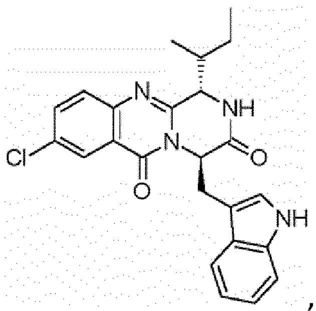 ,

préférablement dans lequel le composé est

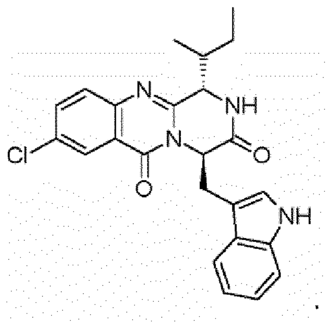

8. Composé selon l'une quelconque des revendications précédentes 1-6, dans lequel le composé est

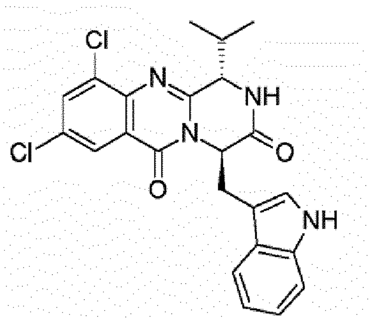

ou,

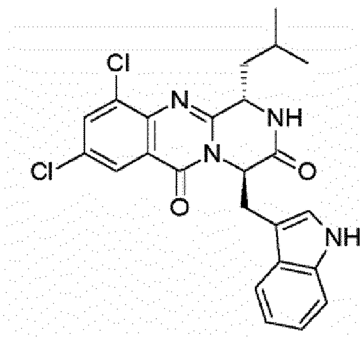

ou,

ou,

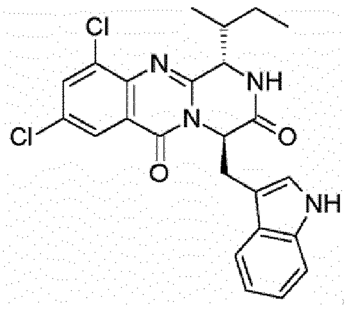

ou,

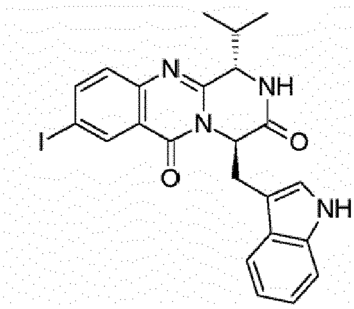

ou,

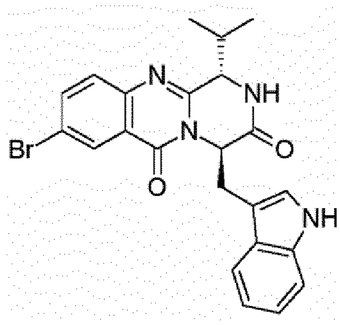

ou,

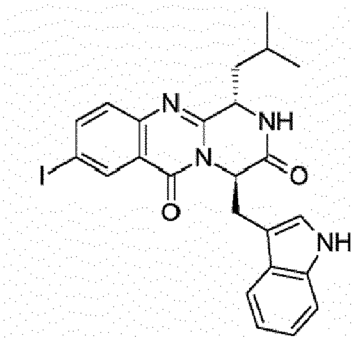

ou,

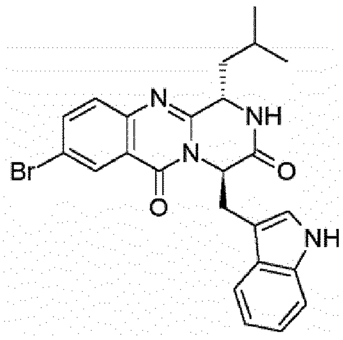 ou, 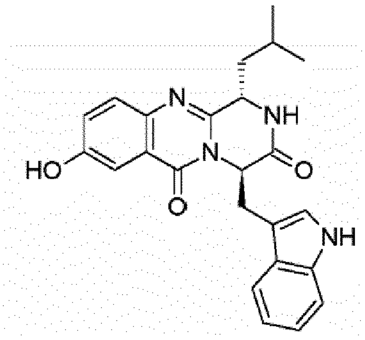

ou,

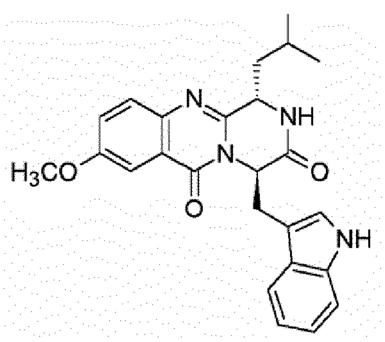 ou, 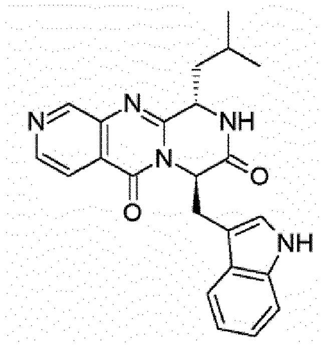

ou,

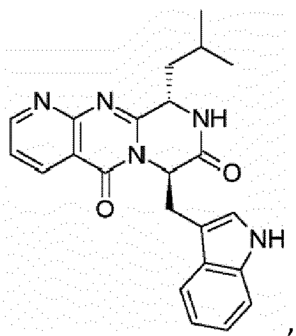,

préférablement dans lequel le composé est

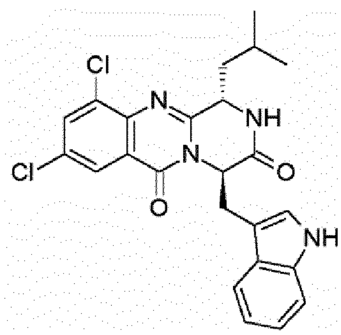 ou, 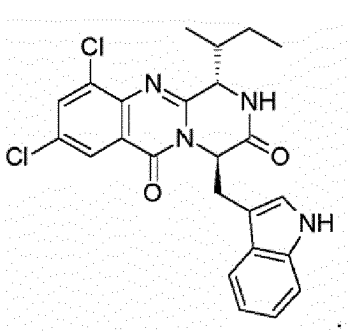.

**9.** Composé de formule

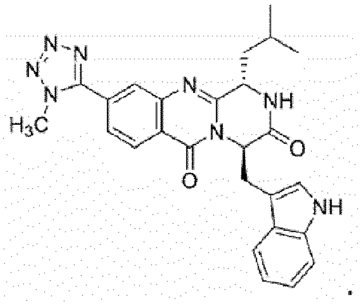

**10.** Composé selon l'une quelconque des revendications précédentes destiné à être utilisé en médecine, préférablement destiné à être utilisé dans le traitement ou la prévention d'infections bactériennes et/ou destiné à être utilisé dans le traitement ou la prévention du paludisme.

**11.** Composé destiné à être utilisé dans le traitement ou la prévention du paludisme selon la revendication précédente 10, dans lequel le composé est

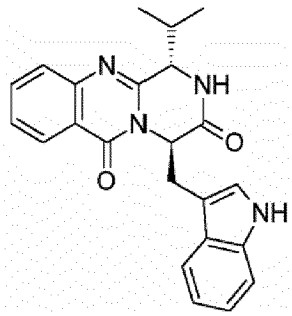

ou,

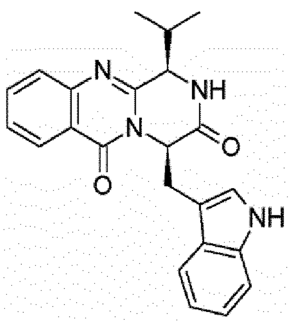 ou, 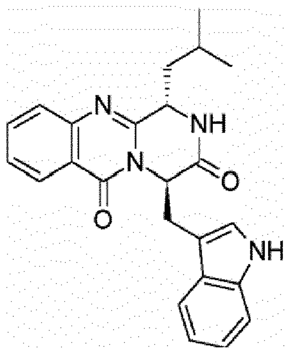 ou, 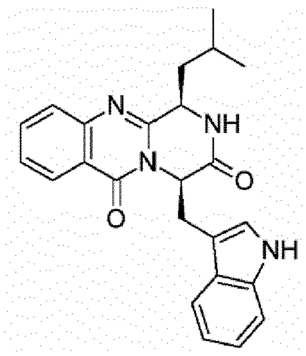

ou,

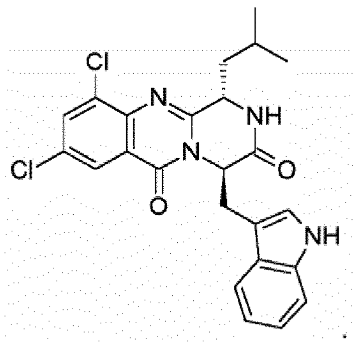

**12.** Composé selon la revendication précédente 10 destiné à être utilisé dans le traitement d'infections bactériennes gram-positives, préférablement causées par le *Staphylococcus* spp. et/ou *l'Enterococcus* spp., plus préférablement causées par le *Staphylococcus* aureus et/ou l'*Enterococcus faecalis,* dans lequel le composé est :

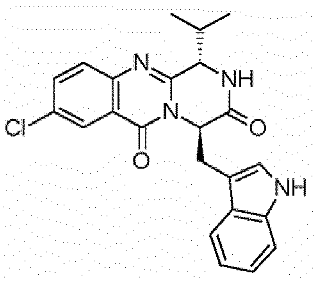 ou, 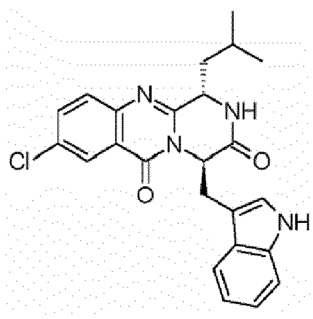 ou, 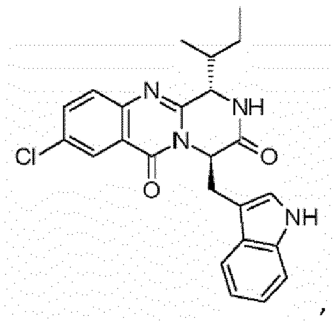 ,

préférablement dans lequel le composé est

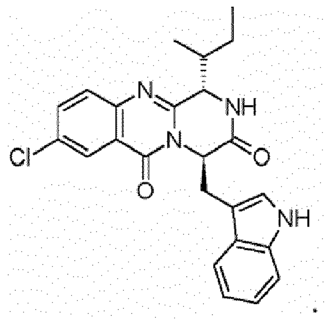 .

**13.** Composé selon la revendication précédente 10 et destiné à être utilisé dans le traitement d'infections bactériennes gram-positives, préférablement causées par le *Staphylococcus* aureus, dans lequel le composé est

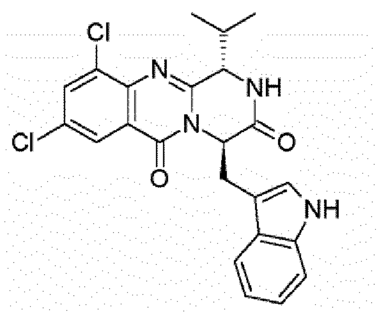 ou, 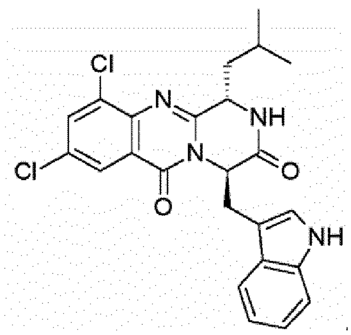 .

ou,

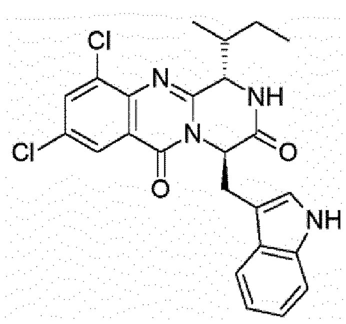 ou, 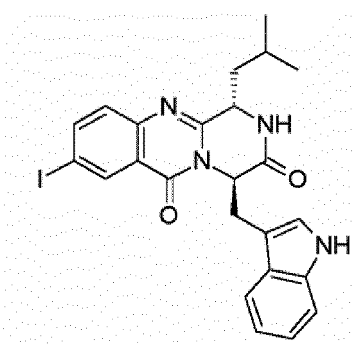

ou,

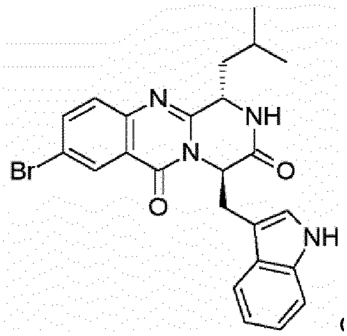

ou, préférablement dans lequel le composé est

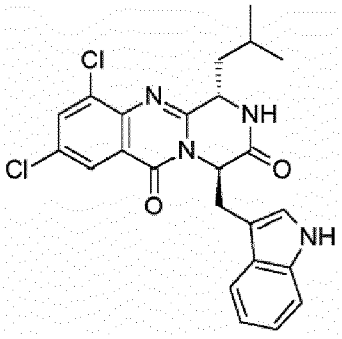

ou,

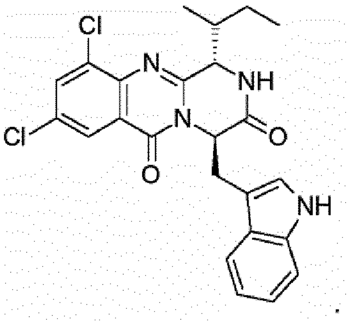

**14.** Composition comprenant le composé selon l'une quelconque des revendications précédentes 1-9, et un excipient pharmaceutiquement acceptable dans lequel le composé est présent en quantité thérapeutiquement efficace.

**15.** Composition selon la revendication précédente comprenant également un antibiotique, préférablement dans laquelle l'antibiotique est une fluoroquinolone, préférablement choisie parmi la ciprofloxacine, norfloxacine, péfloxacine, énofloxacine, ofloxacine, lévofloxacine, moxifloxacine, ou des mélanges de celles-ci.

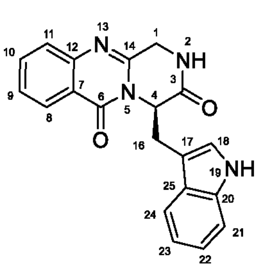

**1**, glyantrypine

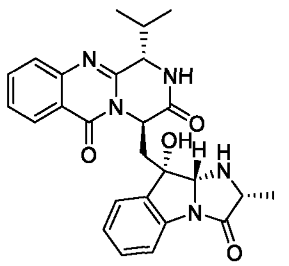

**2**, neofiscalin A

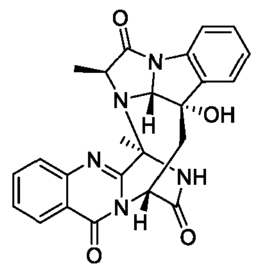

**3**, 2'-*epi*-fumiquinazoline D

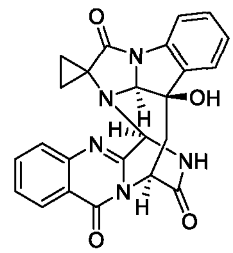

**4**, cottoquinazoline D

**Fig. 1**

**Antimalarial drugs**

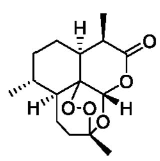

**5**. Artemisinin

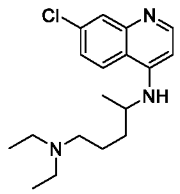

**6**. Chloroquine

**Target compounds**

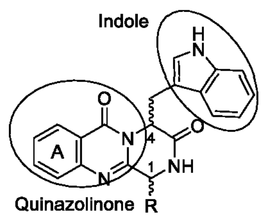

**Pyrazino[2,1-*b*]quinazoline-**
**-3,6-dione indole alkaloid**

**Antimalarial lead drug candidates**

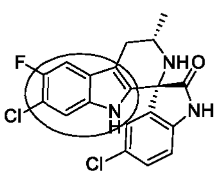

**7**. Spiroindolone

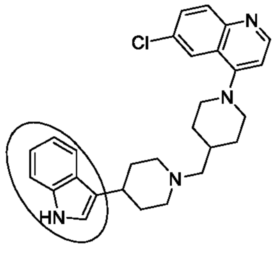

**8**. TCMDC-134281

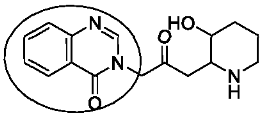

**9**.Febrifugine

**Fig. 2**

**First modification**

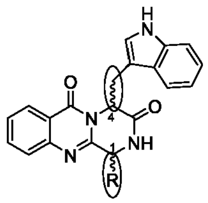

**10**, R = *i*-Pr, (1*S*, 4*S*)
**11**, R = *i*-Pr, (1*R*, 4*S*)
**12**, R = *i*-Pr, (1*S*, 4*R*)
**13**, R = *i*-Pr, (1*R*, 4*R*)
**14**, R = *i*-Bu, (1*S*, 4*S*)
**15**, R = *i*-Bu, (1*R*, 4*S*)
**16**, R = *i*-Bu, (1*S*, 4*R*)
**17**, R = *i*-Bu, (1*R*, 4*R*)

**Second modification**

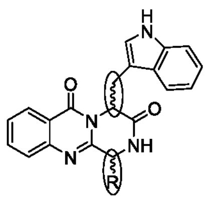

**18**, R = *s*-Bu, (1*R*, 4*S*)
**19**, R = *s*-Bu, (1*S*, 4*R*)
**20**, R = $(CH_2)_2SCH_3$, (1*R*, 4*S*)
**21**, R = $(CH_2)_2SCH_3$, (1*S*, 4*R*)
**22**, R = $CH_2C_6H_4OCH_2C_6H_5$, (1*S*, 4*S*)
**23**, R = $CH_2C_6H_4OCH_2C_6H_5$, (1*R*, 4*S*)
**24**, R = $CH_2C_6H_4OH$, (1*S*, 4*S*)
**25**, R = $CH_2C_6H_4OH$, (1*R*, 4*S*)
**26**, R = $CH_3$ (1*R*, 1*R*)

**Third modification**

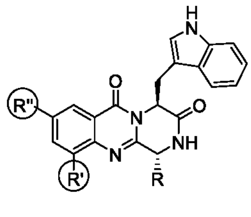

**27**, R = *i*-Pr, R' = Cl, R'' = H, (1*S*, 4*R*)
**28**, R = *i*-Bu, R' = Cl, R'' = H, (1*S*, 4*R*)
**29**, R = *s*-Bu, R' = Cl, R'' = H, (1*S*, 4*R*)
**30**, R = *i*-Pr, R' = R'' = Cl (1*S*, 4*R*)
**31**, R = *i*-Bu, R' = R'' = Cl, (1*S*, 4*R*)
**32**, R = *s*-Bu, R' = R'' = Cl, (1*S*, 4*R*)
**33**, R = *i*-Pr, R' = I, R'' = H, (1*S*, 4*R*)
**34**, R = *i*-Pr, R' = Br, R'' = H, (1*S*, 4*R*)
**35**, R = *i*-Bu, R' = I, R'' = H, (1*S*, 4*R*)
**36**, R = *i*-Bu, R' = Br, R'' = H, (1*S*, 4*R*)
**37**, R = *i*-Bu, R' = R'' = I, (1*S*, 4*R*)
**38**, R= $CH_2C_6H_4OCH_2C_6H_5$, R' =R'' =Cl

**Fourth modification**

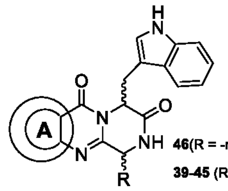

**46**(R = -methylenindole)
**39-45** (R = *i*-Bu)

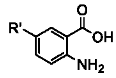

**39**, R = OH
**40**, R = Me
**41**, R = OMe

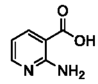

**43**

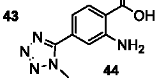

**44**

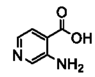

**42**

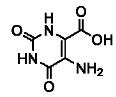

**45**

**Fig. 3**

## *Structure-actiity relationship*

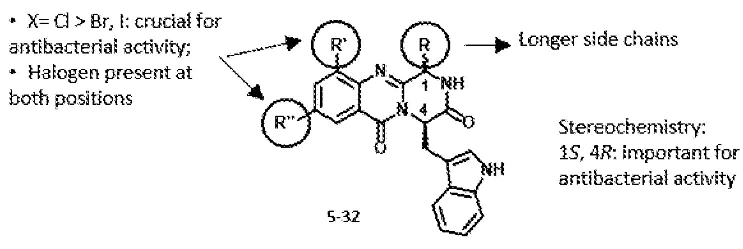


**Fig. 4**

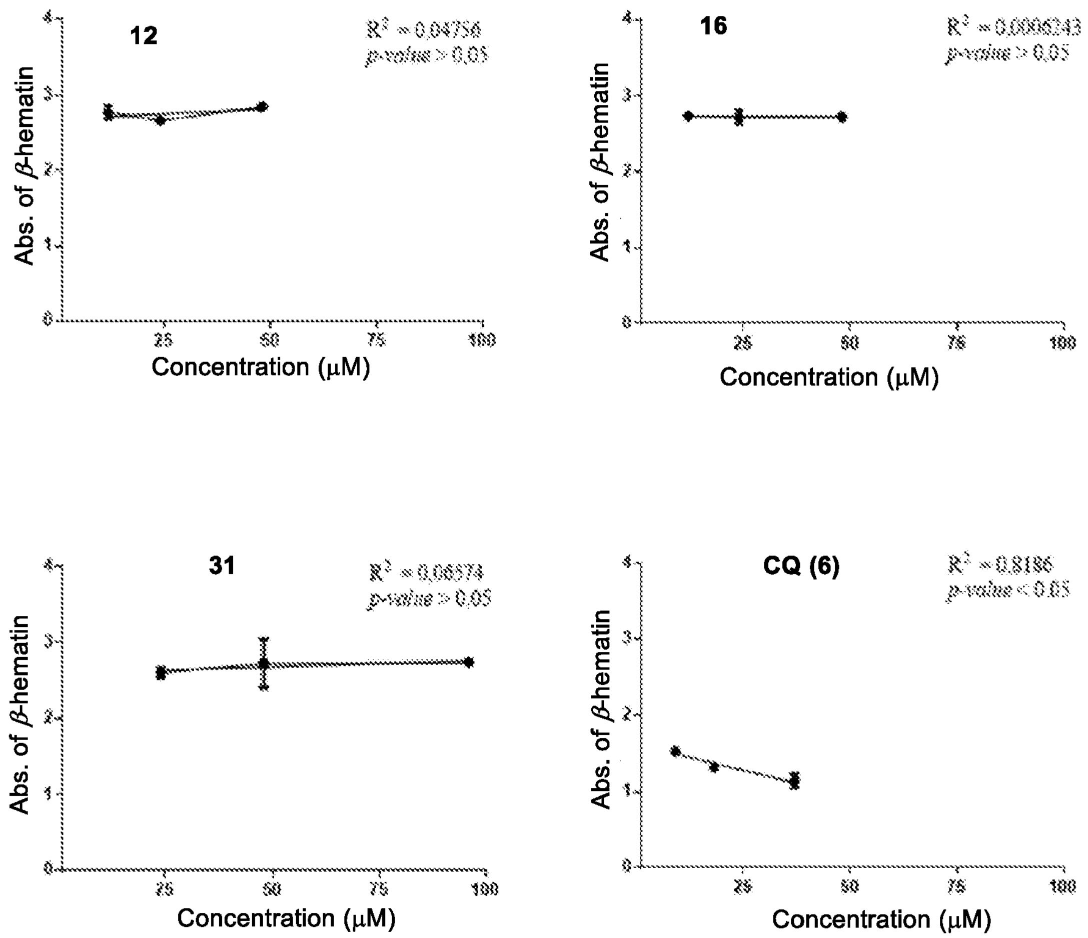
12
$R^2$ = 0.04756
p-value > 0.05
Abs. of β-hematin
Concentration (μM)
16
$R^2$ = 0.0006243
p-value > 0.05
Abs. of β-hematin
Concentration (μM)
31
$R^2$ = 0.06574
p-value > 0.05
Abs. of β-hematin
Concentration (μM)
CQ (6)
$R^2$ = 0.8186
p-value < 0.05
Abs. of β-hematin
Concentration (μM)

**Fig. 5**

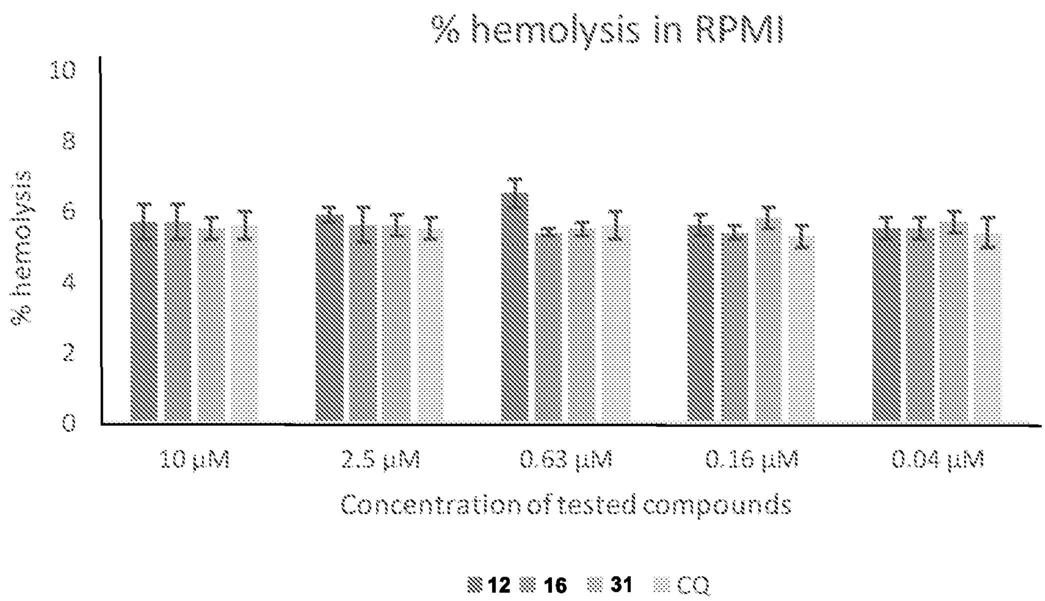
% hemolysis in RPMI
% hemolysis
10 μM
2.5 μM
0.63 μM
0.16 μM
0.04 μM
Concentration of tested compounds
12 16 31 CQ

**Fig. 6**

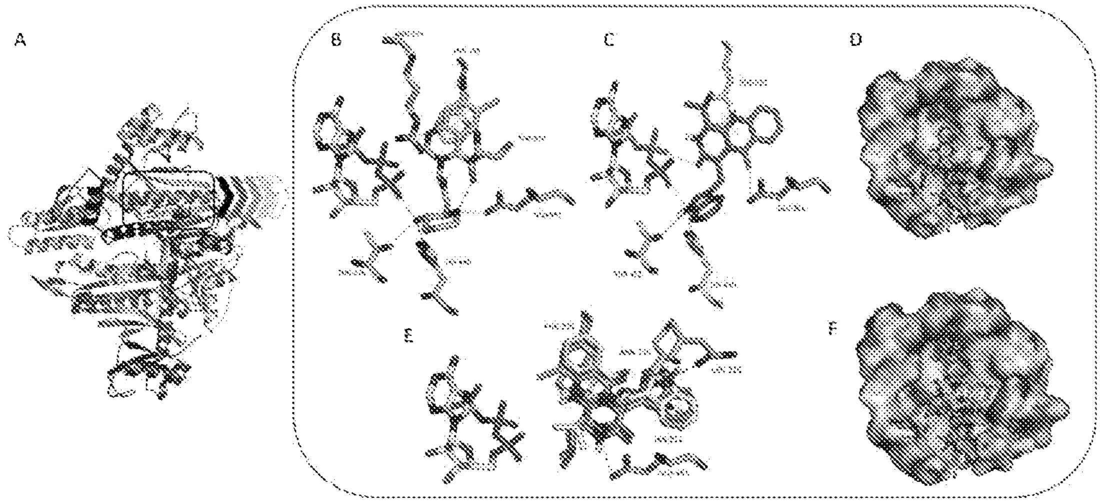
A
B
C
D
E
F

Fig. 7

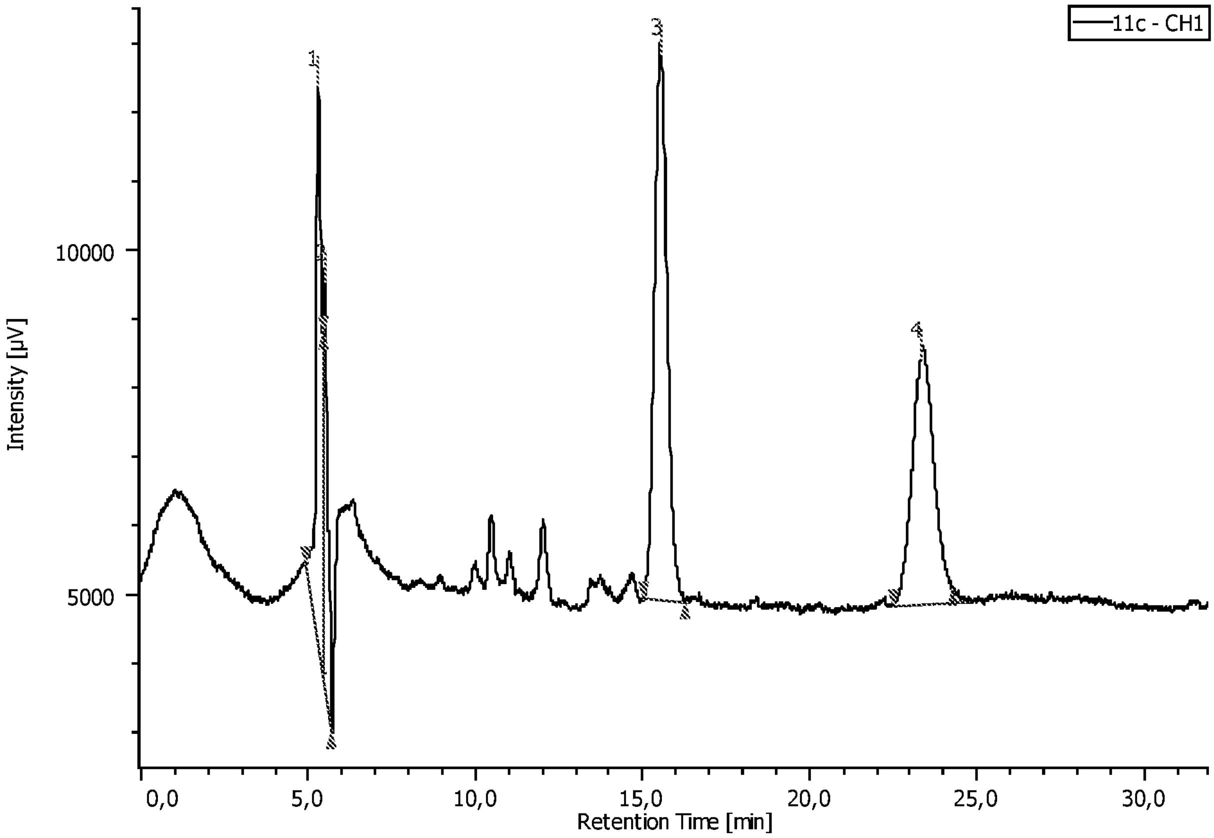
11c - CH1
1
3
4
10000
5000
Intensity [µV]
0,0
5,0
10,0
15,0
20,0
25,0
30,0
Retention Time [min]

Fig. 8A

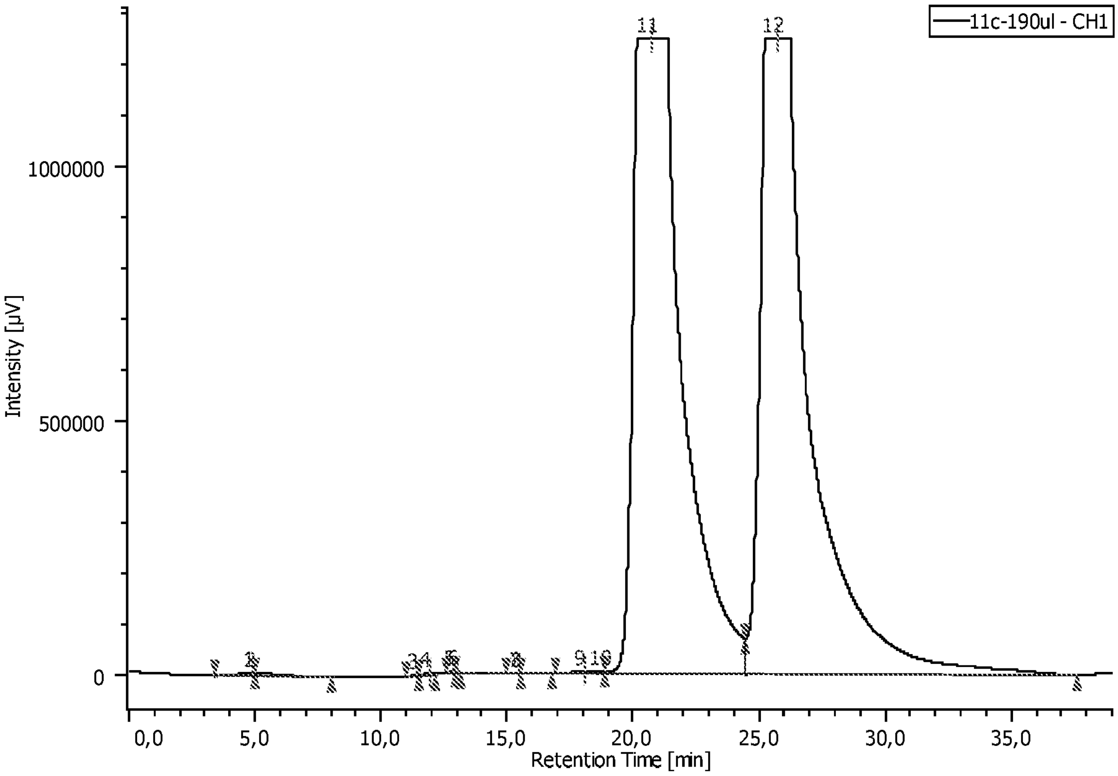
11c-190ul - CH1
Intensity [µV]
1000000
500000
0
Retention Time [min]
0,0
5,0
10,0
15,0
20,0
25,0
30,0
35,0
11
12

**Fig. 8B**

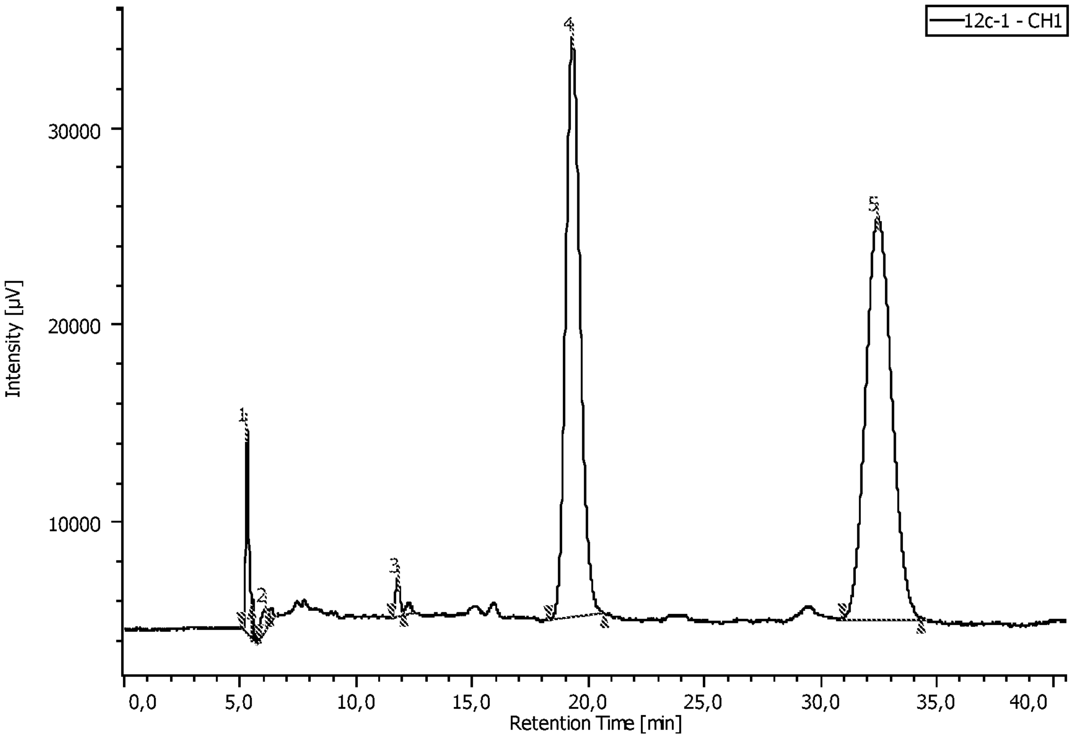
12c-1 - CH1
Intensity [µV]
30000
20000
10000
Retention Time [min]
0,0
5,0
10,0
15,0
20,0
25,0
30,0
35,0
40,0
1
2
3
4
5

**Fig. 8C**

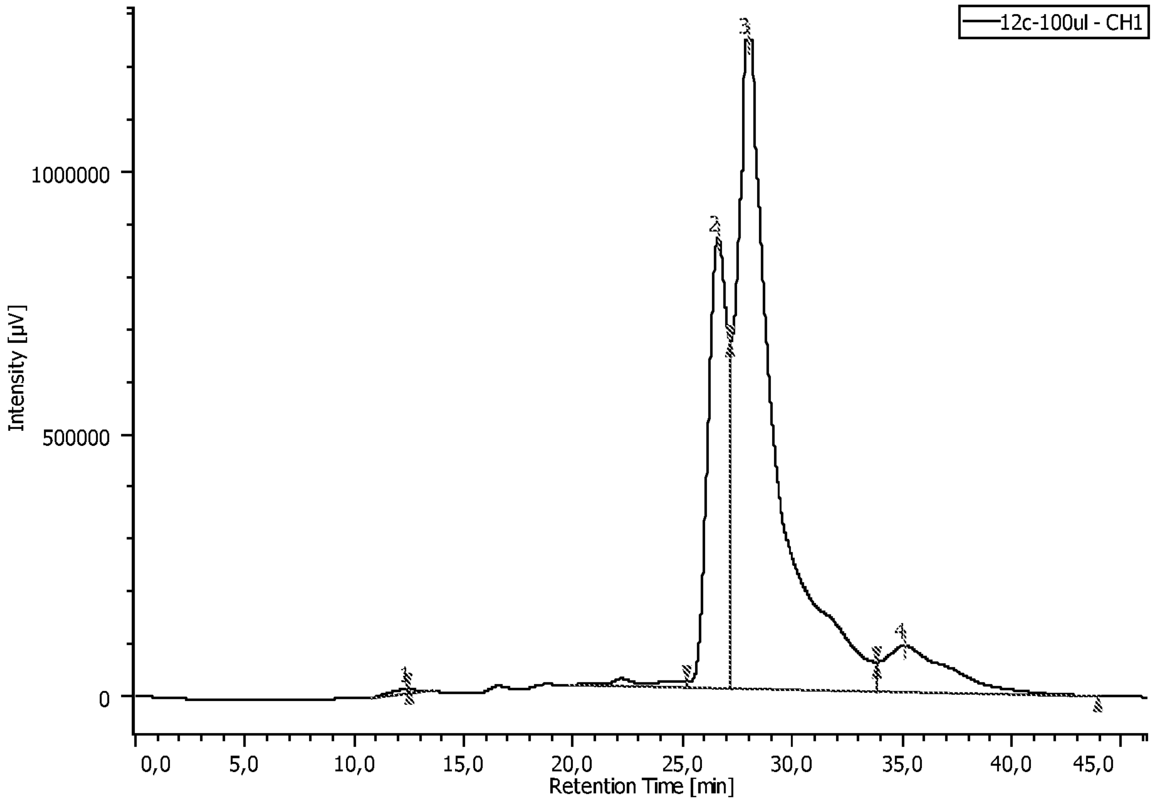
12c-100ul - CH1
Intensity [µV]
1000000
500000
0
0,0
5,0
10,0
15,0
20,0
25,0
30,0
35,0
40,0
45,0
Retention Time [min]

Fig. 8D

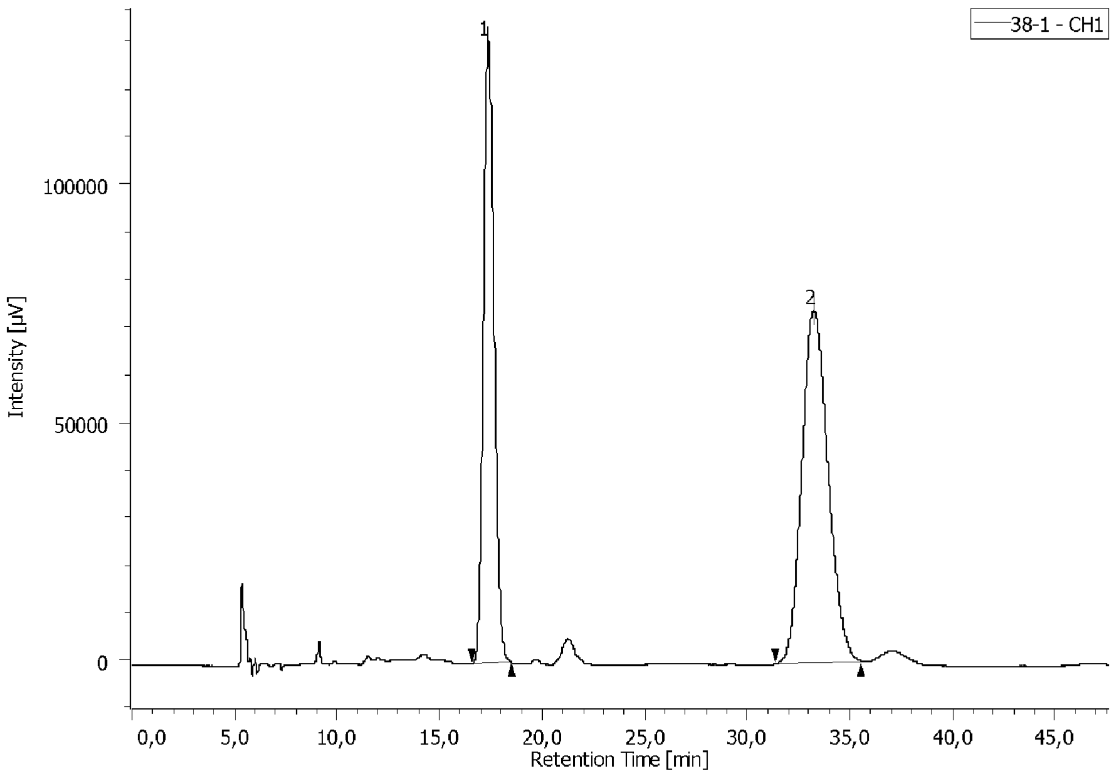
38-1 - CH1
1
2
Intensity [µV]
100000
50000
0
0,0
5,0
10,0
15,0
20,0
25,0
30,0
35,0
40,0
45,0
Retention Time [min]

Fig. 8E

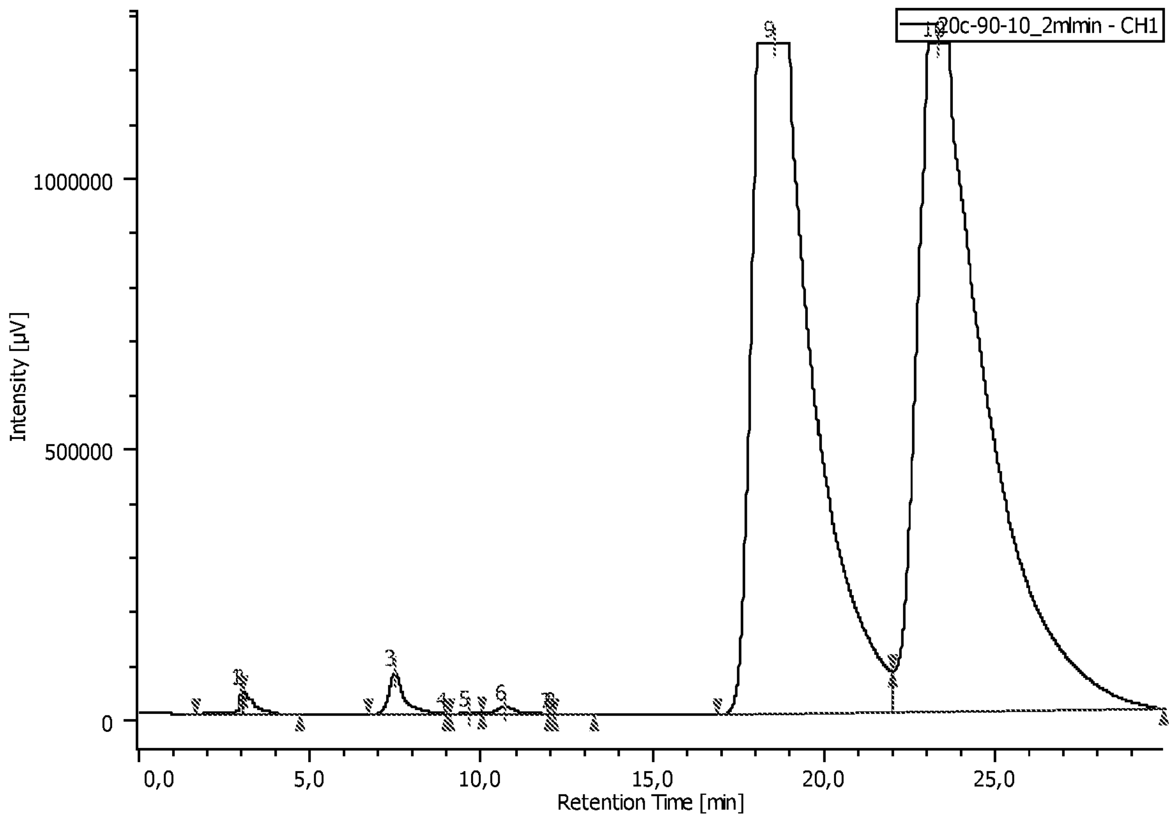
20c-90-10_2mlmin - CH1
1000000
500000
0
Intensity [µV]
0,0
5,0
10,0
15,0
20,0
25,0
Retention Time [min]
1
2
3
4
5
6
7
8
9
10

Fig. 8F

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0018769 A2

### Non-patent literature cited in the description


- **LIAO L** ; **YOU M** ; **CHUNG BK** ; **OH DC** ; **OH KB** ; **SHIN J.** Alkaloidal metabolites from a marine-derived Aspergillus sp. fungus. *J Nat Prod.*, 27 March 2015, vol. 78 (3), 349-54
- **SILVA MG** ; **FURTADO NA** ; **PUPO MT** ; **FONSECA MJ** ; **SAID S** ; **DA SILVA FILHO AA** ; **BASTOS JK**. Antibacterial activity from Penicillium corylophilum Dierckx.. *Microbiol Res.*, 2004, vol. 159 (4), 317-22
- **RESENDE, D. I. S. P** ; **BOONPOTHONG, P.** ; **;** **SOUSA, E.** ; **KIJJOA, A.** ; **PINTO, M. M. M.** Chemistry of the fumiquinazolines and structurally related alkaloids.. *Natural Product Reports*, 2019, vol. 36 (1), 7-34
- **BESSA, L. J.** ; **BUTTACHON, S.** ; **DETHOUP, T** ; **MARTINS, R.** ; **VASCONCELOS, V.** ; **KIJJOA, A** ; **DA COSTA, P. M.** Neofiscalin A and fiscalin C are potential novel indole alkaloid alternatives for the treatment of multidrug-resistant Gram-positive bacterial infections.. *FEMS Microbiol. Lett.*, 2016, vol. 363 (15), 1-5
- **LONG, S.** ; **RESENDE, D. I. S. P.** ; **KIJJOA, A** ; **SILVA, A. M. S.** ; **PINA, A.** ; **FERNÁNDEZ-MARCELO, T.** ; **VASCONCELOS, M. H.** ; **SOUSA, E.** ; **PINTO, M. M. M.** Antitumor Activity of Quinazolinone Alkaloids Inspired by Marine Natural Products.. *Marine Drugs*, 2018, vol. 16 (8)
- **LONG, S** ; **RESENDE, D. I. S. P** ; **KIJJOA, A** ; **SILVA, A. M. S.** ; **FERNANDES, R.** ; **XAVIER, C. P. R.** ; **VASCONCELOS, M. H.** ; **SOUSA, E.** ; **PINTO, M. M. M.** Synthesis of New Proteomimetic Quinazolinone Alkaloids and Evaluation of Their Neuroprotective and Antitumor Effects. *Molecules*, 2019, vol. 24 (3), 534
- **GAMO FJ** ; **SANZ LM** ; **VIDAL J** ; **DE COZAR C** ; **ALVAREZ E** ; **LAVANDERA JL** ; **VANDERWALL DE** ; **GREEN DVS** ; **KUMAR V** ; **HASAN S et al.** Thousands of chemical starting points for antimalarial lead identification. *Nature*, 2010, vol. 465 (7296), 305-310